# FASCICULE DE BREVET EUROPEEN

(11) **EP 3 273 934 B1**
(45) Date de publication et mention de la délivrance du brevet: **24.04.2019**
(21) Numéro de dépôt: 16711842.1
(22) Date de dépôt: 25.03.2016
(51) Int. Cl.: A61K 8/34, A61K 8/37, A61K 8/41, A61K 8/58, A61Q 5/12, A61K 8/81, A61K 8/898, A61Q 5/02, A61Q 5/00

(54) **PROCÉDÉ DE TRAITEMENT COSMÉTIQUE DES CHEVEUX**
VERFAHREN ZUR KOSMETISCHEN HAARBEHANDLUNG
COSMETIC HAIR TREATMENT METHOD

(30) Priorité: 25.03.2015 FR 1552484
(43) Date de publication de la demande: 31.01.2018
(73) Titulaire: L'OREAL, 75008 Paris (FR)
(72) Inventeur: BRUN, Julie, 92600 Asnières sur Seine (FR); MAGGIO, Sandrine, 93400 Saint-Ouen (FR); FACK, Géraldine, 92300 Levallois (FR)
(74) Mandataire: Dodin, Catherine
(86) Numéro de dépôt international: PCT/EP2016/056720
(87) Numéro de publication internationale: WO 2016/151139

(56) Documents cités:
- EP-A2- 2 111 848
- WO-A1-2012/055811
- WO-A2-2010/141683
- FR-A1- 2 958 538
- FR-A1- 2 975 593
- Sexnmakeupdiairies.Wordpress.com: "L'Oréal Professionnel Fiberceutic-hair botox?", , 20 août 2012 (2012-08-20), pages 1-2, XP002751653, Extrait de l'Internet: URL:https://sexnmakeupdiaries.wordpress.co m/2012/08/20/loreal-professionnal-fiberceu tic-hair-botox/ [extrait le 2015-11-30] -& DATABASE GNPD [Online] MINTEL; décembre 2010 (2010-12), "Hair Filing Serum for Damaged Hair", XP002751845, Database accession no. 1459029 -& DATABASE GNPD [Online] MINTEL; décembre 2010 (2010-12), "Hair Filing Serum for Damaged Hair", XP002751845, Database accession no. 1459029 -& DATABASE GNPD [Online] MINTEL; décembre 2010 (2010-12), "Restorative Hair Sealing Treatment for Fine Hair", XP002751846, Database accession no. 1458979 -& DATABASE GNPD [Online] MINTEL; décembre 2010 (2010-12), "Restorative hair Sealing Treatment for Thick Hair", XP002751847, Database accession no. 1458931 -& DATABASE GNPD [Onli
- DATABASE GNPD [Online] MINTEL; décembre 2013 (2013-12), "Hair conditionner", XP002742867, Database accession no. 2260246

## Description

La présente invention concerne un procédé de traitement cosmétique des cheveux, consistant à appliquer de manière séquencée, une composition cosmétique comprenant un tensioactif cationique, une silicone et un corps gras non siliconé, puis une composition cosmétique comprenant un tensioactif cationique, un polymère cationique particulier et un organosilane.

Les cheveux peuvent être fragilisés ou abîmés par l'action des agents atmosphériques extérieurs tels que la lumière et les intempéries, ou par des traitements mécaniques ou chimiques tels que le brossage, le peignage, les teintures, les décolorations, les permanentes et/ou les défrisages.
Pour remédier à ces inconvénients, il est usuel d'avoir recours à des soins capillaires qui permettent d'apporter du conditionnement aux cheveux. Ces compositions de soins capillaires peuvent être des shampooings conditionneurs ou des après-shampoings pouvant se présenter sous la forme de gels, de lotions capillaires ou de crèmes plus ou moins épaisses.
Pour améliorer les propriétés cosmétiques de ces compositions, il est connu d'introduire dans ces dernières des agents conditionneurs destinés principalement à réparer ou à limiter les effets néfastes ou indésirables induits par les différents traitements ou agressions que subissent, de manière plus ou moins répétée, les fibres capillaires.
Dans ce but, on a déjà proposé d'utiliser, entre autres, des organosilanes dans des compositions cosmétiques de soin, pour conférer aux cheveux des propriétés de conditionnement satisfaisantes. De telles compositions sont par exemple décrites dans les demandes de brevet FR2910276, EP2343042 et EP2111848.

Toutefois, les compositions de soins décrites dans l'art antérieur procurent des propriétés de conditionnement et de démêlage qui ne durent pas suffisamment longtemps dans le temps. En effet, ces propriétés ne résistent généralement pas suffisamment au lavage et ont tendance à diminuer dès le premier shampoing. Autrement dit, même si ces compositions de soin capillaire permettent d'obtenir des résultats convenables pendant et juste après leur utilisation (à T0), on a constaté que ces effets avaient tendance à disparaitre au fur et à mesure des lavages des cheveux; il était alors nécessaire de renouveler régulièrement l'application de soins capillaires pour conserver des effets de conditionnement adéquats.

Il existe donc un réel besoin de disposer d'un procédé de traitement cosmétique des cheveux ne présentant pas les inconvénients mentionnés ci-avant, et qui soit notamment capable de procurer des propriétés de conditionnement et de démêlage non seulement satisfaisantes immédiatement après la première application, mais également rémanentes au lavage, par exemple rémanentes au moins après 3 shampoings.

La présente invention a pour but de proposer un tel procédé de traitement cosmétique des cheveux, permettant l'obtention d'un conditionnement à la fois immédiat et rémanent (ou durable).

La présente invention a donc pour objet un procédé de traitement cosmétique des cheveux, comprenant :
- une étape (i) d'application sur lesdits cheveux, d'une composition cosmétique dite première, comprenant un ou plusieurs tensioactifs cationiques, une ou plusieurs silicones et un ou plusieurs corps gras non siliconés, puis
- une étape (ii) d'application sur lesdits cheveux, d'une composition cosmétique dite deuxième, comprenant un ou plusieurs tensioactifs cationiques, un ou plusieurs polymères cationiques présentant une densité de charge cationique supérieure ou égale à 4 méq/g, et un ou plusieurs organosilanes ;
ledit procédé ne comprenant pas d'étape de rinçage intermédiaire entre lesdites étape (i) et étape (ii) d'application.

La mise en oeuvre du procédé selon l'invention permet notamment d'obtenir, dès l'application, les propriétés recherchées et en particulier de la souplesse, du lissage, du volume et un toucher naturel non chargé, tout en conservant des cheveux légers et individualisés; ce procédé apporte tout particulièrement aux cheveux une aptitude au démêlage améliorée.
Par ailleurs, les propriétés procurées par ledit procédé selon l'invention résistent bien aux diverses agressions que peuvent subir les cheveux, telles que la lumière, les intempéries, le lavage, la transpiration. Elles sont en particulier rémanentes aux shampoings, notamment après au moins 3 shampoings.

Dans la présente description, l'expression "au moins un" est équivalente à l'expression "un ou plusieurs" et peut y être substituée; l'expression "compris entre" est équivalente à l'expression "allant de" et peut y être substituée, et sous-entend que les bornes sont incluses.

### Procédé

Le procédé de traitement cosmétique selon l'invention comprend donc une première étape (étape (i)) consistant en l'application sur les cheveux d'une composition cosmétique (que l'on peut appeler « composition première») comprenant un ou plusieurs tensioactifs cationiques, une ou plusieurs silicones et un ou plusieurs corps gras non siliconés.
Cette première étape peut être suivie ou non d'une étape de pose, par exemple un temps de pose de 1 à 30 minutes, de préférence de 1 à 15 minutes, mieux de 2 à 15 minutes. De préférence, ladite première étape est suivie d'une étape de pose de la composition, de préférence pendant 1 à 15 minutes.
Toutefois, cette première étape n'est pas suivie d'une étape de rinçage, par exemple à l'eau, avant mise en oeuvre de la deuxième étape décrite ci-après. On entend par-là que la composition deuxième ci-après est directement appliquée sur les cheveux sur lesquels se trouve encore ladite composition première. Il n'y a pas d'étape « d'élimination » de ladite 1^{ère} composition avant application de ladite 2^{ème} composition.
En d'autres termes, les étapes (i) et (ii) d'application sont effectuées sans étape de rinçage intermédiaire.

Le procédé de traitement cosmétique selon l'invention comprend une deuxième étape (étape (ii)) consistant en l'application sur les cheveux d'une composition cosmétique (que l'on peut appeler « composition deuxième») comprenant un ou plusieurs tensioactifs cationiques, un ou plusieurs polymères cationiques présentant une densité de charge cationique supérieure ou égale à 4 méq/g, et un ou plusieurs organosilanes.
Ladite 2^{ème} étape est effectuée après ladite 1^{ère} étape ci-dessus; il n'y a pas d'étape de rinçage intermédiaire entre lesdites 1^{ère} et 2^{ème} étapes.
Par rinçage, on entend notamment une étape usuelle de rinçage, par exemple à l'eau, froide ou chaude, par exemple de 30-40°C, par exemple pendant quelques 1 à 10 minutes, notamment 1 à 10 minutes; l'eau pouvant éventuellement comprendre des composés additionnels.
De préférence, ladite 2^{ème} étape d'application est effectuée immédiatement après ladite 1^{ère} étape d'application, notamment environ 1 à 30 minutes après ladite 1^{ère} étape d'application, c'est-à-dire après l'éventuel temps de pose de ladite 1^{ère} composition appliquée à la 1^{ère} étape.

Cette deuxième étape peut être suivie ou non d'une étape de pose, par exemple un temps de pose de 1 à 25 minutes; elle peut également être suivie d'une étape de rinçage, par exemple à l'eau et/ou elle peut être suivie d'une étape de séchage, par exemple à l'air chaud.
De préférence, ladite deuxième étape est suivie d'une étape de pose de la composition, de préférence pendant 1 à 25 minutes, notamment 1 à 20 minutes, et d'une étape de rinçage, puis d'une étape de séchage.

L'étape d'application de ladite composition première et l'étape d'application de ladite composition deuxième sont faites de manière successive, ou séquentiellement, ce qui signifie que l'étape d'application de ladite composition première est antérieure à l'étape d'application de ladite composition deuxième; des étapes intermédiaires, par exemple de pose et/ou de rinçage, pouvant être présentes entre ces deux étapes d'applications.

Dans un mode de réalisation particulier de l'invention, il est possible de prévoir, antérieurement à ladite première étape du procédé, une étape dite de lavage des cheveux, comprenant l'application sur lesdits cheveux d'une composition lavante, par exemple de type shampoing, comprenant de préférence un ou plusieurs tensioactifs détergents; de préférence, ladite étape antérieure de lavage peut être suivie d'une étape de rinçage, par exemple à l'eau, avant mise en oeuvre du procédé selon l'invention.

### Première composition

Le procédé de traitement cosmétique selon l'invention comprend donc une première étape consistant en l'application sur les cheveux d'une composition cosmétique dite première comprenant un ou plusieurs tensioactifs cationiques, une ou plusieurs silicones et un ou plusieurs corps gras non siliconés.

### a/ Tensioactifs cationiques

Ladite première composition cosmétique comprend donc un ou plusieurs tensioactifs cationiques, avantageusement choisis parmi les sels d'amines grasses primaire, secondaire ou tertiaire, éventuellement polyoxyalkylénées; les sels d'ammonium quaternaire, et leurs mélanges.

Comme sels d'ammonium quaternaire, on peut notamment citer :
- les sels d'ammonium quaternaire de formule (la) : 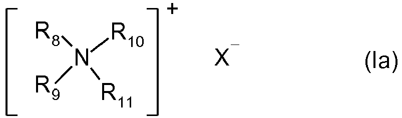 dans laquelle :
   les groupes R₈ à R₁₁, identiques ou différents, représentent un groupe aliphatique linéaire ou ramifié, comportant de 1 à 30 atomes de carbone, ou un groupe aromatique tel que aryle ou alkylaryle, au moins l'un des groupes R₈ à R₁₁ comportant de 8 à 30 atomes de carbone, de préférence de 12 à 24 atomes de carbone; les groupes aliphatiques pouvant comporter des hétéroatomes tels que notamment l'oxygène, l'azote, le soufre et les halogènes ; et
   - X⁻ est un anion notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.

Les groupes aliphatiques R8 à R11 peuvent être choisis parmi les groupes alkyle en C₁-C₃₀, alcoxy en C₁-C₃₀, polyoxyalkylène (C₂-C₆), alkylamide en C₁-C₃₀, alkyl(C₁₂-C₂₂)amidoalkyle(C₂-C₆), alkyl(C₁₂-C₂₂)acétate et hydroxyalkyle en C₁-C₃₀.

On peut notamment citer les halogénures, notamment les chlorures, de tétraalkylammonium comme les chlorures de dialkyldiméthylammonium ou d'alkyltriméthylammonium dans lesquels le groupe alkyle comporte de 12 à 22 atomes de carbone, en particulier les chlorures de béhényltriméthylammonium, de distéaryldiméthylammonium, de cétyltriméthylammonium, de benzyldiméthylstéarylammonium. On peut également citer les halogénures, et notamment les chlorures, de palmitylamidopropyltriméthylammonium ou de stéaramidopropyldiméthyl-(myristyl acétate)-ammonium; notamment le produit commercialisé sous la dénomination CERAPHYL® 70 par la société VAN DYK.
- les sels d'ammonium quaternaire de l'imidazoline de formule (IIa) : 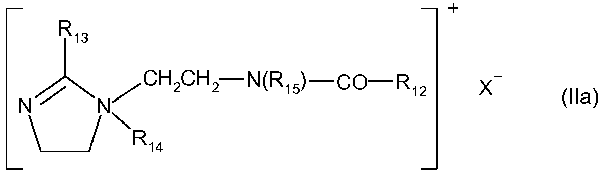 dans laquelle
   R₁₂ représente un groupe alcényle ou alkyle comportant de 8 à 30 atomes de carbone, par exemple dérivés des acides gras du suif,
   R₁₃ représente un atome d'hydrogène, un groupe alkyle en C₁-C₄ ou un groupe alcényle ou alkyle comportant de 8 à 30 atomes de carbone,
   R₁₄ représente un groupe alkyle en C₁-C₄,
   R₁₅ représente un atome d'hydrogène ou un groupe alkyle en C₁-C₄,
   X⁻ est un anion, notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.
   De préférence, R₁₂ et R₁₃ désignent un mélange de groupes alcényle ou alkyle comportant de 12 à 21 atomes de carbone, par exemple dérivés des acides gras du suif, R₁₄ désigne un groupe méthyle, R₁₅ désigne un atome d'hydrogène. Un tel produit est par exemple commercialisé sous la dénomination REWOQUAT® W75 ou W90 par la société Evonik.

- les sels de di- ou de triammonium quaternaire de formule (IIIa) : 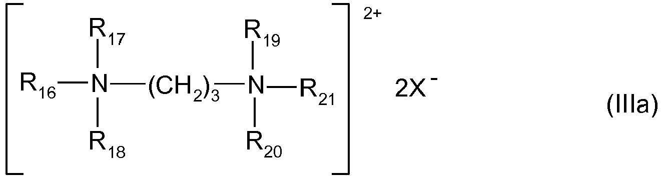 dans laquelle :
   - R₁₆ désigne un groupe alkyle comportant de 16 à 30 atomes de carbone éventuellement hydroxylé et/ou éventuellement interrompu par un ou plusieurs atomes d'oxygène,
   - R₁₇ désigne l'hydrogène, un groupe alkyle comportant de 1 à 4 atomes de carbone ou un groupe -(CH₂)₃-N⁺(R₁₆ₐ)(R₁₇ₐ)(R₁₈ₐ), R₁₆ₐ, R₁₇ₐ, R₁₈ₐ, identiques ou différents désignant l'hydrogène ou un groupe alkyle comportant de 1 à 4 atomes de carbone,
   - R₁₈, R₁₉, R₂₀ et R₂₁, identiques ou différents, désignent l'hydrogène ou un groupe alkyle comportant de 1 à 4 atomes de carbone, et
   - X⁻ est un anion notamment choisi dans le groupe des halogénures, acétates, phosphates, nitrates, alkyl(C₁-C₄)sulfates, alkyl(C₁-C₄)sulfonates et alkyl(C₁-C₄)aryl-sulfonates, en particulier méthylsulfate et éthylsulfate.
      De tels composés sont par exemple le Finquat CT-P (Quaternium 89) et le Finquat CT (Quaternium 75) proposés par la société FINETEX.
- les sels d'ammonium quaternaire contenant une ou plusieurs fonctions esters de formule (IVa) suivante : 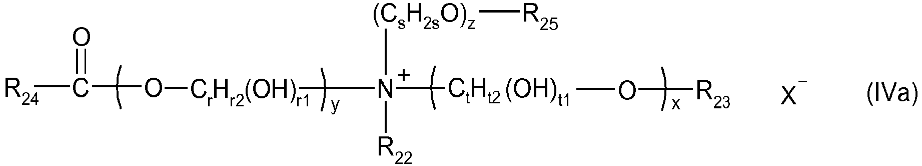 dans laquelle :
   - R₂₂ est choisi parmi les groupes alkyles en C₁-C₆ et les groupes hydroxyalkyle ou dihydroxyalkyle en C₁-C₆,
   - R₂₃ est choisi parmi le groupe R₂₆-C(=O)-; les groupes R₂₇ hydrocarbonés en C₁-C₂₂, linéaires ou ramifiés, saturés ou insaturés; et l'atome d'hydrogène,
   - R₂₅ est choisi parmi le groupe R₂₈-C(=O)-; les groupes R₂₉ hydrocarbonés en C₁-C₆, linéaires ou ramifiés, saturés ou insaturés; et l'atome d'hydrogène,
   - R₂₄, R₂₆ et R₂₈, identiques ou différents, sont choisis parmi les groupes hydrocarbonés en C₇-C₂₁, linéaires ou ramifiés, saturés ou insaturés,
   - r, s et t, identiques ou différents, sont des entiers valant de 2 à 6,
   - r1 et t1, identiques ou différents, valent 0 ou 1,
   - y est un entier valant de 1 à 10,
   - x et z, identiques ou différents, sont des entiers valant de 0 à 10,
   - X⁻ est un anion,
   étant entendu que r2 + r1 = 2r et t1 + t2 = 2t, et que la somme x + y + z vaut de 1 à 15,
   sous réserve que lorsque x = 0 alors R₂₃ désigne R₂₇ et que lorsque z = 0 alors R₂₅ désigne R₂₉.

Les groupes alkyles R₂₂ peuvent être linéaires ou ramifiés, de préférence linéaires. De préférence, R₂₂ désigne un groupe méthyle, éthyle, hydroxyéthyle ou dihydroxypropyle, et plus particulièrement un groupe méthyle ou éthyle. Avantageusement, la somme x + y + z vaut de 1 à 10.
Lorsque R₂₃ est un groupe R₂₇ hydrocarboné, il peut comprendre de 12 à 22 atomes de carbone, ou bien comprendre de 1 à 3 atomes de carbone.
Lorsque R₂₅ est un groupe R₂₉ hydrocarboné, il a de préférence 1 à 3 atomes de carbone.
Avantageusement, R₂₄, R₂₆ et R₂₈, identiques ou différents, sont choisis parmi les groupes hydrocarbonés en C₁₁-C₂₁, linéaires ou ramifiés, saturés ou insaturés, et plus particulièrement parmi les groupes alkyle et alcényle en C₁₁-C₂₁, linéaires ou ramifiés.
De préférence, x et z, identiques ou différents, valent 0 ou 1.
Avantageusement, y est égal à 1.
De préférence, r, s et t, identiques ou différents, valent 2 ou 3, et encore plus particulièrement sont égaux à 2.
L'anion X⁻ est de préférence un halogénure, de préférence chlorure, bromure ou iodure, un alkyl(C₁-C₄)sulfate, un alkyl(C₁-C₄)sulfonate ou un alkyl(C₁-C₄)aryl-sulfonate, un méthanesulfonate, un phosphate, un nitrate, un tosylate, un anion dérivé d'acide organique tel qu'un acétate ou un lactate ou tout autre anion compatible avec l'ammonium à fonction ester. L'anion X⁻ est plus particulièrement un chlorure, un méthylsulfate ou un éthylsulfate.

On utilise plus particulièrement dans la composition selon l'invention, les sels d'ammonium de formule (IVa) dans laquelle :
- R₂₂ désigne un groupe méthyle ou éthyle,
- x et y sont égaux à 1,
- z est égal à 0 ou 1,
- r, s et t sont égaux à 2,
- R₂₃ est choisi parmi le groupe R₂₆-C(=O)-; les groupes méthyle, éthyle ou hydrocarbonés en C₁₄-C₂₂, l'atome d'hydrogène,
- R₂₅ est choisi parmi le groupe R₂₈-C(=O)-; l'atome d'hydrogène,
- R₂₄, R₂₆ et R₂₈, identiques ou différents, sont choisis parmi les groupes hydrocarbonés en C₁₃-C₁₇, linéaires ou ramifiés, saturés ou insaturés, et de préférence parmi les groupes alkyle et alcényle en C₁₃-C₁₇, linéaires ou ramifiés, saturés ou insaturés.
Avantageusement, les groupes hydrocarbonés sont linéaires.

On peut citer parmi les composés de formule (IVa) les sels, notamment le chlorure ou le méthylsulfate de diacyloxyéthyldiméthylammonium, de diacyloxyéthylhydroxyéthylméthylammonium, de monoacyloxyéthyldihydroxyéthyl méthylammonium, de triacyloxyéthylméthylammonium, de monoacyloxyéthylhydroxyéthyldiméthylammonium, et leurs mélanges. Les groupes acyles ont de préférence 14 à 18 atomes de carbone et proviennent plus particulièrement d'une huile végétale comme l'huile de palme ou de tournesol. Lorsque le composé contient plusieurs groupes acyles, ces derniers peuvent être identiques ou différents.
Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras, ou sur des mélanges d'acides gras notamment d'origine végétale ou animale, ou par transestérification de leurs esters méthyliques. Cette estérification peut être suivie d'une quaternisation à l'aide d'un agent d'alkylation tel qu'un halogénure d'alkyle, de préférence de méthyle ou d'éthyle, un sulfate de dialkyle, de préférence de méthyle ou d'éthyle, le méthanesulfonate de méthyle, le para-toluènesulfonate de méthyle, la chlorhydrine du glycol ou du glycérol. De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société Evonik.
La composition selon l'invention peut contenir par exemple un mélange de sels de mono-, di- et triester d'ammonium quaternaire avec une majorité en poids de sels de diester. On peut aussi utiliser les sels d'ammonium contenant au moins une fonction ester décrits dans les brevets US-A-4874554 et US-A-4137180. On peut également utiliser le chlorure de béhénoylhydroxypropyltriméthylammonium, par exemple, proposé par la société KAO sous la dénomination Quartamin BTC 131. De préférence, les sels d'ammonium contenant au moins une fonction ester contiennent deux fonctions esters.

De préférence, les tensioactifs cationiques sont choisis parmi ceux de formule (la) ou (IVa), et mieux parmi les sels de cétyltriméthylammonium, de béhényltriméthylammonium, de dipalmitoyléthylhydroxyéthylméthylammonium et leurs mélanges; et plus particulièrement parmi le chlorure ou le méthosulfate de béhényltriméthylammonium, le chlorure ou le méthosulfate de cétyltriméthylammonium, le chlorure ou le méthosulfate de dipalmitoyléthylhydroxyéthylméthylammonium et leurs mélanges.

Dans un mode de réalisation préféré, ladite première composition comprend au moins deux tensioactifs cationiques différents; notamment au moins un tensioactif cationique choisi parmi ceux de formule (la) et au moins un tensioactif cationique choisi parmi ceux de formule (IVa).

Ladite première composition cosmétique comprend le ou lesdits tensioactifs cationiques en une quantité allant de 0,1 à 3,5% en poids, de préférence de 0,2 à 3,5% en poids, préférentiellement de 0,3 à 3% en poids, par rapport au poids total de la composition.

### b/ Silicones

Ladite première composition cosmétique selon l'invention comprend également une ou plusieurs silicones, qui peuvent être solides ou liquides, volatiles ou non volatiles, aminées ou non aminées.

Comme silicones susceptibles d'être employées, on peut citer, seul ou en mélange, les polydialkylsiloxanes et notamment les polydiméthylsiloxanes (PDMS), les polydiarylsiloxanes, les polyalkylarylsiloxanes, les gommes et les résines de silicone, ainsi que les organopolysiloxanes (ou polysiloxanes organomodifiés, ou encore silicones organomodifiées) qui sont des polysiloxanes comportant dans leur structure un ou plusieurs groupements organofonctionnels, généralement fixés par l'intermédiaire d'un groupe hydrocarboné, et de préférence choisi parmi les groupements aryle, les groupements aminés, les groupements alcoxy et les groupements polyoxyéthylénés, ou polyoxypropylénés.

Les silicones organomodifiées peuvent être des polydiarylsiloxanes, notamment des polydiphénylsiloxanes, et des polyalkylarylsiloxanes fonctionnalisés par les groupes organofonctionnels mentionnés précédemment. Les polyalkylarylsiloxanes sont particulièrement choisis parmi les polydiméthyl/méthylphénylsiloxanes, les polydiméthyl /diphénylsiloxanes linéaires et/ou ramifiés.
Parmi les silicones organomodifiées, on peut citer les organopolysiloxanes comportant :
- des groupements polyoxyéthylèney et/ou polyoxypropylène comportant éventuellement des groupements alkyle en C₆-C₂₄ tels que les diméthicone-copolyols, et notamment ceux commercialisés par la société DOW CORNING sous la dénomination DC 1248 ou les huiles SILWET® L 722, L 7500, L 77, L 711 de la société UNION CARBIDE; ou encore les alkyl(C₁₂)-méthicone-copolyols, et notamment ceux commercialisés par la société DOW CORNING sous la dénomination Q2-5200;
- des groupements aminés substitués ou non, en particulier des groupements aminoalkyle en C₁-C₄; on peut citer les produits commercialisés sous la dénomination GP4 Silicone Fluid et GP7100 par la société GENESEE, ou sous les dénominations Q2-8220 et DC929 ou DC939 par la société DOW CORNING ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle;
- des groupements acyloxyalkyle tels que les polyorganosiloxanes décrits dans le brevet US-A-4957732.
- des groupements anioniques du type acide carboxylique, comme par exemple décris dans EP186507, ou du type alkyl-carboxylique comme le produit X-22-3701E de la société SHIN-ETSU; ou encore du type 2-hydroxyalkylsulfonate ou 2-hydroxyalkylthiosulfate, comme les produits commercialisés par la société GOLDSCHMIDT sous les dénominations "ABIL® S201" et "ABIL® S255".
- des groupements hydroxyacylamino, comme les polyorganosiloxanes décrits dans la demande EP342834; on peut citer, par exemple, le produit Q2-8413 de la société DOW CORNING.

Les silicones peuvent également être choisies parmi les polydialkylsiloxanes parmi lesquels on peut citer principalement les polydiméthylsiloxanes à groupements terminaux triméthylsilyl. Parmi ces polydialkylsiloxanes, on peut citer les produits commerciaux suivants :
- les huiles SILBIONE® des séries 47 et 70 047 ou les huiles MIRASIL® commercialisées par RHODIA telles que, par exemple l'huile 70 047 V 500 000;
- les huiles de la série MIRASIL® commercialisées par la société RHODIA;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.
On peut également citer les polydiméthylsiloxanes à groupements terminaux diméthylsilanol connus sous le nom de dimethiconol (CTFA), tels que les huiles de la série 48 de la société RHODIA.
Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.
Des produits plus particulièrement utilisables conformément à l'invention sont des mélanges tels que :
- les mélanges formés à partir d'un polydiméthylsiloxane hydroxylé en bout de chaîne, ou dimethiconol (CTFA) et d'un polydiméthylsiloxane cyclique également appelé cyclométhicone (CTFA) tel que le produit Q2-1401 commercialisé par la société DOW CORNING.
Les polyalkylarylsiloxanes sont particulièrement choisis parmi les polydiméthyl/méthylphénylsiloxanes, les polydiméthyl /diphénylsiloxanes linéaires et/ou ramifiés de viscosité allant de 1.10⁻⁵ à 5.10⁻²m²/s à 25°C.
Parmi ces polyalkylarylsiloxanes, on peut citer les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

De préférence la composition selon l'invention comprend une ou plusieurs silicones aminées. On désigne par silicone aminée toute silicone comportant au moins une amine primaire, secondaire, tertiaire ou un groupement ammonium quaternaire.

Les masses moléculaires moyennes en poids de ces silicones aminées peuvent être mesurées par Chromatographie par Perméation de Gel (GPC) à température ambiante (25°C) en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Comme silicone aminée susceptible d'être employée dans le cadre de l'invention, on peut citer :
a) les polysiloxanes répondant à la formule (A): 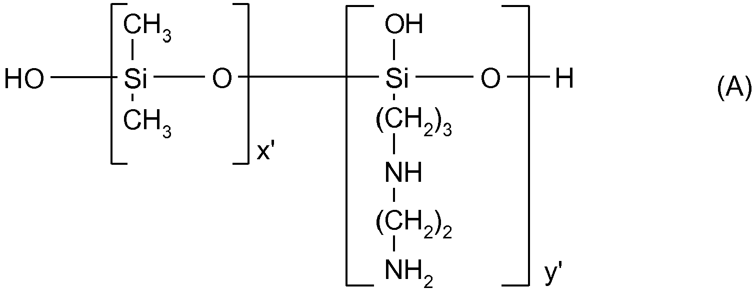 dans laquelle x' et y' sont des nombres entiers tels que le poids moléculaire moyen en poids (Mw) est compris entre 5 000 et 500 000 environ ;
b) les silicones aminées répondant à la formule (B) :

   R'ₐG₃₋ₐ-Si(OSiG₂)ₙ-(OSiG_{b}R'_{2-b})ₘ-O-SiG₃₋ₐ-R'ₐ (B)

   dans laquelle :
   - G, identique ou différent, désigne un atome d'hydrogène, un groupement phényle, OH, alkyle en C₁-C₈, par exemple méthyle, ou alcoxy en C₁-C₈, par exemple méthoxy,
   - a, identique ou différent, désigne 0 ou un entier de 1 à 3, en particulier 0,
   - b désigne 0 ou 1, en particulier 1,
   - m et n sont des nombres tels que la somme (n + m) varie de 1 à 2000, en particulier de 50 à 150, n pouvant désigner un nombre de 0 à 1999, et notamment de 49 à 149 et m pouvant désigner un nombre de 1 à 2000, et notamment de 1 à 10;
   - R', identique ou différent, désigne un radical monovalent de formule -CqH2qL dans laquelle q est un nombre allant de 2 à 8, et L est un groupement aminé éventuellement quaternisé choisi parmi les groupements :
      -N(R")₂ ; -N⁺(R")₃ A- ; -NR"-Q-N(R")₂ et -NR"-Q-N⁺(R")₃ A-,
      dans lesquels R", identique ou différent, désigne hydrogène, phényle, benzyle, ou un radical hydrocarboné saturé monovalent, par exemple un radical alkyle en C1-C20; Q désigne un groupement de formule CᵣH₂ᵣ, linéaire ou ramifié, r étant un entier allant de 2 à 6, de préférence de 2 à 4; et A- représente un anion cosmétiquement acceptable, notamment halogénure tel que fluorure, chlorure, bromure ou iodure.

Un premier groupe de silicones aminées correspondant à la formule (B) est représentée par les silicones dénommées "triméthylsilylamodiméthicone" répondant à la formule (C) : 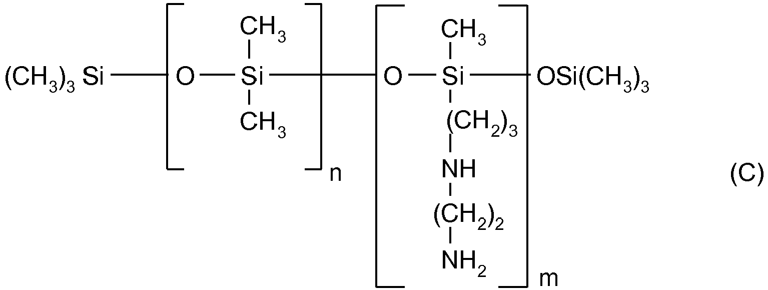 dans laquelle m et n sont des nombres tels que la somme (n + m) varie de 1 à 2000, en particulier de 50 à 150, n pouvant désigner un nombre de 0 à 1999, et notamment de 49 à 149 et m pouvant désigner un nombre de 1 à 2000, et notamment de 1 à 10.

Un deuxième groupe de silicones aminées correspondant à la formule (B) est représentée par les silicones de formule (D) suivante : 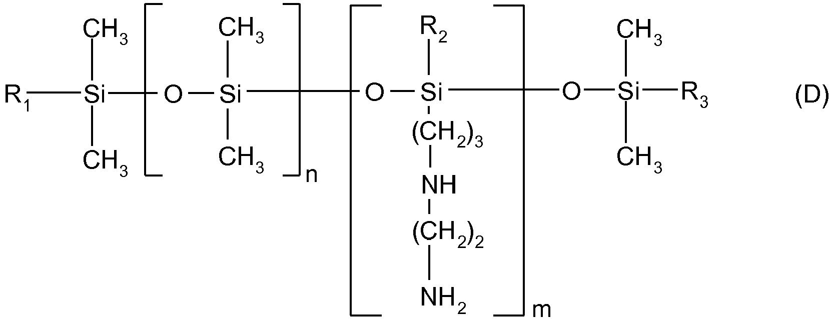 dans laquelle :
- m et n sont des nombres tels que la somme (n + m) varie de 1 à 1000, en particulier de 50 à 250 et plus particulièrement de 100 à 200; n pouvant désigner un nombre de 0 à 999 et notamment de 49 à 249 et plus particulièrement de 125 à 175 et m pouvant désigner un nombre de 1 à 1000, notamment de 1 à 10, plus particulièrement de 1 à 5;
- R1, R2, R3, identiques ou différents, représentent un radical hydroxy ou alcoxy en C₁-C₄, l'un au moins des radicaux R1 à R3 désignant un radical alcoxy.

De préférence le radical alcoxy est un radical méthoxy.
Le rapport molaire hydroxy/alcoxy va de préférence de 0,2:1 à 0,4:1 et de préférence de 0,25:1 à 0,35:1 et plus particulièrement est égal à 0,3:1.
La masse moléculaire moyenne en poids (Mw) de ces silicones va de préférence de 2000 à 1 000 000, plus particulièrement de 3500 à 200000.

Un troisième groupe de silicones aminées correspondant à la formule (B) est représentée par les silicones de formule (E) suivante : 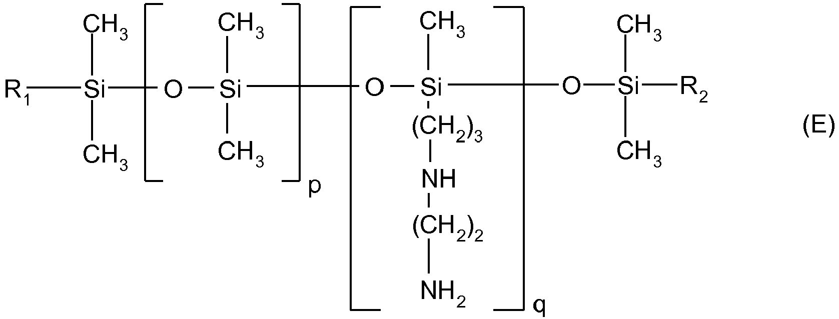 dans laquelle :
- p et q sont des nombres tels que la somme (p+q) varie de 1 à 1000, en particulier de 50 à 350, et plus particulièrement de 150 à 250; p pouvant désigner un nombre de 0 à 999 et notamment de 49 à 349 et plus particulièrement de 159 à 239 et q pouvant désigner un nombre de 1 à 1000, notamment de 1 à 10 et plus particulièrement de 1 à 5;
- R1, R2, différents, représentent un radical hydroxy ou alcoxy en C₁-C₄, l'un au moins des radicaux R1 ou R2 désignant un radical alcoxy.
   De préférence le radical alcoxy est un radical méthoxy.
   Le rapport molaire hydroxy/alcoxy va généralement de 1:0,8 à 1:1,1 et de préférence de 1:0,9 à 1:1 et plus particulièrement est égal à 1:0,95.
   La masse moléculaire moyenne en poids (Mw) de la silicone va de préférence de 2000 à 200000 et encore plus particulièrement de 5000 à 100000 et plus particulièrement de 10000 à 50000.

Les produits commerciaux comprenant des silicones de structure (D) ou (E) peuvent inclure dans leur composition une ou plusieurs autres silicones aminées dont la structure est différente des formules (D) ou (E).
Un produit contenant des silicones aminées de structure (D) est proposé par la société WACKER sous la dénomination BELSIL® ADM 652.
Un produit contenant des silicones aminées de structure (E) est proposé par WACKER sous la dénomination Fluid WR 1300®.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs. Les tensioactifs peuvent être de toute nature mais de préférence cationique et/ou non ionique. La taille moyenne en nombre des particules de silicone dans l'émulsion va généralement de 3 nm à 500 nanomètres. De préférence, notamment comme silicones aminées de formule (E), on utilise des microémulsions dont la taille moyenne des particules va de 5 nm à 60 nanomètres (bornes incluses) et plus particulièrement de 10 nm à 50 nanomètres (bornes incluses). Ainsi, on peut utiliser selon l'invention les microémulsions de silicone aminée de formule (E) proposées sous les dénominations FINISH CT 96 E® ou SLM 28020® par la société WACKER.

Un autre groupe de silicones aminées correspondant à la formule (B) est représenté par les silicones de formule suivante (F) : 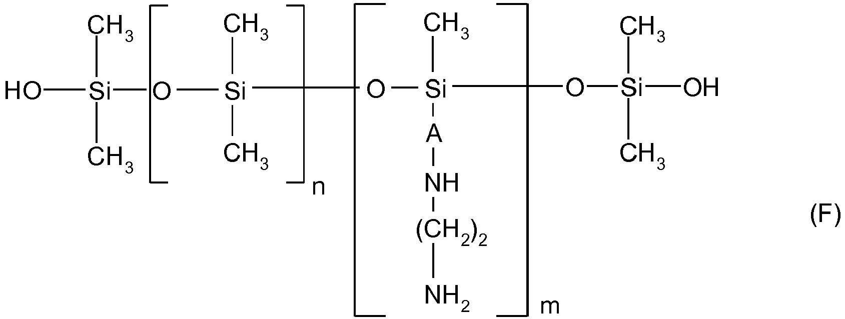 dans laquelle :
- m et n sont des nombres tels que la somme (n + m) varie de 1 à 2000 et en particulier de 50 à 150, n pouvant désigner un nombre de 0 à 1999 et notamment de 49 à 149 et m pouvant désigner un nombre de 1 à 2000, et notamment de 1 à 10;
- A désigne un radical alkylène linéaire ou ramifié ayant de 4 à 8 atomes de carbone et de préférence 4 atomes de carbone. Ce radical est de préférence linéaire. La masse moléculaire moyenne en poids (Mw) de ces silicones aminées va de préférence de 2000 à 1000000 et encore plus particulièrement de 3500 à 200000. Une silicone répondant à cette formule est par exemple la XIAMETER MEM 8299 EMULSION de DOW CORNING.

Un autre groupe de silicones aminées correspondant à la formule (B) est représenté par les silicones de formule suivante (G): 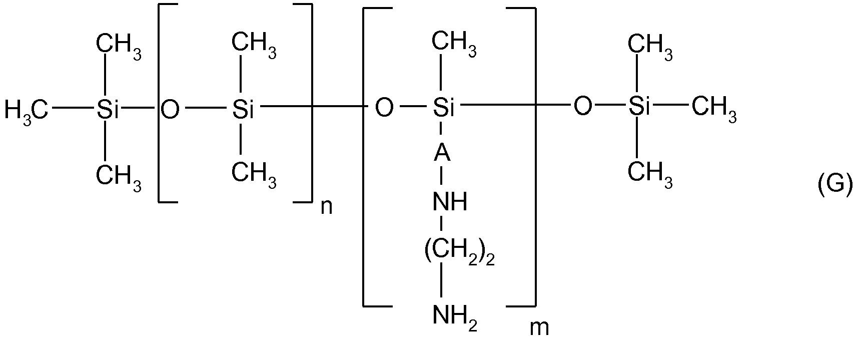 dans laquelle :
- m et n sont des nombres tels que la somme (n + m) varie de 1 à 2000 et en particulier de 50 à 150, n pouvant désigner un nombre de 0 à 1 999 et notamment de 49 à 149 et m pouvant désigner un nombre de 1 à 2000, et notamment de 1 à 10 ;
- A désigne un radical alkylène linéaire ou ramifié ayant de 4 à 8 atomes de carbone et de préférence 4 atomes de carbone. Ce radical est de préférence ramifié. La masse moléculaire moyenne en poids (Mw) de ces silicones aminées va de préférence de 500 à 1000000 et encore plus particulièrement de 1000 à 200.000.

Une silicone répondant à cette formule est par exemple la DC2-8566 Amino Fluid de DOW CORNING.
c) les silicones aminées répondant à la formule (H) : 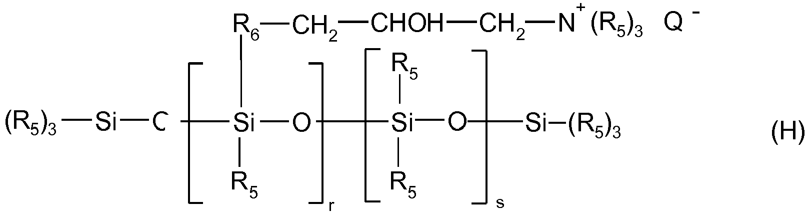 dans laquelle :
   - R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   - R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   - Q- est un anion tel qu'un ion halogénure, notamment chlorure ou un sel d'acide organique, notamment acétate;
   - r représente une valeur statistique moyenne allant de 2 à 20, en particulier de 2 à 8;
   - s représente une valeur statistique moyenne allant de 20 à 200, en particulier de 20 à 50.
   De telles silicones aminées sont notamment décrites dans le brevet US 4 185 087.
d) les silicones à ammonium quaternaire de formule (I) : 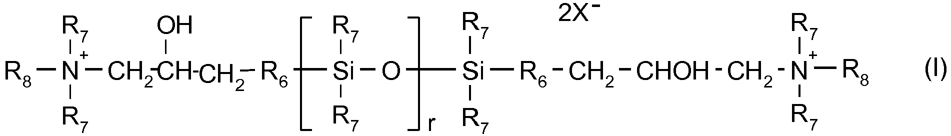 dans laquelle :
   - R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   - R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
   - R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   - X- est un anion tel qu'un ion halogénure, notamment chlorure ou un sel d'acide organique, notamment acétate;
   - r représente une valeur statistique moyenne allant de 2 à 200, en particulier de 5 à 100.
   Ces silicones sont par exemple décrites dans la demande EP-A-0530974.
e) les silicones aminées de formule (J) : 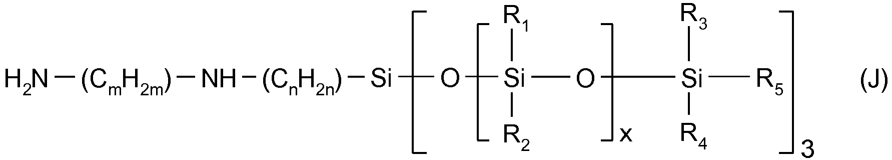 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5, et
   - x est choisi de manière telle que l'indice d'amine varie de 0,01 à 1 meq/g.
f) les silicones aminées polyoxyalkylénées multibloc, de type (AB)n, A étant un bloc polysiloxane et B étant un bloc polyoxyalkyléné comportant au moins un groupement amine.

Lesdites silicones sont de préférence constituées d'unités répétitives de formules générales suivantes :

[-(SiMe₂O)ₓSiMe₂-R-N(R")-R'-O(C₂H₄O)ₐ(C₃H₆O)_{b}-R'-N(H)-R-]

ou bien

[-(SiMe₂O)ₓSiMe₂-R-N(R")-R'-O(C₂H₄O)ₐ(C₃H₆O)_{b}-]

dans lesquelles :
- a est un nombre entier supérieur ou égal à 1, de préférence allant de 5 à 200, plus particulièrement allant de 10 à 100;
- b est un nombre entier compris entre 0 et 200, de préférence allant de 4 et 100, plus particulièrement entre 5 et 30;
- x est un nombre entier allant de 1 à 10000, plus particulièrement de 10 à 5000;
- R" est un atome d'hydrogène ou un méthyl;
- R, identiques ou différents, représentent un radical divalent hydrocarboné en C2-C12, linéaire ou ramifié, comportant éventuellement un ou plusieurs hétéroatomes tels que l'oxygène; de préférence, R désigne un radical éthylène, un radical propylène linéaire ou ramifié, un radical butylène linéaire ou ramifié, ou un cal -CH2CH2CH2OCH(OH)CH2-; préférentiellement R désigne un cal -CH2CH2CH2OCH(OH)CH2-; - R', identiques ou différents, représentent un radical divalent hydrocarboné en C2-C12, linéaire ou ramifié, comportant éventuellement un ou plusieurs hétéroatomes tels que l'oxygène; de préférence, R' désigne un radical éthylène, un radical propylène linéaire ou ramifié, un radical butylène linéaire ou ramifié, ou un cal -CH2CH2CH2OCH(OH)CH2-; préférentiellement R' désigne -CH(CH3)-CH2-.

Les blocs siloxane représentent de préférence 50 et 95% en moles du poids total de la silicone, plus particulièrement de 70 à 85% en moles.
Le taux d'amine est de préférence compris entre 0,02 et 0,5 meq/g de copolymère dans une solution à 30% dans le dipropylèneglycol, plus particulièrement entre 0,05 et 0,2.
La masse moléculaire moyenne en poids (Mw) de la silicone est de préférence comprise entre 5000 et 1000000, plus particulièrement entre 10000 et 200000.

On peut notamment citer les silicones commercialisées sous les dénominations SILSOFT A-843 ou SILSOFT A+ par Momentive.

La composition comprend de préférence une ou plusieurs silicones répondant à la formule (B), de préférence à l'une des formules (C), (D), (E), (F) et (G), et tout particulièrement répondant à la formule (F).

Ladite première composition selon l'invention comprend ladite ou lesdites silicones en une quantité allant de 0,1 à 2% en poids, de préférence de 0,2 à 1,7% en poids, préférentiellement de 0,3 à 1,6% en poids, par rapport au poids total de la composition.

Tout particulièrement, ladite première composition selon l'invention comprend ladite ou lesdites silicones aminées en une quantité allant de 0,1 à 2% en poids, de préférence de 0,2 à 1,7% en poids, préférentiellement de 0,3 à 1,6% en poids, par rapport au poids total de la composition.

### c/ Corps gras non siliconés

La première composition selon l'invention comprend également un ou plusieurs corps gras non siliconés, notamment un ou plusieurs corps gras non siliconés solides.

Par "corps gras", on entend un composé organique insoluble dans l'eau à température ambiante (25°C) et à pression atmosphérique (1 atm), c'est-à-dire de solubilité inférieure à 5% en poids, de préférence inférieure à 1% en poids. Ils sont généralement solubles, dans les mêmes conditions de température et de pression, dans des solvants organiques tels que le chloroforme, l'éthanol, le benzène, l'huile de vaseline ou le décaméthylcyclopentasiloxane.

Par "corps gras non siliconé", on entend un corps gras dont la structure ne comporte pas d'atome de silicium, donc ne comprenant pas notamment de groupe siloxane. Ils présentent généralement dans leur structure une chaîne hydrocarbonée comportant au moins 6 atomes de carbone. Avantageusement, ils ne sont pas oxyalkylénés et ne contiennent pas de fonction -COOH.

Par "corps gras solide", on entend un corps gras solide à température ambiante et à pression atmosphérique (25°C, 1 atm); ils présentent de préférence une viscosité supérieure à 2 Pa.s, mesurée à 25°C et à un taux de cisaillement de 1 s⁻¹.

Les corps gras non siliconés solides susceptibles d'être utilisés dans le cadre de l'invention peuvent être choisis parmi les alcools gras, les esters d'acide gras et/ou d'alcool gras, les cires non siliconées, les céramides et leurs mélanges.

Par « alcool gras », on entend un alcool aliphatique à longue chaine comprenant de 8 à 40 atomes de carbone, et comprenant au moins un groupe hydroxyle OH. Ces alcools gras ne sont ni oxyalkylénés, ni glycérolés.
Les alcools gras solides peuvent être saturés ou insaturés, linéaires ou ramifiés, et comportent de 8 à 40 atomes de carbone. De préférence, les alcools gras solides sont de structure R-OH avec R désignant un groupe alkyle linéaire, éventuellement substitué par un ou plusieurs groupes hydroxy, comprenant de 8 à 40, mieux de 10 à 30, voire de 12 à 24 atomes, encore mieux de 14 à 22 atomes de carbone.
Les alcools gras solides susceptibles d'être utilisés sont de préférence choisis parmi les (mono)alcools saturés ou insaturés, linéaires ou ramifiés, de préférence linéaires et saturés, comportant de 8 à 40 atomes de carbone, mieux de 10 à 30, voire de 12 à 24 atomes, encore mieux de 14 à 22 atomes de carbone.
Les alcools gras solides susceptibles d'être utilisés peuvent être choisis parmi, seul ou en mélange :
- l'alcool laurique ou laurylique (ou 1-dodécanol) ;
- l'alcool myristique ou myristylique (ou 1-tétradécanol) ;
- l'alcool cétylique (ou 1-hexadécanol) ;
- l'alcool stéarylique (ou 1-octadécanol) ;
- l'alcool arachidylique (ou 1-eicosanol) ;
- l'alcool béhénylique (ou 1-docosanol) ;
- l'alcool lignocérylique (ou 1-tetracosanol) ;
- l'alcool cérylique (ou 1-hexacosanol) ;
- l'alcool montanylique (ou 1-octacosanol) ;
- l'alcool myricylique (ou 1-triacontanol).
Préférentiellement, l'alcool gras solide est choisi parmi l'alcool cétylique, l'alcool stéarylique, l'alcool béhénylique, l'alcool myristique et leurs mélanges, tels que l'alcool cétylstéarylique ou cétéarylique.

Les esters d'acide gras et/ou d'alcool gras solides susceptibles d'être utilisés sont de préférence choisis parmi les esters issus d'acide gras carboxylique en C₉-C₂₆ et/ou d'alcool gras en C₉-C₂₆.
De préférence, ces esters gras solides sont des esters d'acide carboxylique saturé, linéaire ou ramifié, comportant au moins 10 atomes de carbone, de préférence de 10 à 30 atomes de carbone et plus particulièrement de 12 à 24 atomes de carbone, et de monoalcool saturé, linéaire ou ramifié, comportant au moins 10 atomes de carbone, de préférence de 10 à 30 atomes de carbone et plus particulièrement de 12 à 24 atomes de carbone. Les acides carboxyliques saturés peuvent être éventuellement hydroxylés, et sont de préférence des monoacides carboxyliques.
On peut également utiliser les esters d'acides di- ou tricarboxyliques en C₄-C₂₂ et d'alcools en C₁-C₂₂ et les esters d'acides mono-, di- ou tricarboxyliques et d'alcools di-, tri-, tétra- ou pentahydroxylés en C₂-C₂₆.
On peut notamment citer le béhénate d'octyldodécyle, le béhénate d'isocétyle, le lactate de cétyle, l'octanoate de stéaryle, l'octanoate d'octyle, l'octanoate de cétyle, l'oléate de décyle, le stéarate d'hexyle, le stéarate d'octyle, le stéarate de myristyle, le stéarate de cétyle, le stéarate de stéaryle, le pélargonate d'octyle, le myristate de cétyle, le myristate de myristyle, le myristate de stéaryle, le sébaçate de diéthyle, le sébaçate de diisopropyle, l'adipate de diisopropyle, l'adipate de di n-propyle, l'adipate de dioctyle, le maléate de dioctyle, le palmitate d'octyle, le palmitate de myristyle, le palmitate de cétyle, le palmitate de stéaryle, et leurs mélanges.
De préférence, les esters d'acide gras et/ou d'alcool gras solides sont choisis parmi les palmitates d'alkyle en C9-C26, notamment de myristyle, de cétyle, de stéaryle ; les myristates d'alkyle en C9-C26 tels que le myristate de cétyle, le myristate de stéaryle et le myristate de myristyle ; les stéarates d'alkyle en C9-C26, notamment les stéarates de myristyle, de cétyle et de stéaryle; et leurs mélanges.

Une cire, au sens de la présente invention, est un composé lipophile, solide à température ambiante (25°C) et pression atmosphérique, à changement d'état solide/liquide réversible, ayant une température de fusion supérieure à environ 40°C et pouvant aller jusqu'à 200°C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire, détectable microscopiquement et macroscopiquement (opalescence).

En particulier, les cires convenant à l'invention peuvent être choisies parmi les cires d'origine animale, végétale, minérale, les cires synthétiques non siliconées et leurs mélanges.
On peut notamment citer les cires hydrocarbonées, comme la cire d'abeille, notamment d'origine biologique, la cire de lanoline, et les cires d'insectes de Chine; la cire de son de riz, la cire de Carnauba, la cire de Candellila, la cire d'Ouricury, la cire d'Alfa, la cire de Berry, la cire de Shellac, la cire du Japon et la cire de sumac; la cire de Montan, les cires d'orange et de citron, les cires microcristallines, les paraffines et l'ozokérite; les cires de polyéthylène, les cires obtenues par la synthèse de Fisher-Tropsch et les copolymères cireux, ainsi que leurs esters.
On peut ainsi les cires microcristallines en C₂ à C₆₀, telles que la Microwax HW. On peut également citer la cire de polyéthylène PM 500 commercialisée sous la référence Permalen 50-L polyéthylène.
On peut aussi citer les cires obtenues par hydrogénation catalytique d'huiles animales ou végétales ayant des chaînes grasses, linéaires ou ramifiées, en C₈ à C₃₂. Parmi celles-ci, on peut notamment citer l'huile de jojoba isomérisée, telle que l'huile de jojoba partiellement hydrogénée isomérisée trans, notamment celle fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane), notamment celui vendu sous la dénomination de Hest 2T-4S® par la société HETERENE.
On peut également utiliser les cires obtenues par hydrogénation d'huile de ricin estérifiée avec l'alcool cétylique, telles que celles vendues sous les dénominations de Phytowax ricin 16L64® et 22L73® par la société SOPHIM.
Comme cire, on peut encore utiliser un (hydroxystéaryloxy)stéarate d'alkyle en C₂₀ à C₄₀ (le groupe alkyle comprenant de 20 à 40 atomes de carbone), seul ou en mélange. Une telle cire est notamment vendue sous les dénominations « Kester Wax K 82 P® », « Hydroxypolyester K 82 P® » et « Kester Wax K 80 P® » par la société KOSTER KEUNEN.
Il est également possible d'utiliser des microcires dans les compositions de l'invention ; on peut citer notamment les microcires de carnauba, telles que celle commercialisée sous la dénomination MicroCare 350® par la société MICRO POWDERS, les microcires de cire synthétique, telles que celle commercialisée sous la dénomination MicroEase 114S® par la société MICRO POWDERS, les microcires constituées d'un mélange de cire de carnauba et de cire de polyéthylène, telles que celles commercialisées sous les dénominations de Micro Care 300® et 310® par la société MICRO POWDERS, les microcires constituées d'un mélange de cire de carnauba et de cire synthétique, telles que celle commercialisée sous la dénomination Micro Care 325® par la société MICRO POWDERS, les microcires de polyéthylène, telles que celles commercialisées sous les dénominations de Micropoly 200®, 220®, 220L® et 250S® par la société MICRO POWDERS et les microcires de polytétrafluoroéthylène, telles que celles commercialisées sous les dénominations de Microslip 519® et 519 L® par la société
MICRO POWDERS.
Les cires sont de préférence choisies parmi les cires minérales comme la cire de paraffine, de vaseline, de lignite ou l'ozokérite; les cires végétales comme le beurre de cacao ou les cires de fibres de liège ou de canne à sucre, la cire d'olivier, la cire de riz, la cire de jojoba hydrogénée, la cire d'Ouricoury, la cire de Carnauba, la cire de Candelila, la cire d'Alfa, ou les cires absolues de fleurs telles que la cire essentielle de fleur de cassis vendue par la société BERTIN (France); les cires d'origine animale comme les cires d'abeilles ou les cires d'abeilles modifiées (cerabellina), le spermaceti, la cire de lanoline et les dérivés de lanoline; les cires microcristallines ; et leurs mélanges.

Les céramides ou analogues de céramides tels que les glycocéramides, susceptibles d'être utilisés dans les compositions selon l'invention, sont connus; on peut citer en particulier les céramides des classes I, II, III et V selon la classification de DAWNING.

Les céramides ou leurs analogues susceptibles d'être employés répondent de préférence à la formule suivante : 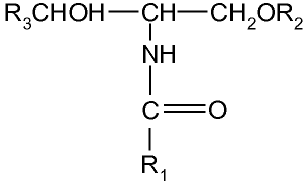 dans laquelle :
- R₁ désigne un groupe alkyle, linéaire ou ramifié, saturé ou insaturé, dérivé d'acides gras en C₁₄-C₃₀, ce groupe pouvant être substitué par un groupement hydroxyle en position alpha, ou un groupement hydroxyle en position oméga estérifié par un acide gras saturé ou insaturé en C₁₆-C₃₀ ;
- R₂ désigne un atome d'hydrogène, un groupe (glycosyle)n, un groupe (galactosyle)m ou un groupe sulfogalactosyle, dans lesquels n est un entier variant de 1 à 4 et m est un entier variant de 1 à 8 ;
- R₃ désigne un groupe hydrocarboné en C₁₅-C₂₆, saturé ou insaturé en position alpha, ce groupe pouvant être substitué par un ou plusieurs groupes alkyle en C₁-C₁₄ ;
   étant entendu que dans le cas des céramides ou glycocéramides naturelles, R₃ peut également désigner un groupe alpha-hydroxyalkyle en C₁₅-C₂₆, le groupement hydroxyle étant éventuellement estérifié par un alpha-hydroxyacide en C₁₆-C₃₀.

Les céramides plus particulièrement préférés sont les composés pour lesquels R₁ désigne un alkyle saturé ou insaturé dérivé d'acides gras en C₁₆-C₂₂; R₂ désigne un atome d'hydrogène et R₃ désigne un groupe linéaire saturé en C₁₅. Préférentiellement, on utilise les céramides pour lesquels R₁ désigne un groupe alkyle saturé ou insaturé dérivé d'acides gras en C14-C30; R₂ désigne un groupe galactosyle ou sulfogalactosyle; et R₃ désigne un groupement -CH=CH-(CH₂)₁₂-CH₃.
On peut également utiliser les composés pour lesquels R1 désigne un radical alkyle saturé ou insaturé dérivé d'acides gras en C12-C22; R2 désigne un radical galactosyle ou sulfogalactosyle et R3 désigne un radical hydrocarboné en C12-C22, saturé ou insaturé et de préférence un groupement -CH=CH-(CH2)12-CH3. Comme composés particulièrement préférés, on peut citer également le 2-N-linoléoylamino-octadécane-1,3-diol; le 2-N-oléoylamino-octadécane-1,3-diol; le 2-N-palmitoylamino-octadécane-1,3-diol; le 2-N-stéaroylamino-octadécane-1,3-diol; le 2-N-béhénoylamino-octadécane-1,3-diol; le 2-N-[2-hydroxy-palmitoyl]-amino-octadécane-1,3-diol; le 2-N-stéaroyl amino-octadécane-1,3,4 triol et en particulier la N-stéaroyl phytosphingosine; le 2-N-palmitoylamino-hexadécane-1,3-diol, la N-linoléoyldihydrosphingosine, la N-oléoyldihydrosphingosine, la N-palmitoyldihydrosphingosine, la N-stéaroyldihydrosphingosine, et la N-béhénoyldihydrosphingosine, le N-docosanoyl N-méthyl-D-glucamine, le N-(2-hydroxyéthyl)-N-(3-cétyloxy-2-hydroxypropyl)amide d'acide cétylique et le bis-(N-hydroxyéthyl N-cétyl)malonamide; et leurs mélanges. De préférence, on utilise la N-oléoyldihydrosphingosine.

De préférence, ladite première composition selon l'invention comprend ledit ou lesdits corps gras non siliconés en une quantité allant de 0,1 à 20% en poids, de préférence de 0,5 à 15% en poids, préférentiellement de 1 à 10% en poids, par rapport au poids total de la composition.

Tout particulièrement, ladite première composition selon l'invention comprend ledit ou lesdits corps gras non siliconés solides en une quantité allant de 0,5 à 15% en poids, de préférence de 1 à 10% en poids, préférentiellement de 1,5 à 8% en poids, par rapport au poids total de la composition.

### d/ Autres ingrédients

Ladite première composition peut également comprendre de l'eau ou d'un mélange d'eau et d'un ou plusieurs solvants cosmétiquement acceptables choisis parmi les monoalcools en C₁ à C₄ tels que l'éthanol, l'isopropanol, le tertio-butanol ou le n-butanol; les polyols tels que le glycérol, le propylèneglycol et les polyéthylèneglycols ; et leurs mélanges.
De préférence, ladite première composition comprend de l'eau à une concentration allant de préférence de 50 à 99% en poids, notamment de 60 à 98% en poids, mieux de 70 à 97% en poids, par rapport au poids total de ladite composition. Ladite première composition peut éventuellement comprendre en outre un ou plusieurs additifs additionnels usuellement employés en cosmétique.
Le pH de la composition, si elle est aqueuse, est de préférence compris entre 3 et 7, notamment entre 3 et 5,5.

### Deuxième composition

Le procédé de traitement cosmétique selon l'invention comprend également une deuxième étape consistant en l'application sur les cheveux d'une composition cosmétique (ou deuxième composition) comprenant un ou plusieurs tensioactifs cationiques, un ou plusieurs polymères cationiques présentant une densité de charge cationique supérieure ou égale à 4 méq/g, et un ou plusieurs organosilanes.

### a/ Tensioactifs cationiques

Ladite deuxième composition cosmétique comprend donc un ou plusieurs tensioactifs cationiques.
La description des tensioactifs cationiques susceptibles d'être employés dans ladite deuxième composition étant identique à la description donnée ci-dessus, pour les tensioactifs cationiques susceptibles d'être employés dans la première composition cosmétique, cette description ne sera pas répétée ici.

De préférence, les tensioactifs cationiques susceptibles d'être utilisés dans la deuxième composition selon l'invention sont choisis parmi ceux de formule (la) ou (IVa), et mieux parmi les sels de cétyltriméthylammonium, de béhényltriméthylammonium, de dipalmitoyléthylhydroxyéthylméthylammonium et leurs mélanges; et plus particulièrement parmi le chlorure ou le méthosulfate de béhényltriméthylammonium, le chlorure ou le méthosulfate de cétyltriméthylammonium, le chlorure ou le méthosulfate de dipalmitoyléthylhydroxyéthylméthylammonium et leurs mélanges.

De préférence, ladite deuxième composition cosmétique comprend le ou lesdits tensioactifs cationiques en une quantité allant de 0,05 à 10% en poids, de préférence de 0,1 à 5% en poids, préférentiellement de 0,3 à 3% en poids, par rapport au poids total de la composition.

### b/ Organosilanes

Ladite deuxième composition comprend un ou plusieurs organosilanes, de préférence choisis parmi les composés de formule (I) et/ou leurs oligomères :

RiSi(OR₂)_{z}(R₃)ₓ(OH)_{y} (I)

dans laquelle :
- R₁ est une chaîne hydrocarbonée en C₁ à C₂₂, notamment en C₂ à C₂₀, linéaire ou ramifiée, saturée ou insaturée, cyclique ou acyclique, pouvant être substituée par un groupement choisi parmi les groupements amine NH₂ ou NHR (R étant un alkyle linéaire ou ramifié en C₁ à C₂₀, notamment en C₁ à C₆, ou un cycloalkyle en C₃ à C₄₀ ou un radical aromatique en C₆ à C₃₀); le groupement hydroxy (OH) ; un groupement thiol ; un groupement aryle (plus particulièrement benzyle) substitué ou non par un groupement NH₂ ou NHR; R₁ pouvant être interrompu par un hétéroatome (O, S, NH) ou un groupement carbonyle (CO);
- R₂ et R₃ identiques ou différents, représentent un groupe alkyle, linéaire ou ramifié, comprenant de 1 à 6 atomes de carbone,
- y désigne un nombre entier allant de 0 à 3,
- z désigne un nombre entier allant de 0 à 3, et
- x désigne un nombre entier allant de 0 à 2,
- avec z+x+y=3.

Par oligomère, on entend les produits de polymérisation des composés de formule (I) comportant de 2 à 10 atomes de silicium.

De préférence, R₁ est une chaîne hydrocarbonée linéaire ou ramifiée, saturée, en C₁ à C₂₂, notamment C₂ à C₁₂, de préférence linéaire, pouvant être substituée par un groupement amine NH₂ ou NHR (R= alkyle en C₁ à C₂₀, notamment en C₁ à C₆). De préférence, R₂ représente un groupe alkyle comprenant de 1 à 4 atomes de carbone, mieux un groupe alkyle linéaire comprenant de 1 à 4 atomes de carbone, et de préférence le groupe éthyle.
De préférence, z varie de 1 à 3.
De préférence, y=0.
Préférentiellement, z = 3 et donc x=y=0.

Dans un mode de réalisation de l'invention, le ou les organosilanes sont choisis parmi les composés de formule (I) dans laquelle R₁ représente un groupe alkyle linéaire, comprenant de 7 à 18 atomes de carbone et plus particulièrement de 7 à 12 atomes de carbone ou un groupe aminoalkyle en C₁ à C₆, de préférence en C₂ à C₄. Plus particulièrement, R₁ représente un groupe octyle.
Dans un autre mode de réalisation de l'invention, le ou les organosilanes sont choisis parmi les composés de formule (I) dans laquelle R₁ est une chaîne hydrocarbonée en C₁ à C₂₂, linéaire ou ramifiée, saturée ou insaturée, substituée par un groupement amine NH₂ ou NHR (avec R= alkyle en C₁ à C₂₀, notamment C₁ à C₆, cycloalkyle en C₃ à C₄₀ ou aromatique en C₆ à C₃₀). Dans cette variante, R₁ représente de préférence un groupe aminoalkyle en C₁ à C₆, et plus préférentiellement en C₂ à C₄.
De préférence, la deuxième composition selon l'invention comprend un ou plusieurs organosilanes choisis parmi l'octyltriéthoxysilane (OTES), le dodécyltriéthoxysilane, l'octadécyltriéthoxysilane, l'hexadécyltriéthoxysilane, le 3-aminopropyl triéthoxysilane (APTES); le 2-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane, leurs oligomères et leurs mélanges; et plus particulièrement choisis parmi l'octyltriéthoxysilane (OTES), le 3-aminopropyltriéthoxysilane (APTES), leurs oligomères et leurs mélanges.
Les organosilanes utilisés dans la composition de l'invention, notamment ceux comportant une fonction basique, peuvent être neutralisés, partiellement ou totalement, afin d'améliorer leur solubilité dans l'eau. En particulier, l'agent de neutralisation peut être choisi parmi les acides organiques ou minéraux, tels que l'acide citrique, l'acide tartrique, l'acide lactique, l'acide chlorhydrique. De préférence, les organosilanes selon l'invention, éventuellement neutralisés, sont solubles dans l'eau et notamment solubles à la concentration de 2% en poids, mieux à la concentration de 5% en poids et encore mieux à la concentration de 10% en poids dans l'eau à la température de 25°C, et à la pression atmosphérique (1 atm.). Par soluble, on entend la formation d'une phase macroscopique unique.

De préférence, ladite deuxième composition cosmétique comprend le ou lesdits organosilanes en une quantité allant de 0,1 à 15% en poids, de préférence de 1 à 10% en poids, préférentiellement de 2 à 8% en poids, par rapport au poids total de la composition.

### c/ Polymères cationiques de densité de charge particulière

Ladite deuxième composition selon l'invention comprend en outre un ou plusieurs polymères cationiques ayant une densité de charge supérieure ou égale à 4 méq/g (milliéquivalents par gramme), de préférence de densité de charge cationique supérieure ou égale à 5 méq/g ; notamment de densité de charge cationique allant de 4 à 20 méq/g, voire de 5 à 20 méq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl.

Les polymères cationiques ayant une densité de charge cationique supérieure ou égale à 4 méq/g, utilisables conformément à la présente invention, peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.
De manière générale, au sens de la présente invention, l'expression "polymère cationique" désigne tout polymère contenant des groupes cationiques et/ou des groupes ionisables en groupes cationiques.

Les polymères cationiques utilisés dans la présente invention ont de préférence un poids moléculaire moyen en nombre (Mn) supérieur ou égal à 50 000 g/mol, préférentiellement supérieur ou égal à 100 000 g/mol.

Les polymères cationiques susceptibles d'être utilisés selon la présente invention sont avantageusement choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci. Plus particulièrement, les polymères cationiques peuvent être choisis parmi les polymères du type polyamine, polyaminoamide et polyammonium quaternaire, les polyalkylèneimines et leurs mélanges.

Parmi les polymères cationiques susceptibles d'être employés, on peut citer plus particulièrement :
(1) les homopolymères ou copolymères dérivés d'esters ou d'amides acryliques ou méthacryliques et comportant au moins un des motifs de formule suivante : 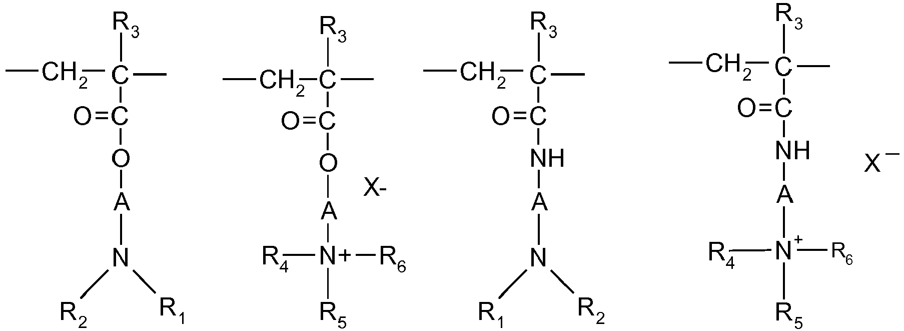 dans lesquelles:
   - R3, identiques ou différents, désignent un atome d'hydrogène ou un radical CH3;
   - A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
   - R4, R5, R6, identiques ou différents, représentent un groupe alkyle ayant de 1 à 18 atomes de carbone ou un radical benzyle; de préférence un groupe alkyle ayant de 1 à 6 atomes de carbone;
   - R1 et R2, identiques ou différents, représentent un atome d'hydrogène ou un groupe alkyle ayant de 1 à 6 atomes de carbone, de préférence méthyle ou éthyle;
   - X désigne un anion dérivé d'un acide minéral ou organique tel qu'un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Parmi ces copolymères de la famille (1), on peut citer :
   - les copolymères d'acrylamide et de diméthylaminoéthyl méthacrylate quaternisé au sulfate de diméthyle ou avec un halogénure de diméthyle, tels que celui vendu sous la dénomination HERCOFLOC par la société HERCULES,
   - les copolymères d'acrylamide et de chlorure de méthacryloyloxyéthyltriméthylammonium, tels que ceux vendus sous la dénomination BINA QUAT P 100 par la société CIBA GEIGY,
   - le copolymère d'acrylamide et de méthosulfate de méthacryloyloxyéthyltriméthylammonium, tel que celui vendu sous la dénomination RETEN par la société HERCULES,
   - les copolymères vinylpyrrolidone/acrylate ou méthacrylate de dialkylaminoalkyle, quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573 ;
   - les terpolymères méthacrylate de diméthylaminoéthyle/ vinylcaprolactame/ vinylpyrrolidone, tel que le produit vendu sous la dénomination GAFFIX VC 713 par la société ISP,
   - les copolymères vinylpyrrolidone/ méthacrylamidopropyldimethylamine, tels que ceux commercialisés sous la dénomination STYLEZE CC 10 par ISP;
   - les copolymères vinylpyrrolidone/ méthacrylamide de diméthylaminopropyle quaternisés, tel que le produit vendu sous la dénomination "GAFQUAT HS 100" par la société ISP,
   - les polymères, de préférence réticulés, de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo- ou la copolymérisation étant suivie d'une réticulation par un composé à insaturation oléfinique, en particulier le méthylène bisacrylamide. On peut plus particulièrement utiliser un copolymère réticulé acrylamide/chlorure de méthacryloyloxyéthyltriméthylammonium (20/80 en poids) sous forme de dispersion comprenant 50% en poids dudit copolymère dans de l'huile minérale. Cette dispersion est commercialisée sous le nom de "SALCARE® SC 92" par la société CIBA. On peut également utiliser un homopolymère réticulé du chlorure de méthacryloyloxyéthyltriméthylammonium comprenant environ 50% en poids de l'homopolymère dans de l'huile minérale ou dans un ester liquide. Ces dispersions sont commercialisées sous les noms "SALCARE® SC 95" et "SALCARE® SC 96" par la société CIBA.
(2) les polymères constitués de motifs pipérazinyle et de radicaux divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères.
(3) les polyaminoamides solubles dans l'eau, préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés.
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone; le rapport molaire entre le polyalkylène polyamine et l'acide dicarboxylique étant de préférence compris entre 0,8:1 et 1,4:1; le polyaminoamide en résultant étant amené à réagir avec l'épichlorhydrine dans un rapport molaire d'épichlorhydrine par rapport au groupement amine secondaire du polyaminoamide compris de préférence entre 0,5:1 et 1,8:1. Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyldiallylamine ou de dialkyldiallylammonium tels que les homopolymères ou copolymères comportant comme constituant principal de la chaîne des motifs répondant aux formules (I) ou (II) : 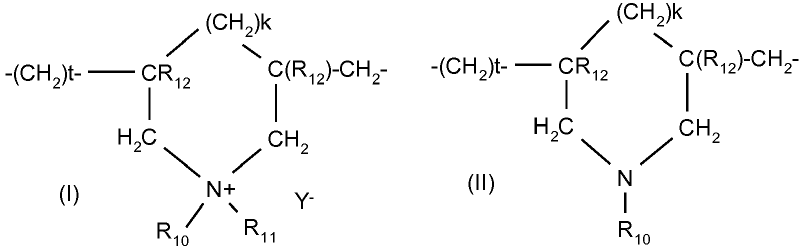 dans lesquelles
   - k et t sont égaux à 0 ou 1, la somme k + t étant égale à 1 ;
   - R12 désigne un atome d'hydrogène ou un radical méthyle ;
   - R10 et R11, indépendamment l'un de l'autre, désignent un groupement alkyle en C₁-C₆, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11, indépendamment l'un de l'autre, désignent de préférence un groupement alkyle en C1-C4;
   - Y⁻ est un anion tel que bromure, chlorure, acétate, borate, citrate, tartrate, bisulfate, bisulfite, sulfate, phosphate.
   On peut citer plus particulièrement l'homopolymère de sels (par exemple chlorure) de diméthyldiallylammonium par exemple vendu sous la dénomination "MERQUAT 100" par la société NALCO, et les copolymères de sels (par exemple chlorure) de diallyldiméthylammonium et d'acrylamide commercialisés notamment sous la dénomination "MERQUAT 550" ou "MERQUAT 7SPR".
(7) les polymères de diammonium quaternaire comprenant des motifs récurrents de formule : 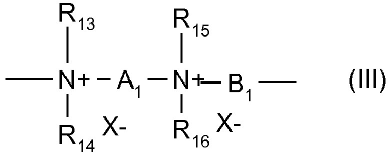 dans laquelle :
   - R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12,
      ou bien R13, R14, R15 et R16, ensemble ou séparément, constituent avec les atomes d'azote auxquels ils sont rattachés des hétérocycles comprenant éventuellement un second hétéroatome autre que l'azote
      ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène notamment en C1-C6 et D un groupement ammonium quaternaire ;
   - A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   - X⁻ désigne un anion dérivé d'un acide minéral ou organique;
      étant entendu que A1, R13 et R15 peuvent former avec les deux atomes d'azote auxquels ils sont rattachés un cycle pipérazinique ;
      en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désigne :
      a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH- où Y désigne un radical hydrocarboné linéaire ou ramifié, ou bien le radical divalent -CH2-CH2-S-S-CH2-CH2- ;
      d) un groupement uréylène de formule -NH-CO-NH- .
   De préférence, X⁻ est un anion tel que le chlorure ou le bromure. Ces polymères ont une masse molaire moyenne en nombre (Mn) généralement comprise entre 1000 et 100000.
   On peut citer plus particulièrement les polymères qui sont constitués de motifs récurrents répondant à la formule : 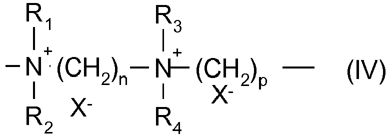 dans laquelle R1, R2, R3 et R4, identiques ou différents, désignent un radical alkyle ou hydroxyalkyle ayant de 1 à 4 atomes de carbone, n et p sont des nombres entiers variant de 2 à 20, et X- est un anion dérivé d'un acide minéral ou organique.
   Un composé de formule (IV) particulièrement préféré est celui pour lequel R1, R2, R3 et R4 représentent un radical méthyle, n=3, p=6 et X = Cl, dénommé Hexadimethrine chloride selon la nomenclature INCI (CTFA).
(8) les polymères de polyammonium quaternaires comprenant des motifs de formule (V): 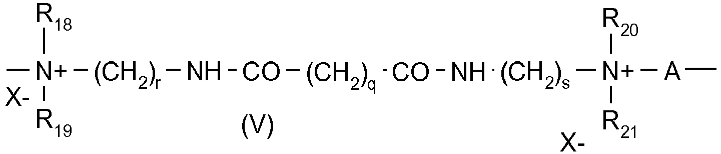 dans laquelle :
   - R18, R19, R20 et R21, identiques ou différents, représentent un atome d'hydrogène ou un radical méthyle, éthyle, propyle, β-hydroxyéthyle, β-hydroxypropyle ou
   - CH2CH2(OCH2CH2)pOH, où p est égal à 0 ou à un nombre entier compris entre 1 et 6, sous réserve que R18, R19, R20 et R21 ne représentent pas simultanément un atome d'hydrogène,
   - r et s, identiques ou différents, sont des nombres entiers compris entre 1 et 6,
   - q est égal à 0 ou à un nombre entier compris entre 1 et 34,
   - X- désigne un anion tel qu'un halogénure,
   - A désigne un radical divalent d'un dihalogénure ou représente de rence -CH2-CH2-O-CH2-CH2-.
   On peut par exemple citer les produits "Mirapol® A 15", "Mirapol® AD1", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) les polymères comportant dans leur structure :
   (a) un ou plusieurs motifs répondant à la formule (A) suivante : 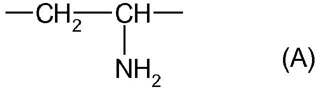
   (b) éventuellement un ou plusieurs motifs répondant à la formule (B) suivante : 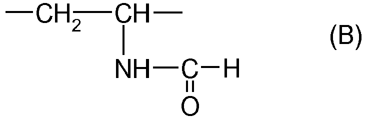 Autrement dit, ces polymères peuvent être notamment choisis parmi les homo- ou copolymères comportant un ou plusieurs motifs issus de la vinylamine et éventuellement un ou plusieurs motifs issus du vinylformamide.
      De préférence, ces polymères cationiques sont choisis parmi les polymères comportant, dans leur structure, de 5 à 100% en moles de motifs répondant à la formule (A) et de 0 à 95% en moles de motifs répondant à la formule (B), préférentiellement de 10 à 100% en moles de motifs répondant à la formule (A) et de 0 à 90% en moles de motifs répondant à la formule (B).
      Ces polymères peuvent être obtenus par exemple par hydrolyse partielle du polyvinylformamide. Cette hydrolyse peut se faire en milieu acide ou basique.
      La masse moléculaire moyenne en poids dudit polymère, mesurée par diffraction de la lumière, peut varier de 1000 à 3.000.000 g/mole, de préférence de 10 000 à 1.000.000 et plus particulièrement de 100 000 à 500.000 g/mole.
      Les polymères comportant des motifs de formule (A) et éventuellement des motifs de formule (B) sont notamment vendus sous la dénomination LUPAMIN par la société BASF, tels que par exemple, et de manière non limitative, les produits proposés sous la dénomination LUPAMIN 9095, LUPAMIN 5095, LUPAMIN 1095, LUPAMIN 9030 (ou LUVIQUAT 9030) et LUPAMIN 9010.

D'autres polymères cationiques utilisables dans le cadre de l'invention sont des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi ceux des familles (1), (6) et (7) ci-dessus citées.
On peut utiliser de préférence, seul ou en mélange :
- les cyclopolymères d'alkyldiallylamine ou de dialkyldiallylammonium, en particulier les homopolymères ou copolymères de sels (par exemple chlorure) de diméthyldiallylammonium, vendus sous les dénominations MERQUAT 100, MERQUAT 550 et MERQUAT S par la société NALCO,
- les homopolymères ou copolymères éventuellement réticulés de sels de méthacryloyloxyalkyl(C1-C4) trialkyl(C1-C4)ammonium;
- les polymères de diammonium quaternaire comprenant des motifs récurrents de formule (III) ;
- les homopolymères ou copolymères dérivés des acides acrylique ou méthacrylique et comportant des motifs ci-après, tels que définis ci-dessus :

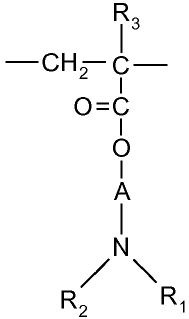

Encore mieux, le ou les polymères cationiques ayant une densité de charge supérieure ou égale à 4 méq/g sont choisis parmi le chlorure de 2-méthacryloyloxyéthyl triméthyl ammonium (Polyquaternium-37), le chlorure de diméthyl diallyl ammonium (Polyquaternium-6) et leurs mélanges.

De préférence, ladite deuxième composition cosmétique comprend le ou lesdits polymères cationiques ayant une densité de charge supérieure ou égale à 4 méq/g, en une quantité allant de 0,01 à 15% en poids, de préférence de 0,1 à 10% en poids, préférentiellement de 0,2 à 5% en poids, par rapport au poids total de la composition.

### d/ Autres ingrédients

Ladite deuxième composition peut également comprendre de l'eau ou d'un mélange d'eau et d'un ou plusieurs solvants cosmétiquement acceptables choisis parmi les monoalcools en C₁ à C₄ tels que l'éthanol, l'isopropanol, le tertio-butanol ou le n-butanol; les polyols tels que le glycérol, le propylèneglycol et les polyéthylèneglycols ; et leurs mélanges.
De préférence, ladite deuxième composition comprend de l'eau à une concentration allant de préférence de 40 à 99% en poids, notamment de 50 à 95% en poids, mieux de 60 à 90% en poids, par rapport au poids total de ladite composition.

Le pH de la composition, si elle est aqueuse, est de préférence compris entre 3,5 et 7, notamment entre 4 et 6.

Ladite deuxième composition peut éventuellement comprendre en outre un ou plusieurs corps gras, liquides ou solides, siliconés ou non.
De préférence, les corps gras utilisables dans la composition selon l'invention sont non siliconés.
De préférence, le ou les corps gras sont choisis parmi les alcools gras, liquides ou solides; les esters gras, liquides ou solides; les huiles d'origine minérale, les huiles végétales de type triglycérides et leurs mélanges.
La teneur du ou des corps gras, lorsqu'ils sont présents dans la composition selon l'invention, peut aller de 1 à 30% en poids, de préférence, de 5 à 25% en poids, et plus préférentiellement de 10 à 20% en poids, par rapport au poids total de la composition.

La deuxième composition peut éventuellement comprendre en outre un ou plusieurs acides organiques, de préférence choisis parmi les acides carboxyliques, saturés ou insaturés ; les acides sulfoniques et leurs mélanges.
De préférence, le ou les acides organiques sont choisis parmi les acides carboxyliques et notamment l'acide lactique, l'acide propanoïque, l'acide butanoïque, l'acide acétique, l'acide citrique, l'acide maléique, l'acide glycolique, l'acide salicylique, l'acide malique, l'acide tartrique et leurs mélanges, et plus préférentiellement l'acide lactique.
La teneur du ou des acides organiques, lorsqu'ils sont présents dans la composition selon l'invention peut aller de 0,1 à 10% en poids, et de préférence de 0,5 à 5% en poids, par rapport au poids total de la composition.

Ladite deuxième composition peut éventuellement comprendre en outre un ou plusieurs additifs additionnels usuellement employés en cosmétique.

### Composition optionnelle préalable

Ainsi que mentionné plus haut, dans un mode de réalisation particulier de l'invention, il est possible de prévoir, antérieurement à ladite première étape du procédé, une étape dite de lavage des cheveux, comprenant l'application sur lesdits cheveux d'une composition lavante, par exemple de type shampoing, comprenant de préférence un ou plusieurs tensioactifs notamment choisis parmi les tensioactifs anioniques, les tensioactifs amphotères, et leurs mélanges.
De préférence, ladite étape antérieure de lavage peut être suivie d'une étape de rinçage, par exemple à l'eau, avant mise en oeuvre du procédé selon l'invention.

Lesdits tensioactifs notamment choisis parmi les tensioactifs anioniques, les tensioactifs amphotères, et leurs mélanges, peuvent être présents dans la composition lavante en une teneur totale qui peut aller de 5 à 35% en poids, de préférence de 10 à 30% en poids, préférentiellement de 15 à 25% en poids, par rapport au poids total de la composition.

Lesdits tensioactifs anioniques peuvent être choisis les tensioactifs sulfates, sulfonates et/ carboxyliques (ou carboxylates). On peut bien évidemment employer un mélange de ces tensioactifs.
Les tensioactifs anioniques carboxyliques susceptibles d'être utilisés comportent donc au moins une fonction carboxylique ou carboxylate (-COOH ou -COO⁻).
Ils peuvent être choisis parmi les composés suivants : les acylglycinates, les acyllactylates, les acylsarcosinates, les acylglutamates ; les acides alkyl-D-galactoside-uroniques, les acides alkyléthercarboxyliques, les acides alkyl(aryl en C6-30)éthercarboxyliques, les acides alkylamidoéthercarboxyliques ; ainsi que les sels de ces composés;
les groupes alkyle et/ou acyle de ces composés comportant de 6 à 30 atomes de carbone, notamment de 12 à 28, encore mieux de 14 à 24, voire de 16 à 22, atomes de carbone ; le groupe aryle désignant de préférence un groupe phényle ou benzyle ;
ces composés pouvant être polyoxyalkylénés, notamment polyoxyéthylénés et comportant alors de préférence de 1 à 50 motifs oxyde d'éthylène, mieux de 2 à 10 motifs oxyde d'éthylène.
On peut également utiliser les monoesters d'alkyle en C6-C24 et d'acides polyglycoside-polycarboxyliques tels que les polyglycoside-citrates d'alkyle en C6-C24, les polyglycoside-tartrates d'alkyle en C6-C24 et les polyglycoside-sulfosuccinates d'alkyle en C6-C24, et leurs sels.
Parmi les tensioactifs carboxyliques ci-dessus, on peut tout particulièrement citer les acides alkyl(amido)éther carboxyliques polyoxyalkylénés et leurs sels, en particulier ceux comportant de 2 à 50 groupements oxyde d'alkylène, en particulier d'éthylène, tels que les composés proposés par la société KAO sous les dénominations AKYPO,
Les acides alkyl(amido)éther carboxyliques polyoxyalkylénés susceptibles d'être utilisés sont de préférence choisis parmi ceux de formule (1) : 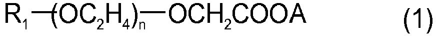 dans laquelle :
- R1 représente un radical alkyle ou alcényle linéaire ou ramifié en C6-C24, un radical alkyl(C8-C9)phényle, un radical R2CONH-CH2-CH2- avec R2 désignant un radical alkyle ou alcényle linéaire ou ramifié en C9-C21;
   de préférence R1 est un radical alkyle en C8-C20, de préférence en C8-C18, et aryle désigne de préférence phényle,
- n est un nombre entier ou décimal (valeur moyenne) variant de 2 à 24, de préférence de 2 à 10,
- A désigne H, ammonium, Na, K, Li, Mg ou un reste monoéthanolamine ou triéthanolamine.
On peut également utiliser des mélanges de composés de formule (1), en particulier des mélanges de composés ayant des groupements R1 différents.
Les acides alkyl(amido)éther carboxyliques polyoxyalkylénés particulièrement préférés sont ceux de formule (1) dans laquelle :
- R1 désigne un radical alkyl en C12-C14, cocoyle, oléyle, nonylphényle ou octylphényle,
- A désigne un atome d'hydrogène ou de sodium, et
- n varie de 2 à 20, de préférence 2 à 10.
Plus préférentiellement encore, on utilise des composés de formule (1) dans laquelle R désigne un radical alkyl en C12, A désigne un atome d'hydrogène ou de sodium et n varie de 2 à 10.
Préférentiellement, les tensioactifs anioniques carboxyliques sont choisis parmi, seuls ou en mélange :
- les acylglutamates notamment en C6-C24, voire en C12-C20, tels que les stéaroylglutamates, et en particulier le stéaroylglutamate de disodium ;
- les acylsarcosinates notamment en C6-C24, voire en C12-C20, tels que les palmitoylsarcosinates, et en particulier le palmitoylsarcosinate de sodium ;
- les acyllactylates notamment en C12-C28, voire en C14-C24, tels que les béhénoyllactylates, et en particulier le behenoyllactylate de sodium ;
- les acylglycinates en C6-C24, notamment en C12-C20;
- les alkyl(C6-C24)éthercarboxylates, et notamment les alkyl(C12-C20) éthercarboxylates ;
- les acides alkyl(C₆-C₂₄) (amido) éther carboxyliques polyoxyalkylénés, en particulier ceux comportant de 2 à 50 groupements oxyde d'éthylène;
en particulier sous forme de sels de métaux alcalins ou alcalino-terreux, d'ammonium, ou d'aminoalcool.

Les tensioactifs anioniques sulfonates susceptibles d'être utilisés comportent au moins une fonction sulfonate (-SO₃H ou -SO₃⁻).
Ils peuvent être choisis parmi les composés suivants : les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamidesulfosuccinates, les alkylsulfoacétates, les N-acyltaurates, les acyliséthionates ; les alkylsulfolaurates ; ainsi que les sels de ces composés;
les groupes alkyle de ces composés comportant de 6 à 30 atomes de carbone, notamment de 12 à 28, encore mieux de 14 à 24, voire de 16 à 22, atomes de carbone ; le groupe aryle désignant de préférence un groupe phényle ou benzyle ; ces composés pouvant être polyoxyalkylénés, notamment polyoxyéthylénés et comportant alors de préférence de 1 à 50 motifs oxyde d'éthylène, mieux de 2 à 10 motifs oxyde d'éthylène.
Préférentiellement, les tensioactifs anioniques sulfonates sont choisis parmi, seuls ou en mélange :
- les alkylsulfosuccinates en C6-C24, notamment en C12-C20, notamment les laurylsulfosuccinates.
- les alkyléthersulfosuccinates en C6-C24, notamment en C12-C20;
- les (C6-C24)acyliséthionates, de préférence les (C12-C18) acyliséthionates,
en particulier sous forme de sels de métaux alcalins ou alcalino-terreux, d'ammonium, ou d'aminoalcool.

Les tensioactifs anioniques sulfates susceptibles d'être utilisés comportent au moins une fonction sulfate (-OSO₃H ou -OSO₃⁻).
Ils peuvent être choisis parmi les composés suivants : les alkylsulfates, les alkyléthersulfates, les alkylamidoéthersulfates, les alkylarylpolyéthersulfates, les mono-glycéride-sulfates; ainsi que les sels de ces composés;
les groupes alkyle de ces composés comportant de 6 à 30 atomes de carbone, notamment de 12 à 28, encore mieux de 14 à 24, voire de 16 à 22, atomes de carbone; le groupe aryle désignant de préférence un groupe phényle ou benzyle; ces composés pouvant être polyoxyalkylénés, notamment polyoxyéthylénés et comportant alors de préférence de 1 à 50 motifs oxyde d'éthylène, mieux de 2 à 10 motifs oxyde d'éthylène.
Préférentiellement, les tensioactifs anioniques sulfates sont choisis parmi, seuls ou en mélange :
- les alkylsulfates notamment en C6-C24, voire en C12-C20,
- les alkyléthersulfates, notamment en C6-C24, voire en C12-C20, comprenant de préférence de 2 à 20 motifs oxyde d'éthylène ;
en particulier sous forme de sels de métaux alcalins ou alcalino-terreux, d'ammonium, ou d'aminoalcool.

Lorsque le tensioactif anionique est sous forme de sel, ledit sel peut être choisi parmi les sels de métaux alcalins tels que le sel de sodium ou de potassium, les sels d'ammonium, les sels d'amines et en particulier d'aminoalcools, et les sels de métaux alcalino-terreux tels que le sel de magnésium.
Comme exemple de sels d'aminoalcools, on peut citer les sels de mono-, di- et triéthanolamine, les sels de mono-, di- ou tri-isopropanolamine, les sels de 2-amino 2-méthyl 1-propanol, 2-amino 2-méthyl 1,3-propanediol et tris(hydroxyméthyl)amino méthane.
On utilise de préférence les sels de métaux alcalins ou alcalinoterreux, et en particulier les sels de sodium ou de magnésium.

Préférentiellement, les tensioactifs anioniques sont choisis parmi, seul ou en mélange,
- les alkylsulfates en C6-C24, notamment en C12-C20,
- les alkyléthersulfates en C6-C24, notamment en C12-C20; comprenant de préférence de 2 à 20 motifs oxyde d'éthylène;
- les alkylsulfosuccinates en C6-C24, notamment en C12-C20, notamment les laurylsulfosuccinates.
- les alkyléthersulfosuccinates en C6-C24, notamment en C12-C20;
- les (C6-C24)acyliséthionates, de préférence les (C12-C18)acyliséthionates,
- les acylsarcosinates en C6-C24, notamment en C12-C20; notamment les palmitoylsarcosinates;
- les alkyl(C6-C24)éthercarboxylates, de préférence les alkyl(C12-C20)éthercarboxylates;
- les acides alkyl(C₆-C₂₄) (amido) éther carboxyliques polyoxyalkylénés et leurs sels, en particulier ceux comportant de 2 à 50 groupements oxyde d'alkylène, en particulier d'éthylène;
- les acylglutamates en C6-C24, notamment en C12-C20;
- les acylglycinates en C6-C24, notamment en C12-C20;
en particulier sous forme de sels de métaux alcalins ou alcalino-terreux, d'ammonium, ou d'aminoalcool.

Lesdits tensioactifs amphotères peuvent être des dérivés d'amines aliphatiques secondaire ou tertiaire, éventuellement quaternisées, dans lesquels le groupe aliphatique est une chaîne linéaire ou ramifiée comportant 8 à 22 atomes de carbone, lesdits dérivés d'amines contenant au moins un groupe anionique tel que, par exemple, un groupe carboxylate, sulfonate, sulfate, phosphate ou phosphonate. On peut citer en particulier les alkyl(C8-C20)bétaïnes, les sulfobétaïnes, les alkyl(C₈-C₂₀)sulfobétaïnes, les alkyl(C8-C20)amidoalkyl(C1-C6)bétaïnes telles que la cocoamidopropylbétaïne, les alkyl(C8-C20)amidoalkyl(C1-C6)sulfobétaïnes, ainsi que leurs mélanges.

Parmi les dérivés d'amines aliphatiques secondaires ou tertiaires éventuellement quaternisées susceptibles d'être employés, on peut également citer les produits de structures respectives (A1) et (A2) suivantes :

(A1) Rₐ-CON(Z)CH₂-(CH₂)ₘ-N⁺(R_{b})(R_{c})(CH₂COO⁻)

dans laquelle :
Rₐ représente un groupe alkyle ou alkényle en C₁₀-C₃₀ dérivé d'un acide Rₐ-COOH de préférence présent dans l'huile de coprah hydrolysée, un groupe heptyle, nonyle ou undécyle,
R_{b} représente un groupe beta-hydroxyéthyle,
R_{c} représente un groupe carboxyméthyle ;
m est égal à 0,1 ou 2,
Z représente un atome d'hydrogène ou un groupe hydroxyéthyl ou carboxyméthyl;

(A2) R_{a'}-CON(Z)CH₂-(CH₂)_{m'}-N(B)(B')

dans laquelle :
B représente -CH₂CH₂OX', avec X' représentant -CH₂-COOH, CH₂-COOZ',-CH₂CH₂-COOH, -CH₂CH₂-COOZ', ou un atome d'hydrogène,
B' représente -(CH₂)_{z}-Y', avec z = 1 ou 2, et Y' représentant -COOH, -COOZ',-CH₂-CHOH-SO₃H ou -CH₂-CHOH-SO₃Z',
m' est égal à 0,1 ou 2,
Z représente un atome d'hydrogène ou un groupe hydroxyéthyl ou carboxyméthyl,
Z' représente un ion issu d'un métal alcalin ou alcalinoterreux, tel que le sodium, le potassium ou le magnésium; un ion ammonium; ou un ion issu d'une amine organique et notamment d'un aminoalcool, tel que la mono-, di- et triéthanolamine, la mono-, di- ou tri-isopropanol-amine, le 2-amino 2-méthyl 1-propanol, le 2-amino 2-méthyl 1,3-propanediol et le tris(hydroxyméthyl)amino méthane.
R_{a'} représente un groupe alkyle ou alkényle en C₁₀-C₃₀ d'un acide R_{a'}COOH de préférence présent dans l'huile de coprah ou dans l'huile de lin hydrolysée, un groupe alkyle, notamment en C₁₇ et sa forme iso, un groupe en C₁₇ insaturé.

Les composés répondant à la formule (A2) sont particulièrement préférés.
Parmi les composés de formule (A2) pour lesquels X' représente un atome d'hydrogène, on peut citer les composés connus sous les dénominations (CTFA) cocoamphoacétate de sodium, lauroamphoacétate de sodium, caproamphoacétate de sodium et capryloamphoacétate de sodium.
D'autres composés de formule (A2) sont connus sous les dénominations (CTFA) cocoamphodiacétate de disodium, lauroamphodiacétate de disodium, caproamphodiacétate de disodium, capryloamphodiacétate de disodium, cocoamphodipropionate de disodium, lauroamphodipropionate de disodium, caproamphodipropionate de disodium, capryloamphodipropionate de disodium, acide lauroamphodipropionique et acide cocoamphodipropionique.

Comme exemples de composés de formule (A2), on peut citer le cocoamphodiacétate commercialisé par la société RHODIA sous la dénomination commerciale MIRANOL® C2M concentré, le cocoamphoacétate de sodium commercialisé sous la dénomination commerciale MIRANOL ULTRA C 32 et le produit commercialisé par la société CHIMEX sous la dénomination commerciale CHIMEXANE HA.

On peut aussi utiliser des composés de formule (A3) :

(A3) R_{a"}-NH-CH(Y")-(CH₂)n-C(O)-NH-(CH₂)n'-N(R_{d})(Rₑ)

dans laquelle :
- R_{a"} représente un groupe alkyle ou alcényle en C₁₀-C₃₀ d'un acide R_{a"}-C(O)OH, de préférence présent dans l'huile de coprah ou dans l'huile de lin hydrolysée;
- Y" représente le groupe -C(O)OH, -C(O)OZ", -CH₂-CH(OH)-SO₃H ou le groupe -CH₂-CH(OH)-SO₃-Z" avec Z" représentant un cation issu d'un métal alcalin ou alcalinoterreux, tel que le sodium, un ion ammonium ou un ion issu d'une amine organique;
- R_{d} et Rₑ, indépendamment l'un de l'autre, représentent un radical alkyle ou hydroxyalkyle en C1-C4; et
- n et n', indépendamment l'un de l'autre, désignent un nombre entier allant de 1 à 3.

Parmi les composés de formule (A3), on peut notamment citer le composé classé dans le dictionnaire CTFA sous la dénomination sodium diethylaminopropyl cocoaspartamide, et notamment celui commercialisé par la société CHIMEX sous l'appellation CHIMEXANE HB.

De préférence, les tensioactifs amphotères sont choisis parmi les alkyl(C8-C20)bétaïnes, les alkyl(C8-C20)amidoalkyl(C1-C6)bétaïnes, les alkyl(C8-C20) amphoacétates et les alkyl(C8-C20) amphodiacétates, et leurs mélanges.

La composition lavante peut en outre comprendre de l'eau ou d'un mélange d'eau et d'un ou plusieurs solvants cosmétiquement acceptables choisis parmi les monoalcools en C₁ à C₄ tels que l'éthanol, l'isopropanol, le tertio-butanol ou le n-butanol; les polyols tels que le glycérol, le propylèneglycol et les polyéthylèneglycols ; et leurs mélanges.
De préférence, ladite composition lavante comprend de l'eau à une concentration allant de préférence de 40 à 99% en poids, notamment de 50 à 95% en poids, mieux de 60 à 90% en poids, par rapport au poids total de ladite composition.

Dans un mode de réalisation préféré, la composition lavante comprend avantageusement un ou plusieurs polymères cationiques ayant une densité de charge supérieure ou égale à 4 méq/g (milliéquivalents par gramme), de préférence supérieure ou égale à 5 méq/g ; notamment ayant une densité de charge cationique allant de 4 à 20 méq/g, voire de 5 à 20 méq/g.

Lesdits polymères cationiques sont tels que ceux déjà décrits ci-dessus. De préférence, lesdits polymères cationiques sont choisis parmi ceux des familles (1), (6) et (7) ci-dessus citées.
On peut utiliser de préférence, seul ou en mélange :
- les cyclopolymères d'alkyldiallylamine ou de dialkyldiallylammonium, en particulier les homopolymères ou copolymères de sels (par exemple chlorure) de diméthyldiallylammonium, vendus sous les dénominations MERQUAT 100, MERQUAT 550 et MERQUAT S par la société NALCO,
- les homopolymères ou copolymères éventuellement réticulés de sels de méthacryloyloxyalkyl(C1-C4) trialkyl(C1-C4)ammonium;
- les polymères de diammonium quaternaire comprenant des motifs récurrents de formule (III) ;
- les homopolymères ou copolymères dérivés des acides acrylique ou méthacrylique et comportant des motifs ci-après, tels que définis ci-dessus :

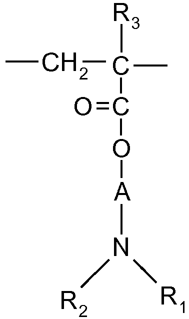

Encore mieux, le ou les polymères cationiques ayant une densité de charge supérieure ou égale à 4 méq/g est/sont choisis parmi le chlorure de 2-méthacryloyloxyéthyl triméthyl ammonium (Polyquaternium-37), le chlorure de diméthyl diallyl ammonium (Polyquaternium-6) et leurs mélanges.

De préférence, ladite composition lavante comprend le ou lesdits polymères cationiques ayant une densité de charge supérieure ou égale à 4 méq/g, en une quantité allant de 0,01 à 15% en poids, de préférence de 0,1 à 10% en poids, préférentiellement de 0,2 à 5% en poids, par rapport au poids total de la composition.

Dans une variante particulièrement préférée de l'invention, ladite composition lavante comprend au moins un polymère cationique de densité de charge supérieure ou égale à 4 méq/g identique à au moins un polymère cationique de densité de charge supérieure ou égale à 4 méq/g présent dans la deuxième composition cosmétique telle que décrite précédemment.

Le procédé de traitement cosmétique des cheveux selon l'invention trouve une application particulièrement intéressante pour l'hygiène, le nettoyage, le soin et/ou le conditionnement des cheveux; avantageusement, pour le nettoyage et le conditionnement des cheveux.
De préférence, il s'agit d'un procédé cosmétique de nettoyage et/ou de soin et/ou de conditionnement des cheveux.
En particulier, il s'agit d'un procédé cosmétique de nettoyage et/ou de soin et/ou de conditionnement des cheveux, permettant l'obtention d'un conditionnement à la fois immédiat et durable, c'est-à-dire rémanent à au moins 3 shampoings (ou lavages).

La présente invention est illustrée plus en détails dans les exemples qui suivent (MA = matière active).

### Exemple 1

On prépare les compositions premières ci-après (% en poids):

| | Composition 1A | Composition 1B | Composition 1C |
|---|---|---|---|
| Mélange d'alcools myristique, cétylique, stéarylique (2,5/95/2,5) | 1,23% | 1,85% | 3,7% |
| Mélange de stéarate de myristyle et de palmitate de myristyle | 0,27% | 0,4% | 0,8% |
| Alcool myristique | 0,13% | 0,2% | 0,4% |
| Hydroxyéthylcellulose (PM 1300000) | 1,2% | 1% | 0,25% |
| Méthosulfate de dipalmitoyléthyl hydroxyéthyl méthyl ammonium (à 30% dans l'alcool cétéarylique) | 0,1% MA | 0,15% MA | 0,3% MA |
| Chlorure de cétyl triméthyl ammonium | 0,2% MA | 0,32% MA | 0,62% |
| Chlorure de béhényl triméthyl ammonium | 0,16% MA | 0,24% MA | 0,47% MA |
| Silicone aminée (XIAMETER MEM-8299 EMULSION) | 0,5% MA | 0,8% MA | 1,5% MA |
| Céramide | 0,01% | 0,01% | 0,01% |
| Acide lactique ou citrique | 0,1% | 0,1% | 0,03% |
| Conservateur, Parfum | Qs | Qs | Q.s. |
| Eau | Qsp 100% | Qsp 100% | Qsp 100% |

### Exemple 2

On prépare la composition deuxième ci-après (% en poids):

| | Composition 2 |
|---|---|
| Alcool cétylstéarylique (C₁₆/C₁₈, 50/50) | 9% |
| Mélange de stéarate de myristyle et de palmitate de myristyle | 1% |
| Huile minérale blanche | 3% |
| Méthosulfate de dipalmitoyléthylhydroxyéthylméthyl ammonium / alcool cétéarylique (30/70) | 4,5% (1,35 MA + 3,15 MA) |
| Chlorure de cétyl triméthyl ammonium, en solution aqueuse à 25% | 3,2% (0,8 MA) |
| 3-aminopropyltriéthoxysilane | 5% |
| Acide lactique | 2% |
| Chlorure de polydiméthyldiallyl ammonium, en solution aqueuse à 40% (PQ6) | 1,2% (0,48 MA) |
| Conservateur, Parfum | Qs |
| Eau | Qsp 100% |

### Exemple 3

On prépare les compositions lavantes ci-après (% en poids):

| | Composition 3A | Composition 3B | Composition 3C |
|---|---|---|---|
| Lauryl éther sulfate de sodium | 17,46% MA | 17,5% MA | 5,6% MA |
| Acide lauryl éther carboxylique (5 OE) | - | - | 4,16% MA |
| Cocobétaine | 3% MA | 3% MA | 7,56% MA |
| Distéarate de glycol | 1,6% | 1,6% | - |
| Diméthicone | 2,7% MA | 0,75% MA | - |
| Amodimethicone | - | 1,15% MA | - |
| 2-oleamido-1,3-octadecanediol | 0,01% | 0,01% | 0,01% |
| Carbomer | 0,15% | 0,15% | - |
| Chlorure de polydiméthyldiallyl ammonium, en solution aqueuse à 40% (PQ6) | 0,16% MA | 0,3% MA | 0,4% MA |
| PPG-5 ceteth-20 | 0,8% | 0,85% | 1% |
| Glycérine | - | - | 2% |
| NaCl | 0,73% | 0,73% | 1% |
| Conservateur, Parfum | Q.s. | Q.s. | Q.s. |
| Agent pH | Q.s. pH = 5,5 | Q.s. pH = 5,5 | Q.s. pH = 5,5 |
| Eau | Qsp 100% | Qsp 100% | Qsp 100% |

### Exemple 4

On a préalablement lavé la chevelure avec 12 g de la composition 3A.
Après rinçage, on a appliqué 12 g de la composition cosmétique première 1B que l'on a laissé poser pendant 5 minutes.
Sans rinçage intermédiaire, on a ensuite appliqué 18 g de la composition cosmétique deuxième 2, que l'on a laissé poser pendant 5 minutes.

Après rinçage, les cheveux humides sont faciles à démêler, souples, lisses au toucher, individualisés.
Lors du séchage, les cheveux sèchent rapidement.
Après séchage, les cheveux sont faciles à démêler, lisses visuellement, lisses au toucher, avec un toucher régulier de la racine à la pointe.

Les performances cosmétiques obtenues persistent après 4 shampoings, en particulier en matière de souplesse, lissage et démêlage, notamment sur cheveux secs.

### Exemple 5

On prépare les compositions suivantes (% en poids de matière première en l'état)

### Composition A:

| | % en poids |
|---|---|
| ALCOOL CETYLSTEARYLIQUE (C16/C18 50/50) | 6 |
| MELANGE DE STEARATE DE MYRISTYLE ET DE PALMITATE DE MYRISTYLE | 1 |
| N-OLEYL DI-HYDROSPHINGOSINE | 0.1 |
| METHOSULFATE DE DIPALMITOYLETHYL HYDROXYETHYLMETHYLAMMONIUM à 30% dans l'ALCOOL CETEARYLIQUE | 4.5 |
| OCTANE-1,2-DIOL | 0.3 |
| ACIDE CITRIQUE, 1 H₂O | 0.03 |
| CHLORURE DE CETYL TRIMETHYL AMMONIUM à 25% dans l'eau | 3.2 |
| HUILE MINERALE BLANCHE | 2 |
| POLYDIMETHYLSILOXANE A GROUPEMENTS AMINOETHYLAMINOPROPYL, A FONCTION METHOXY ET/OU HYDROXY ET ALPHA-OMEGA SILANOLS EN EMULSION aqueuse CATIONIQUE A 60% | 1.7 |
| PARFUM | q.s. |
| EAU | Q.s. 100% |

### Composition B:

| | % en poids |
|---|---|
| CHLORURE DE CETYL TRIMETHYL AMMONIUM à 25% EN SOLUTION AQUEUSE | 0.25 |
| HYDROXYETHYL CELLULOSE (PM : 1.300.000) | 0.25 |
| COPOLYMERE SMDI / POLYETHYLENE GLYCOL A TERMINAISON ALKYLE (METHYL/C18) A 15% DANS UNE MATRICE MALTODEXTRINE / EAU | 3 |
| HOMOPOLYMERE CHLORURE DE METHACRYLATE D'ETHYL TRIMETHYL AMMONIUM RETICULE EN EMULSION INVERSE DANS HUILE MINERALE 50% (PQ37) | 1 |
| PARFUM | q.s. |
| ACIDE LACTIQUE | 1.6 |
| 3-AMINOPROPYL TRIETHOXYSILANE | 5 |
| ALCOOL CETYLSTEARYLIQUE (C16/C18 50/50) | 4 |
| N-OLEYL DI-HYDROSPHINGOSINE | 0.01 |
| Eau | Qsp 100% |

### Protocoles d'application des compositions

Les compositions ont été appliquées sur des mèches de cheveux faiblement sensibilisées (solubilité alcaline 20%, SA 20), à raison de 1g de composition pour 2.7 g de cheveux.

| | Protocole 1 (invention) | Protocole 2 (comparatif) |
|---|---|---|
| Etape 1 | Appliquer la composition A | Appliquer la composition A |
| Etape 2 | Laisser poser 5 minutes **Ne pas rincer** | Laisser poser 5 minutes |
| Etape 3 | | **Rincer à l'eau pendant 15 secondes (eau à 38°C, débit : 3 litres/minute)** |
| Etape 4 | Appliquer la composition B | Appliquer la composition B |
| Etape 5 | Laisser poser 5 minutes | Laisser poser 5 minutes |
| Etape 6 | Rincer et sécher | Rincer et sécher |

On compare ensuite les performances en matière de lissage au toucher, de légèreté, de souplesse, d'individualisation et de toucher chargé.

Ces performances ont été évaluées sur cheveux secs, par 10 experts. L'évaluation des performances est faite de manière tactile (sensorielle), par comparaison d'une mèche traitée selon le protocole 1, avec une mèche traitée selon le protocole 2 ; l'expert ne sait pas quel protocole a été appliqué à la mèche (test en aveugle).

Pour chacun des critères, les 10 experts se sont prononcés sur la mèche qui présentait le meilleur niveau de performances (= signifiant que la performance est équivalente pour les 2 mèches).

Ci-après les résultats obtenus :

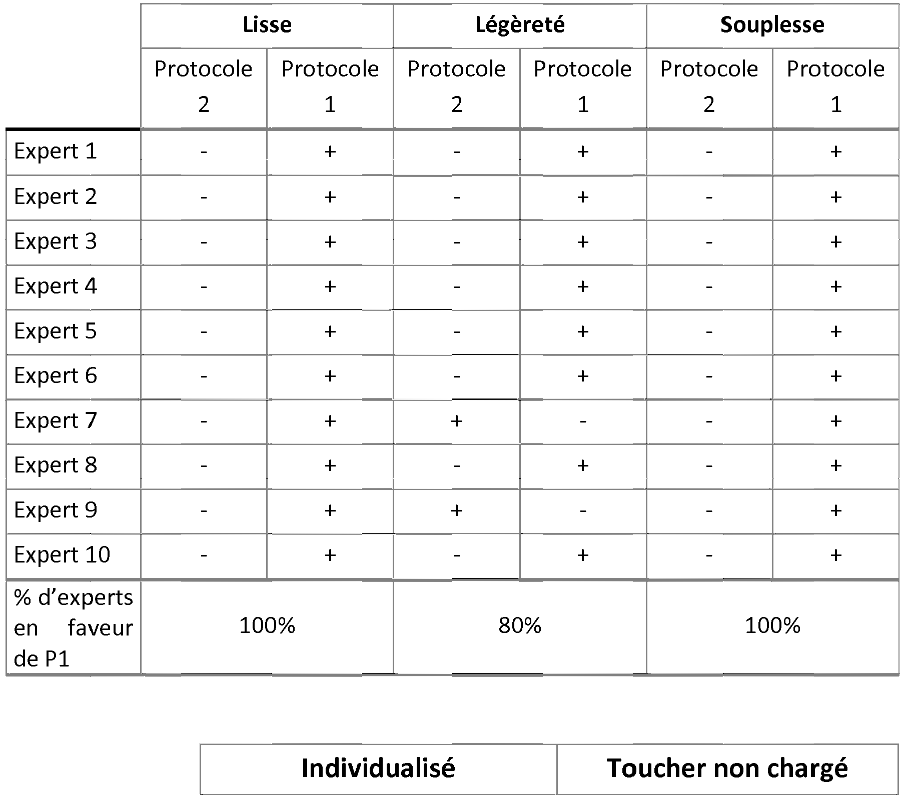

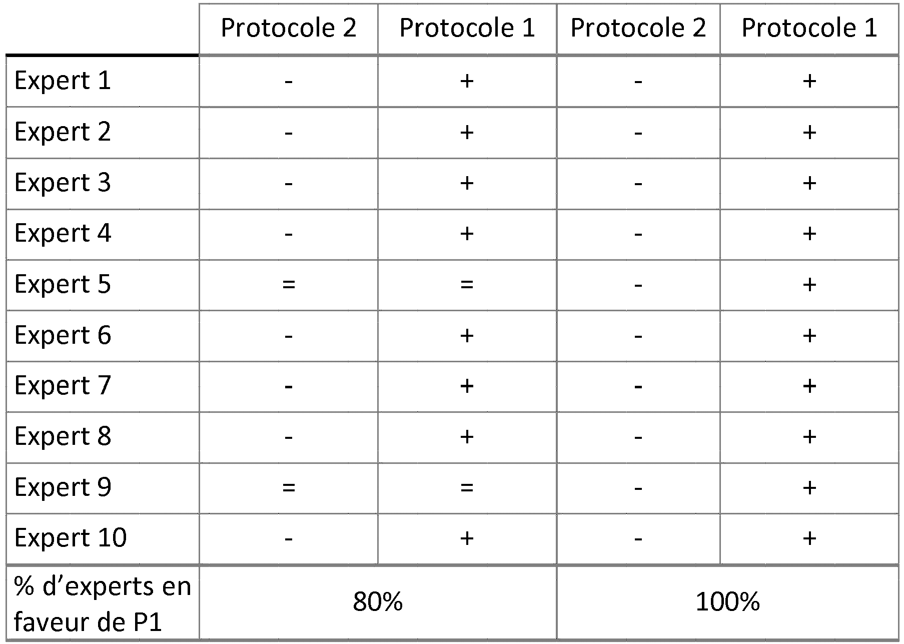

On constate donc que :
- dans 100% des cas, les experts ont jugés que le lissage au toucher, la souplesse et le toucher non chargé des mèches ayant subi le protocole P1 selon l'invention étaient supérieurs à ceux des mèches sur lesquelles le protocole comparatif P2 a été appliqué.
- dans 80% des cas, les experts ont jugés que la légèreté et le caractère individualisé des mèches de cheveux sur lesquelles le protocole P1 selon l'invention a été appliqué étaient supérieurs à ceux des mèches sur lesquelles le protocole comparatif P2 a été appliqué.

## Revendications

1. Procédé de traitement cosmétique des cheveux, comprenant :
- une étape (i) d'application sur lesdits cheveux, d'une composition cosmétique dite première, comprenant un ou plusieurs tensioactifs cationiques, une ou plusieurs silicones et un ou plusieurs corps gras non siliconés, puis
- une étape (ii) d'application sur lesdits cheveux, d'une composition cosmétique dite deuxième, comprenant un ou plusieurs tensioactifs cationiques, un ou plusieurs polymères cationiques présentant une densité de charge cationique supérieure ou égale à 4 méq/g, et un ou plusieurs organosilanes ;
ledit procédé ne comprenant pas d'étape de rinçage intermédiaire entre lesdites étape (i) et étape (ii) d'application.

2. Procédé selon la revendication 1, dans lequel l'étape (i) d'application est suivie d'une étape de pose de la composition cosmétique dite première, de préférence pendant 1 à 15 minutes.

3. Procédé selon l'une des revendications précédentes, dans lequel l'étape (ii) d'application est suivie d'une étape de pose de la composition cosmétique dite deuxième, de préférence pendant 1 à 25 minutes, notamment 1 à 20 minutes, et d'une étape de rinçage, puis éventuellement d'une étape de séchage.

4. Procédé selon l'une des revendications précédentes, dans lequel la composition cosmétique dite première comprend un ou plusieurs tensioactifs cationiques choisis parmi les sels d'amines grasses primaire, secondaire ou tertiaire, éventuellement polyoxyalkylénées; les sels d'ammonium quaternaire, et leurs mélanges ; et notamment choisis parmi :
- les sels d'ammonium quaternaire de formule (la) : 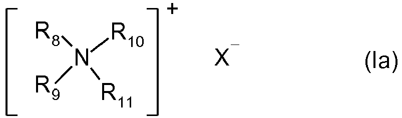 dans laquelle :
les groupes R₈ à R₁₁, identiques ou différents, représentent un groupe aliphatique linéaire ou ramifié, comportant de 1 à 30 atomes de carbone, ou un groupe aromatique tel que aryle ou alkylaryle, au moins l'un des groupes R₈ à R₁₁ comportant de 8 à 30 atomes de carbone, de préférence de 12 à 24 atomes de carbone; les groupes aliphatiques pouvant comporter des hétéroatomes tels que notamment l'oxygène, l'azote, le soufre et les halogènes ; et
- X⁻ est un anion notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.
- les sels d'ammonium quaternaire de l'imidazoline de formule (IIa) : 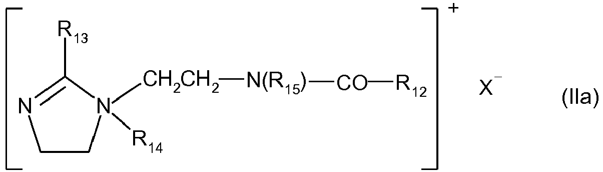 dans laquelle
R₁₂ représente un groupe alcényle ou alkyle comportant de 8 à 30 atomes de carbone, par exemple dérivés des acides gras du suif,
R₁₃ représente un atome d'hydrogène, un groupe alkyle en C₁-C₄ ou un groupe alcényle ou alkyle comportant de 8 à 30 atomes de carbone,
R₁₄ représente un groupe alkyle en C₁-C₄,
R₁₅ représente un atome d'hydrogène ou un groupe alkyle en C₁-C₄,
X⁻ est un anion, notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.
- les sels de di- ou de triammonium quaternaire de formule (IIIa) : 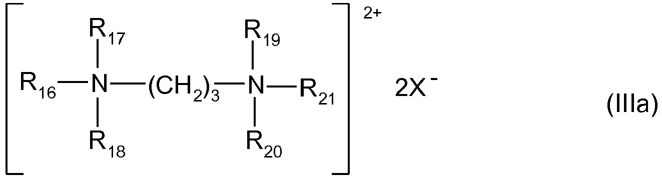 dans laquelle :
- R₁₆ désigne un groupe alkyle comportant de 16 à 30 atomes de carbone éventuellement hydroxylé et/ou éventuellement interrompu par un ou plusieurs atomes d'oxygène,
- R₁₇ désigne l'hydrogène, un groupe alkyle comportant de 1 à 4 atomes de carbone ou un groupe -(CH₂)₃-N⁺(R₁₆ₐ)(R₁₇ₐ)(R₁₈ₐ), R₁₆ₐ, R₁₇ₐ, R₁₈ₐ, identiques ou différents désignant l'hydrogène ou un groupe alkyle comportant de 1 à 4 atomes de carbone,
- R₁₈, R₁₉, R₂₀ et R₂₁, identiques ou différents, désignent l'hydrogène ou un groupe alkyle comportant de 1 à 4 atomes de carbone, et
- X⁻ est un anion notamment choisi dans le groupe des halogénures, acétates, phosphates, nitrates, alkyl(C₁-C₄)sulfates, alkyl(C₁-C₄)sulfonates et alkyl(C₁-C₄)aryl-sulfonates, en particulier méthylsulfate et éthylsulfate.
- les sels d'ammonium quaternaire contenant une ou plusieurs fonctions esters de formule (IVa) suivante : 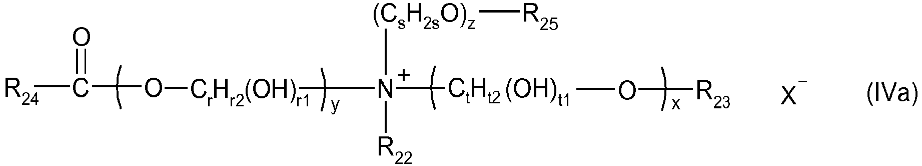 dans laquelle :
- R₂₂ est choisi parmi les groupes alkyles en C₁-C₆ et les groupes hydroxyalkyle ou dihydroxyalkyle en C₁-C₆,
- R₂₃ est choisi parmi le groupe R₂₆-C(=O)-; les groupes R₂₇ hydrocarbonés en C₁-C₂₂, linéaires ou ramifiés, saturés ou insaturés; et l'atome d'hydrogène,
- R₂₅ est choisi parmi le groupe R₂₈-C(=O)-; les groupes R₂₉ hydrocarbonés en C₁-C₆, linéaires ou ramifiés, saturés ou insaturés; et l'atome d'hydrogène,
- R₂₄, R₂₆ et R₂₈, identiques ou différents, sont choisis parmi les groupes hydrocarbonés en C₇-C₂₁, linéaires ou ramifiés, saturés ou insaturés,
- r, s et t, identiques ou différents, sont des entiers valant de 2 à 6,
- r1 et t1, identiques ou différents, valent 0 ou 1,
- y est un entier valant de 1 à 10,
- x et z, identiques ou différents, sont des entiers valant de 0 à 10,
- X⁻ est un anion,
étant entendu que r2 + r1 = 2r et t1 + t2 = 2t, et que la somme x + y + z vaut de 1 à 15,
sous réserve que lorsque x = 0 alors R₂₃ désigne R₂₇ et que lorsque z = 0 alors R₂₅ désigne R₂₉.

5. Procédé selon la revendication précédente, dans lequel la composition cosmétique dite première comprend un ou plusieurs tensioactifs cationiques choisis parmi les sels d'ammonium de formule (la) ou (IVa), de préférence de formule (IVa) dans laquelle :
- R₂₂ désigne un groupe méthyle ou éthyle,
- x et y sont égaux à 1,
- z est égal à 0 ou 1,
- r, s et t sont égaux à 2,
- R₂₃ est choisi parmi le groupe R₂₆-C(=O)-; les groupes méthyle, éthyle ou hydrocarbonés en C₁₄-C₂₂, l'atome d'hydrogène,
- R₂₅ est choisi parmi le groupe R₂₈-C(=O)-; l'atome d'hydrogène,
- R₂₄, R₂₆ et R₂₈, identiques ou différents, sont choisis parmi les groupes hydrocarbonés en C₁₃-C₁₇, linéaires ou ramifiés, saturés ou insaturés, et de préférence parmi les groupes alkyle et alcényle en C₁₃-C₁₇, linéaires ou ramifiés, saturés ou insaturés ;
et encore mieux choisis parmi les sels de cétyltriméthylammonium, de béhényltriméthylammonium, de dipalmitoyléthylhydroxyéthylméthylammonium et leurs mélanges; et plus particulièrement parmi le chlorure ou le méthosulfate de béhényltriméthylammonium, le chlorure ou le méthosulfate de cétyltriméthylammonium, le chlorure ou le méthosulfate de dipalmitoyléthylhydroxyéthylméthylammonium et leurs mélanges.

6. Procédé selon l'une des revendications précédentes, dans lequel la composition cosmétique dite première comprend au moins deux tensioactifs cationiques différents; notamment au moins un tensioactif cationique choisi parmi ceux de formule (la) et au moins un tensioactif cationique choisi parmi ceux de formule (IVa), formules telles que définies à la revendication 4.

7. Procédé selon l'une des revendications précédentes, dans lequel la composition cosmétique dite première comprend un ou plusieurs tensioactifs cationiques en une quantité allant de 0,1 à 3,5% en poids, de préférence de 0,2 à 3,5% en poids, préférentiellement de 0,3 à 3% en poids, par rapport au poids total de la composition.

8. Procédé selon l'une des revendications précédentes, dans lequel la composition cosmétique dite première comprend une ou plusieurs silicones aminées, notamment choisies parmi :
a) les polysiloxanes répondant à la formule (A): 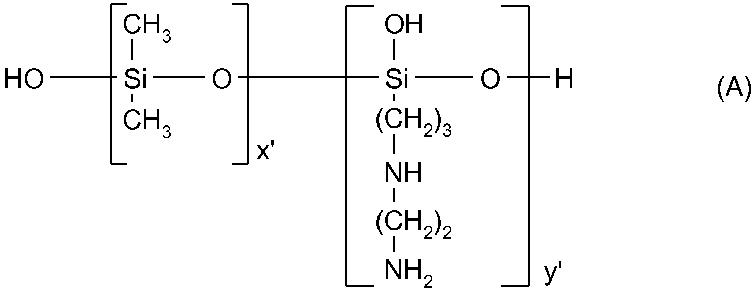 dans laquelle x' et y' sont des nombres entiers tels que le poids moléculaire moyen en poids (Mw) est compris entre 5 000 et 500 000 environ ;
b) les silicones aminées répondant à la formule (B) :
R'ₐG₃₋ₐ-Si(OSiG₂)ₙ-(OSiG_{b}R'_{2-b})ₘ-O-SiG₃₋ₐ-R'ₐ (B)
dans laquelle :
- G, identique ou différent, désigne un atome d'hydrogène, un groupement phényle, OH, alkyle en C₁-C₈, par exemple méthyle, ou alcoxy en C₁-C₈, par exemple méthoxy,
- a, identique ou différent, désigne 0 ou un entier de 1 à 3, en particulier 0,
- b désigne 0 ou 1, en particulier 1,
- m et n sont des nombres tels que la somme (n + m) varie de 1 à 2000, en particulier de 50 à 150, n pouvant désigner un nombre de 0 à 1999, et notamment de 49 à 149 et m pouvant désigner un nombre de 1 à 2000, et notamment de 1 à 10;
- R', identique ou différent, désigne un radical monovalent de formule -CqH2qL dans laquelle q est un nombre allant de 2 à 8, et L est un groupement aminé éventuellement quaternisé choisi parmi les groupements :
-N(R")₂; -N⁺(R")₃A- ; -NR"-Q-N(R")₂ et -NR"-Q-N⁺(R")₃A-,
dans lesquels R", identique ou différent, désigne hydrogène, phényle, benzyle, ou un radical hydrocarboné saturé monovalent, par exemple un radical alkyle en C1-C20; Q désigne un groupement de formule CᵣH₂ᵣ, linéaire ou ramifié, r étant un entier allant de 2 à 6, de préférence de 2 à 4; et A- représente un anion cosmétiquement acceptable, notamment halogénure tel que fluorure, chlorure, bromure ou iodure.
c) les silicones aminées répondant à la formule (H) : 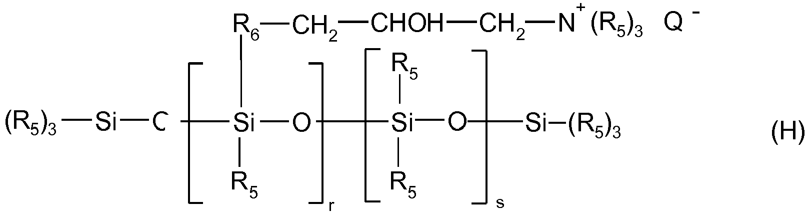 dans laquelle :
- R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
- R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
- Q- est un anion tel qu'un ion halogénure, notamment chlorure ou un sel d'acide organique, notamment acétate;
- r représente une valeur statistique moyenne allant de 2 à 20, en particulier de 2 à 8;
- s représente une valeur statistique moyenne allant de 20 à 200, en particulier de 20 à 50.
d) les silicones à ammonium quaternaire de formule (I) : 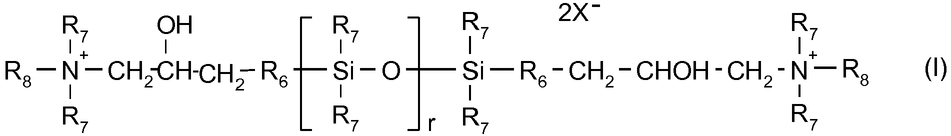 dans laquelle :
- R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
- R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
- R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
- X- est un anion tel qu'un ion halogénure, notamment chlorure ou un sel d'acide organique, notamment acétate;
- r représente une valeur statistique moyenne allant de 2 à 200, en particulier de 5 à 100.
e) les silicones aminées de formule (J) : 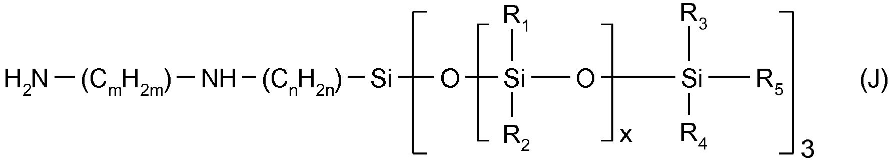 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5, et
- x est choisi de manière telle que l'indice d'amine varie de 0,01 à 1 meq/g.
f) les silicones aminées polyoxyalkylénées multibloc, de type (AB)n, A étant un bloc polysiloxane et B étant un bloc polyoxyalkyléné comportant au moins un groupement amine.

9. Procédé selon l'une des revendications précédentes, dans lequel la composition cosmétique dite première comprend une ou plusieurs silicones aminées choisies parmi :
- les silicones dénommées "triméthylsilylamodiméthicone" répondant à la formule (C): 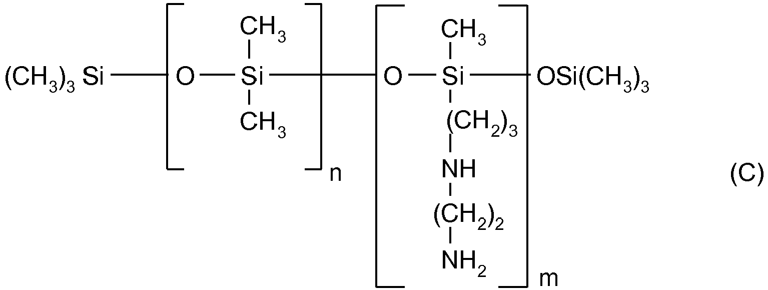 dans laquelle m et n sont des nombres tels que la somme (n + m) varie de 1 à 2000, en particulier de 50 à 150, n pouvant désigner un nombre de 0 à 1999, et notamment de 49 à 149 et m pouvant désigner un nombre de 1 à 2000, et notamment de 1 à 10.
- les silicones de formule (D) suivante : 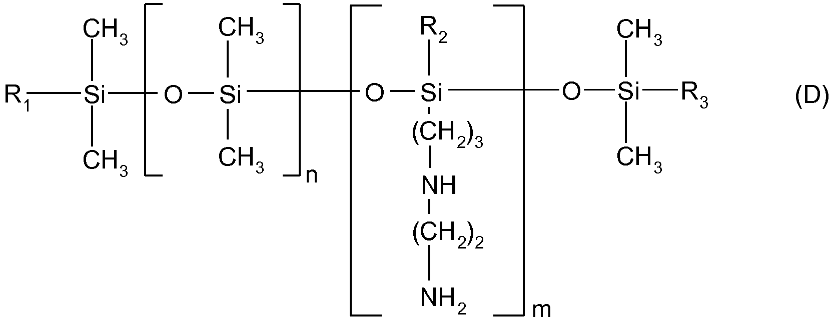 dans laquelle :
- m et n sont des nombres tels que la somme (n + m) varie de 1 à 1000, en particulier de 50 à 250 et plus particulièrement de 100 à 200; n pouvant désigner un nombre de 0 à 999 et notamment de 49 à 249 et plus particulièrement de 125 à 175 et m pouvant désigner un nombre de 1 à 1000, notamment de 1 à 10, plus particulièrement de 1 à 5;
- R1, R2, R3, identiques ou différents, représentent un radical hydroxy ou alcoxy en C1-C4, l'un au moins des radicaux R1 à R3 désignant un radical alcoxy.
- les silicones de formule (E) suivante : 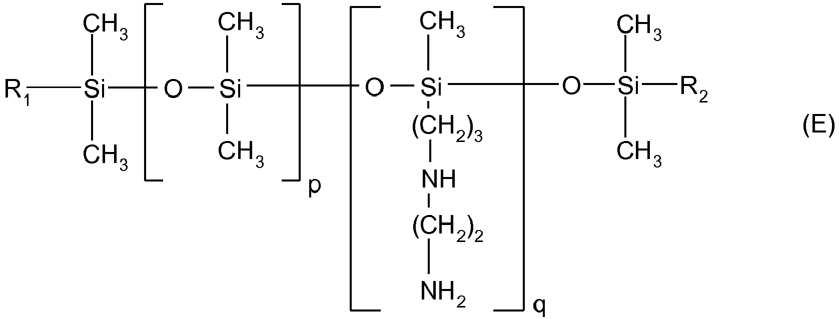 dans laquelle :
- p et q sont des nombres tels que la somme (p+q) varie de 1 à 1000, en particulier de 50 à 350, et plus particulièrement de 150 à 250; p pouvant désigner un nombre de 0 à 999 et notamment de 49 à 349 et plus particulièrement de 159 à 239 et q pouvant désigner un nombre de 1 à 1000, notamment de 1 à 10 et plus particulièrement de 1 à 5;
- R1, R2, différents, représentent un radical hydroxy ou alcoxy en C1-C4, l'un au moins des radicaux R1 ou R2 désignant un radical alcoxy.
- les silicones de formule suivante (F) : 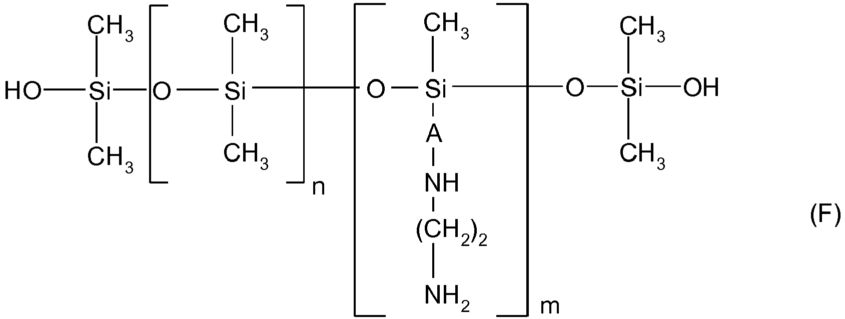 dans laquelle :
- m et n sont des nombres tels que la somme (n + m) varie de 1 à 2000 et en particulier de 50 à 150, n pouvant désigner un nombre de 0 à 1999 et notamment de 49 à 149 et m pouvant désigner un nombre de 1 à 2000, et notamment de 1 à 10;
- A désigne un radical alkylène linéaire ou ramifié ayant de 4 à 8 atomes de carbone et de préférence 4 atomes de carbone. Ce radical est de préférence linéaire.
- les silicones de formule suivante (G): 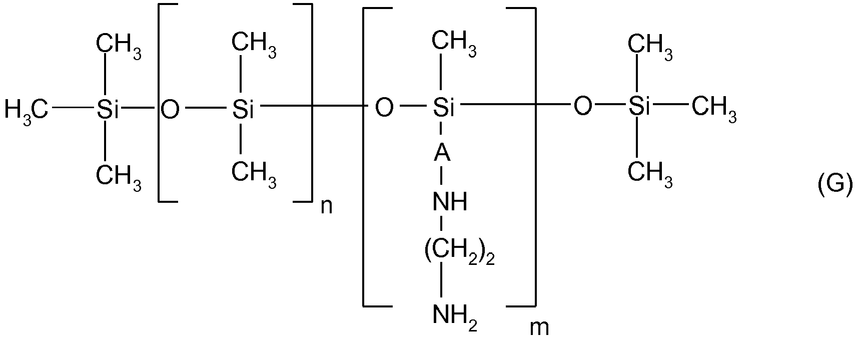 dans laquelle :
- m et n sont des nombres tels que la somme (n + m) varie de 1 à 2000 et en particulier de 50 à 150, n pouvant désigner un nombre de 0 à 1 999 et notamment de 49 à 149 et m pouvant désigner un nombre de 1 à 2000, et notamment de 1 à 10 ;
- A désigne un radical alkylène linéaire ou ramifié ayant de 4 à 8 atomes de carbone et de préférence 4 atomes de carbone ;
et tout particulièrement répondant à la formule (F).

10. Procédé selon l'une des revendications précédentes, dans lequel la composition cosmétique dite première comprend la ou les silicones en une quantité allant de 0,1 à 2% en poids, de préférence de 0,2 à 1,7% en poids, préférentiellement de 0,3 à 1,6% en poids, par rapport au poids total de la composition.

11. Procédé selon l'une des revendications précédentes, dans lequel la composition cosmétique dite première comprend la ou les silicones aminées en une quantité allant de 0,1 à 2% en poids, de préférence de 0,2 à 1,7% en poids, préférentiellement de 0,3 à 1,6% en poids, par rapport au poids total de la composition.

12. Procédé selon l'une des revendications précédentes, dans lequel la composition cosmétique dite première comprend un ou plusieurs corps gras non siliconés solides, notamment choisis parmi les alcools gras, les esters d'acide gras et/ou d'alcool gras, les cires non siliconées, les céramides et leurs mélanges.

13. Procédé selon l'une des revendications précédentes, dans lequel la composition cosmétique dite première comprend ledit ou lesdits corps gras non siliconés en une quantité allant de 0,1 à 20% en poids, de préférence de 0,5 à 15% en poids, préférentiellement de 1 à 10% en poids, par rapport au poids total de la composition.

14. Procédé selon l'une des revendications précédentes, dans lequel la composition cosmétique dite première comprend ledit ou lesdits corps gras non siliconés solides en une quantité allant de 0,5 à 15% en poids, de préférence de 1 à 10% en poids, préférentiellement de 1,5 à 8% en poids, par rapport au poids total de la composition.

15. Procédé selon l'une des revendications précédentes, dans lequel la composition cosmétique dite première comprend de l'eau à une concentration allant de préférence de 50 à 99% en poids, notamment de 60 à 98% en poids, mieux de 70 à 97% en poids, par rapport au poids total de ladite composition.

16. Procédé selon l'une des revendications précédentes, dans lequel la composition cosmétique dite deuxième comprend un ou plusieurs tensioactifs cationiques choisis parmi les sels d'amines grasses primaire, secondaire ou tertiaire, éventuellement polyoxyalkylénées; les sels d'ammonium quaternaire, et leurs mélanges ; et notamment choisis parmi :
- les sels d'ammonium quaternaire de formule (Ia) : 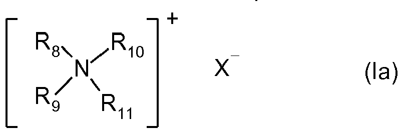 dans laquelle :
les groupes R₈ à R₁₁, identiques ou différents, représentent un groupe aliphatique linéaire ou ramifié, comportant de 1 à 30 atomes de carbone, ou un groupe aromatique tel que aryle ou alkylaryle, au moins l'un des groupes R₈ à R₁₁ comportant de 8 à 30 atomes de carbone, de préférence de 12 à 24 atomes de carbone; les groupes aliphatiques pouvant comporter des hétéroatomes tels que notamment l'oxygène, l'azote, le soufre et les halogènes ; et
- X⁻ est un anion notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.
- les sels d'ammonium quaternaire de l'imidazoline de formule (IIa) : 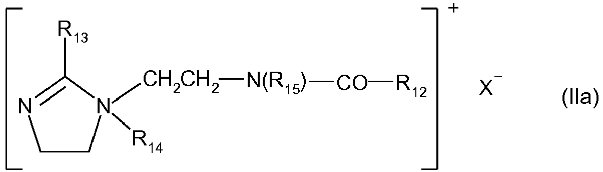 dans laquelle
R₁₂ représente un groupe alcényle ou alkyle comportant de 8 à 30 atomes de carbone, par exemple dérivés des acides gras du suif,
R₁₃ représente un atome d'hydrogène, un groupe alkyle en C₁-C₄ ou un groupe alcényle ou alkyle comportant de 8 à 30 atomes de carbone,
R₁₄ représente un groupe alkyle en C₁-C₄,
R₁₅ représente un atome d'hydrogène ou un groupe alkyle en C₁-C₄,
X⁻ est un anion, notamment choisi dans le groupe des halogénures, phosphates, acétates, lactates, alkyl(C₁-C₄)sulfates, alkyl(C₁-C₄)sulfonates ou alkyl(C₁-C₄)aryl-sulfonates.
- les sels de di- ou de triammonium quaternaire de formule (IIIa) : 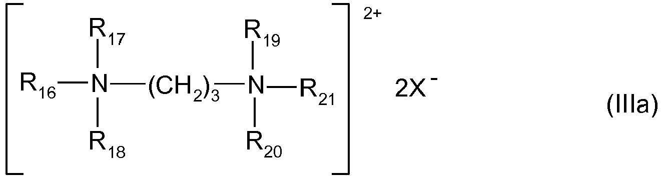 dans laquelle :
- R₁₆ désigne un groupe alkyle comportant de 16 à 30 atomes de carbone éventuellement hydroxylé et/ou éventuellement interrompu par un ou plusieurs atomes d'oxygène,
- R₁₇ désigne l'hydrogène, un groupe alkyle comportant de 1 à 4 atomes de carbone ou un groupe -(CH₂)₃-N⁺(R₁₆ₐ)(R₁₇ₐ)(R₁₈ₐ), R₁₆ₐ, R₁₇ₐ, R₁₈ₐ, identiques ou différents désignant l'hydrogène ou un groupe alkyle comportant de 1 à 4 atomes de carbone,
- R₁₈, R₁₉, R₂₀ et R₂₁, identiques ou différents, désignent l'hydrogène ou un groupe alkyle comportant de 1 à 4 atomes de carbone, et
- X⁻ est un anion notamment choisi dans le groupe des halogénures, acétates, phosphates, nitrates, alkyl(C₁-C₄)sulfates, alkyl(C₁-C₄)sulfonates et alkyl(C₁-C₄)aryl-sulfonates, en particulier méthylsulfate et éthylsulfate.
- les sels d'ammonium quaternaire contenant une ou plusieurs fonctions esters de formule (IVa) suivante : 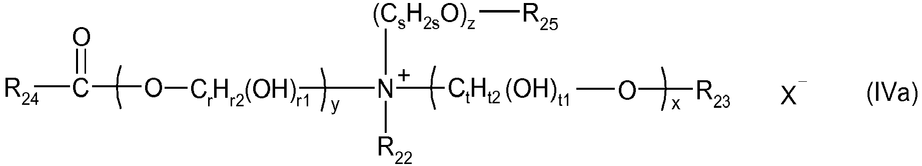 dans laquelle :
- R₂₂ est choisi parmi les groupes alkyles en C₁-C₆ et les groupes hydroxyalkyle ou dihydroxyalkyle en C₁-C₆,
- R₂₃ est choisi parmi le groupe R₂₆-C(=O)-; les groupes R₂₇ hydrocarbonés en C₁-C₂₂, linéaires ou ramifiés, saturés ou insaturés; et l'atome d'hydrogène,
- R₂₅ est choisi parmi le groupe R₂₈-C(=O)-; les groupes R₂₉ hydrocarbonés en C₁-C₆, linéaires ou ramifiés, saturés ou insaturés; et l'atome d'hydrogène,
- R₂₄, R₂₆ et R₂₈, identiques ou différents, sont choisis parmi les groupes hydrocarbonés en C₇-C₂₁, linéaires ou ramifiés, saturés ou insaturés,
- r, s et t, identiques ou différents, sont des entiers valant de 2 à 6,
- r1 et t1, identiques ou différents, valent 0 ou 1,
- y est un entier valant de 1 à 10,
- x et z, identiques ou différents, sont des entiers valant de 0 à 10,
- X⁻ est un anion,
étant entendu que r2 + r1 = 2r et t1 + t2 = 2t, et que la somme x + y + z vaut de 1 à 15,
sous réserve que lorsque x = 0 alors R₂₃ désigne R₂₇ et que lorsque z = 0 alors R₂₅ désigne R₂₉.

17. Procédé selon la revendication précédente, dans lequel la composition cosmétique dite deuxième comprend un ou plusieurs tensioactifs cationiques choisis parmi les sels d'ammonium de formule (la) ou (IVa), de préférence de formul (IVa) dans laquelle :
- R₂₂ désigne un groupe méthyle ou éthyle,
- x et y sont égaux à 1,
- z est égal à 0 ou 1,
- r, s et t sont égaux à 2,
- R₂₃ est choisi parmi le groupe R₂₆-C(=O)-; les groupes méthyle, éthyle ou hydrocarbonés en C₁₄-C₂₂, l'atome d'hydrogène,
- R₂₅ est choisi parmi le groupe R₂₈-C(=O)-; l'atome d'hydrogène,
- R₂₄, R₂₆ et R₂₈, identiques ou différents, sont choisis parmi les groupes hydrocarbonés en C₁₃-C₁₇, linéaires ou ramifiés, saturés ou insaturés, et de préférence parmi les groupes alkyle et alcényle en C₁₃-C₁₇, linéaires ou ramifiés, saturés ou insaturés ;
et encore mieux choisis parmi les sels de cétyltriméthylammonium, de béhényltriméthylammonium, de dipalmitoyléthylhydroxyéthylméthylammonium et leurs mélanges; et plus particulièrement parmi le chlorure ou le méthosulfate de béhényltriméthylammonium, le chlorure ou le méthosulfate de cétyltriméthylammonium, le chlorure ou le méthosulfate de dipalmitoyléthylhydroxyéthylméthylammonium et leurs mélanges.

18. Procédé selon l'une des revendications précédentes, dans lequel la composition cosmétique dite deuxième comprend un ou plusieurs tensioactifs cationiques en une quantité allant de 0,05 à 10% en poids, de préférence de 0,1 à 5% en poids, préférentiellement de 0,3 à 3% en poids, par rapport au poids total de la composition.

19. Procédé selon l'une des revendications précédentes, dans lequel la composition cosmétique dite deuxième comprend un ou plusieurs organosilanes choisis parmi les composés de formule (I) et/ou leurs oligomères :
R₁Si(OR₂)_{z}(R₃)ₓ(OH)_{y} (I)
dans laquelle :
- R₁ est une chaîne hydrocarbonée en C₁ à C₂₂, notamment en C₂ à C₂₀, linéaire ou ramifiée, saturée ou insaturée, cyclique ou acyclique, pouvant être substituée par un groupement choisi parmi les groupements amine NH₂ ou NHR (R étant un alkyle linéaire ou ramifié en C₁ à C₂₀, notamment en C₁ à C₆, ou un cycloalkyle en C₃ à C₄₀ ou un radical aromatique en C₆ à C₃₀); le groupement hydroxy (OH) ; un groupement thiol ; un groupement aryle (plus particulièrement benzyle) substitué ou non par un groupement NH₂ ou NHR; R₁ pouvant être interrompu par un hétéroatome (O, S, NH) ou un groupement carbonyle (CO);
- R₂ et R₃ identiques ou différents, représentent un groupe alkyle, linéaire ou ramifié, comprenant de 1 à 6 atomes de carbone,
- y désigne un nombre entier allant de 0 à 3,
- z désigne un nombre entier allant de 0 à 3, et
- x désigne un nombre entier allant de 0 à 2,
- avec z+x+y=3.

20. Procédé selon l'une des revendications précédentes, dans lequel la composition cosmétique dite deuxième comprend le ou lesdits organosilanes en une quantité allant de 0,1 à 15% en poids, de préférence de 1 à 10% en poids, préférentiellement de 2 à 8% en poids, par rapport au poids total de la composition.

21. Procédé selon l'une des revendications précédentes, dans lequel la composition cosmétique dite deuxième comprend un ou plusieurs polymères cationiques choisis parmi :
(1) les homopolymères ou copolymères dérivés d'esters ou d'amides acryliques ou méthacryliques et comportant au moins un des motifs de formule suivante : 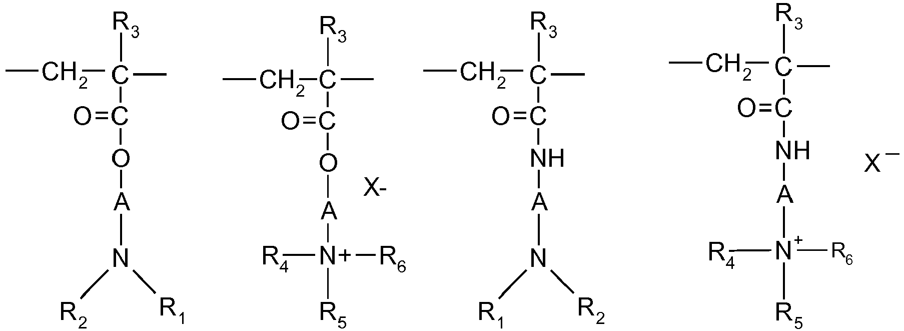 dans lesquelles:
- R3, identiques ou différents, désignent un atome d'hydrogène ou un radical CH3;
- A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
- R4, R5, R6, identiques ou différents, représentent un groupe alkyle ayant de 1 à 18 atomes de carbone ou un radical benzyle; de préférence un groupe alkyle ayant de 1 à 6 atomes de carbone;
- R1 et R2, identiques ou différents, représentent un atome d'hydrogène ou un groupe alkyle ayant de 1 à 6 atomes de carbone, de préférence méthyle ou éthyle;
- X désigne un anion dérivé d'un acide minéral ou organique tel qu'un anion méthosulfate ou un halogénure tel que chlorure ou bromure.
(2) les polymères constitués de motifs pipérazinyle et de radicaux divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères.
(3) les polyaminoamides solubles dans l'eau, préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés.
(4) les dérivés de polyaminoamides résultant de la condensation de polyalcoylènes polyamines avec des acides polycarboxyliques suivie d'une alcoylation par des agents bifonctionnels.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone; le rapport molaire entre le polyalkylène polyamine et l'acide dicarboxylique étant de préférence compris entre 0,8:1 et 1,4:1; le polyaminoamide en résultant étant amené à réagir avec l'épichlorhydrine dans un rapport molaire d'épichlorhydrine par rapport au groupement amine secondaire du polyaminoamide compris de préférence entre 0,5:1 et 1,8:1.
(6) les cyclopolymères d'alkyldiallylamine ou de dialkyldiallylammonium tels que les homopolymères ou copolymères comportant comme constituant principal de la chaîne des motifs répondant aux formules (I) ou (II) : 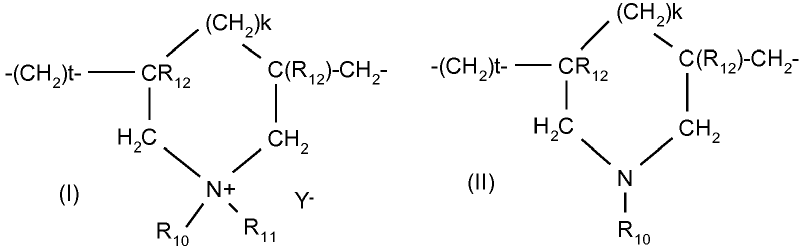 dans lesquelles
- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle en C1-C6, un groupement hydroxyalkyle en C1-C5, un groupement amidoalkyle en C1-C4; ou bien R10 et R11 peuvent désigner conjointement avec l'atome d'azote auquel ils sont rattachés, un groupement hétérocyclique tel que pipéridinyle ou morpholinyle; R10 et R11, indépendamment l'un de l'autre, désignent de préférence un groupement alkyle en C1-C4;
- Y⁻ est un anion tel que bromure, chlorure, acétate, borate, citrate, tartrate, bisulfate, bisulfite, sulfate, phosphate.
(7) les polymères de diammonium quaternaire comprenant des motifs récurrents de formule : 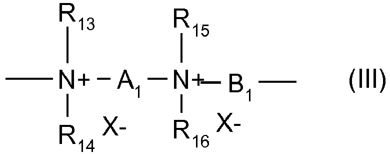 dans laquelle :
- R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques en C1-C12,
ou bien R13, R14, R15 et R16, ensemble ou séparément, constituent avec les atomes d'azote auxquels ils sont rattachés des hétérocycles comprenant éventuellement un second hétéroatome autre que l'azote
ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
- A1 et B1 représentent des groupements divalents polyméthyléniques comprenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
- X⁻ désigne un anion dérivé d'un acide minéral ou organique;
étant entendu que A1, R13 et R15 peuvent former avec les deux atomes d'azote auxquels ils sont rattachés un cycle pipérazinique ;
en outre si A1 désigne un radical alkylène ou hydroxyalkylène linéaire ou ramifié, saturé ou insaturé, B1 peut également désigner un groupement (CH2)n-CO-D-OC-(CH2)p-, avec n et p, identiques ou différents, étant des entiers variant de 2 à 20, et D désigne :
a) un reste de glycol de formule -O-Z-O-, où Z désigne un radical hydrocarboné linéaire ou ramifié ou un groupement répondant à l'une des formules suivantes: -(CH2CH2O)x-CH2CH2- et -[CH2CH(CH3)O]y-CH2CH(CH3)- où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule -NH-Y-NH- où Y désigne un radical hydrocarboné linéaire ou ramifié, ou bien le radical divalent -CH2-CH2-S-S-CH2-CH2- ;
d) un groupement uréylène de formule -NH-CO-NH- .
De préférence, X⁻ est un anion tel que le chlorure ou le bromure. Ces polymères ont une masse molaire moyenne en nombre (Mn) généralement comprise entre 1000 et 100000.
(8) les polymères de polyammonium quaternaires comprenant des motifs de formule (V): 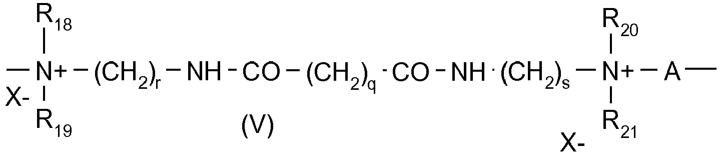 dans laquelle :
- R18, R19, R20 et R21, identiques ou différents, représentent un atome d'hydrogène ou un radical méthyle, éthyle, propyle, β-hydroxyéthyle, β-hydroxypropyle ou
- CH2CH2(OCH2CH2)pOH, où p est égal à 0 ou à un nombre entier compris entre 1 et 6, sous réserve que R18, R19, R20 et R21 ne représentent pas simultanément un atome d'hydrogène,
- r et s, identiques ou différents, sont des nombres entiers compris entre 1 et 6,
- q est égal à 0 ou à un nombre entier compris entre 1 et 34,
- X- désigne un anion tel qu'un halogénure,
- A désigne un radical divalent d'un dihalogénure ou représente de rence -CH2-CH2-O-CH2-CH2-.
(9) Les polymères quaternaires de vinylpyrrolidone et de vinylimidazole ;
(10) les polymères comportant dans leur structure :
(a) un ou plusieurs motifs répondant à la formule (A) suivante : 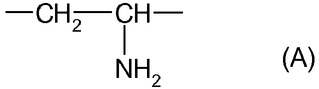
(b) éventuellement un ou plusieurs motifs répondant à la formule (B) suivante : 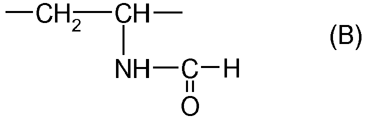 de préférence choisis parmi ceux des familles (1), (6) et (7).

22. Procédé selon l'une des revendications précédentes, dans lequel la composition cosmétique dite deuxième comprend un ou plusieurs polymères cationiques choisis parmi le chlorure de 2-méthacryloyloxyéthyltriméthylammonium (Polyquaternium-37), le chlorure de diméthyldiallylammonium (Polyquaternium-6) et leurs mélanges.

23. Procédé selon l'une des revendications précédentes, dans lequel la composition cosmétique dite deuxième comprend un ou plusieurs polymères cationiques ayant une densité de charge supérieure ou égale à 4 méq/g, en une quantité allant de 0,01 à 15% en poids, de préférence de 0,1 à 10% en poids, préférentiellement de 0,2 à 5% en poids, par rapport au poids total de la composition.

24. Procédé selon l'une des revendications précédentes, dans lequel la composition cosmétique dite deuxième comprend de l'eau à une concentration allant de préférence de 40 à 99% en poids, notamment de 50 à 95% en poids, mieux de 60 à 90% en poids, par rapport au poids total de ladite composition.

25. Procédé selon l'une des revendications précédentes, dans lequel la composition cosmétique dite deuxième comprend un ou plusieurs acides organiques, de préférence choisis parmi les acides carboxyliques, saturés ou insaturés ; les acides sulfoniques et leurs mélanges.

26. Procédé selon l'une des revendications précédentes, comprenant, antérieurement à ladite étape (i) d'application sur lesdits cheveux, d'une composition cosmétique dite première, une étape dite de lavage des cheveux, comprenant l'application sur lesdits cheveux d'une composition lavante, comprenant de préférence un ou plusieurs tensioactifs détergents; de préférence, ladite étape antérieure de lavage étant suivie d'une étape de rinçage, par exemple à l'eau.

27. Procédé selon la revendication 26, dans lequel la composition lavante comprend un ou plusieurs tensioactifs notamment choisis parmi les tensioactifs anioniques, les tensioactifs amphotères, et leurs mélanges ; de préférence présents en une teneur totale qui peut aller de 5 à 35% en poids, de préférence de 10 à 30% en poids, préférentiellement de 15 à 25% en poids, par rapport au poids total de la composition.

28. Procédé selon l'une des revendications 26 à 27, dans lequel la composition lavante comprend un ou plusieurs polymères cationiques ayant une densité de charge supérieure ou égale à 4 méq/g, de préférence supérieure ou égale à 5 méq/g ; notamment ayant une densité de charge cationique allant de 4 à 20 méq/g, voire de 5 à 20 méq/g ; de préférence, lesdits polymères cationiques étant choisis parmi ceux des familles (1), (6) et (7) ci-dessus en revendication 21 ; encore mieux choisis parmi le chlorure de 2-méthacryloyloxyéthyl triméthyl ammonium (Polyquaternium-37), le chlorure de diméthyl diallyl ammonium (Polyquaternium-6) et leurs mélanges.

29. Procédé selon l'une des revendications 26 à 28, dans lequel la composition lavante comprend un ou plusieurs polymères cationiques ayant une densité de charge supérieure ou égale à 4 méq/g, en une quantité allant de 0,01 à 15% en poids, de préférence de 0,1 à 10% en poids, préférentiellement de 0,2 à 5% en poids, par rapport au poids total de ladite composition.

30. Procédé selon l'une des revendications 26 à 29, dans lequel la composition lavante comprend au moins un polymère cationique de densité de charge supérieure ou égale à 4 méq/g identique à au moins un polymère cationique de densité de charge supérieure ou égale à 4 méq/g présent dans la deuxième composition cosmétique.

31. Procédé selon l'une des revendications précédentes, pour le nettoyage et/ou le soin et/ou le conditionnement des cheveux, notamment permettant l'obtention d'un conditionnement à la fois immédiat et durable.

## Patentansprüche

1. Verfahren zur kosmetischen Haarbehandlung, umfassend:
- einen Schritt (i) zum Auftragen einer sogenannten ersten kosmetischen Zusammensetzung auf die Haare, umfassend ein oder mehrere kationische Tenside, ein oder mehrere Silikone und ein oder mehrere Nicht-Silikon-Fettstoffe, dann
- einen Schritt (ii) zum Auftragen einer sogenannten zweiten kosmetischen Zusammensetzung auf die Haare, umfassend ein oder mehrere kationische Tenside, ein oder mehrere kationische Polymere, die eine Kationen-Ladungsdichte größer oder gleich 4 meq/g aufweisen, und ein oder mehrere Organosilane;
wobei das Verfahren zwischen Auftragschritt (i) und Auftragschritt (ii) keinen Spülschritt umfasst.

2. Verfahren nach Anspruch 1, wobei auf den Schritt (i) zum Auftragen ein Schritt zum Einwirkenlassen der sogenannten ersten kosmetischen Zusammensetzung, vorzugsweise für 1 bis 15 Minuten, folgt.

3. Verfahren nach einem der vorhergehenden Ansprüche, wobei auf den Schritt (ii) zum Auftragen ein Schritt zum Einwirkenlassen der sogenannten zweiten kosmetischen Zusammensetzung, vorzugsweise für 1 bis 25 Minuten, insbesondere 1 bis 20 Minuten, und ein Schritt zum Spülen, dann gegebenenfalls ein Schritt zum Trocknen folgt.

4. Verfahren nach einem der vorhergehenden Ansprüche, wobei die sogenannte erste kosmetische Zusammensetzung ein oder mehrere kationische Tenside umfasst, ausgewählt aus gegebenenfalls polyoxyalkylenierten, primären, sekundären oder tertiären Fettaminsalzen; quaternären Ammoniumsalzen und Mischungen davon; und insbesondere ausgewählt aus:
- quaternären Ammoniumsalzen der Formel (Ia): 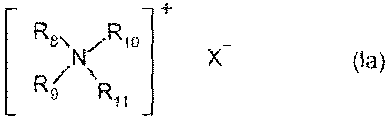 wobei:
die Gruppen R₈ à R₁₁, gleich oder verschieden, für eine aliphatische lineare oder verzweigte Gruppe, umfassend 1 bis 30 Kohlenstoffatome, oder eine aromatische Gruppe, wie Aryl oder Alkylaryl, stehen, wobei mindestens eine der Gruppen R₈ bis R₁₁ 8 bis 30 Kohlenstoffatome, vorzugsweise 12 bis 24 Kohlenstoffatome umfasst; die aliphatischen Gruppen Heteroatome, wie insbesondere Sauerstoff, Stickstoff, Schwefel und Halogene, umfassen können; und
- X⁻ ein Anion ist, das insbesondere ausgewählt ist aus der Gruppe der Halogenide, Phosphate, Acetate, Lactate, Alkyl (C₁-C₄) sulfate, Alkyl(C₁-C₄)sulfonate oder Alkyl(C₁-C₄)arylsulfonate;
- quaternären Ammoniumsalzen von Imidazolin der Formel (IIa): 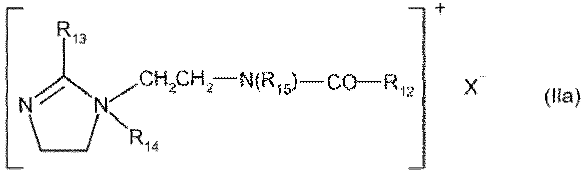 wobei:
R₁₂ für eine Alkenyl- oder Alkylgruppe steht, umfassend 8 bis 30 Kohlenstoffatome, zum Beispiel abgeleitet von Talgfettsäuren,
R₁₃ für ein Wasserstoffatom, eine C₁-C₄-Alkylgruppe oder eine Alkenyl- oder Alkylgruppe, umfassend 8 bis 30 Kohlenstoffatome, steht,
R₁₄ für eine C₁-C₄-Alkylgruppe steht,
R₁₅ für ein Wasserstoffatom oder eine C₁-C₄-Alkylgruppe steht,
X⁻ ein Anion ist, das insbesondere ausgewählt ist aus der Gruppe der Halogenide, Phosphate, Acetate, Lactate, Alkyl (C₁-C₄) sulfate, Alkyl(C₁-C₄)sulfonate oder Alkyl(C₁-C₄)arylsulfonate;
- quaternären Di- oder Triammoniumsalzen der Formel (IIIa): 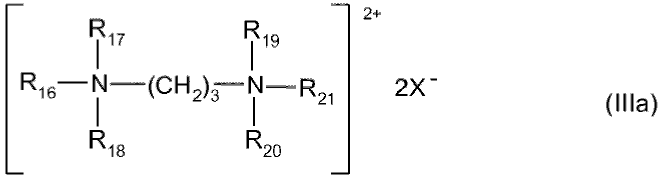 wobei:
R₁₆ eine Alkylgruppe bezeichnet, umfassend 16 bis 30 Kohlenstoffatome, die gegebenenfalls hydroxyliert und/oder gegebenenfalls durch ein oder mehrere Sauerstoffatome unterbrochen ist,
R₁₇ Wasserstoff, eine Alkylgruppe, umfassend 1 bis 4 Kohlenstoffatome oder eine Gruppe -(CH₂)₃-N⁺(R₁₆ₐ)(R₁₇ₐ)(R₁₈ₐ) bezeichnet, wobei R₁₆ₐ, R₁₇ₐ, R₁₈ₐ, gleich oder verschieden, den Wasserstoff oder eine Alkylgruppe, umfassend 1 bis 4 Kohlenstoffatome, bezeichnet,
- R₁₈, R₁₉, R₂₀ und R₂₁, gleich oder verschieden, den Wasserstoff oder eine Alkylgruppe, umfassend 1 bis 4 Kohlenstoffatome, bezeichnen und
- X⁻ ein Anion ist, das insbesondere ausgewählt ist aus der Gruppe der Halogenide, Acetate, Phosphate, Nitrate, Alkyl (C₁-C₄) sulfate, Alkyl(C₁-C₄)sulfonate und Alkyl (C₁-C₄) arylsulfonate, insbesondere Methylsulfat und Ethylsulfat;
- quaternären Ammoniumsalzen, die eine oder mehrere Esterfunktionen enthalten, der folgenden Formel (IVa): 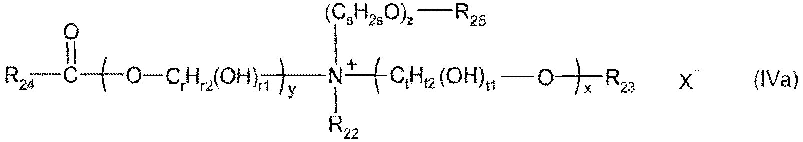 wobei:
- R₂₂ ausgewählt ist aus C₁-C₆-Alkylgruppen und C₁-C₆-Hydroxyalkyl- oder C₁-C₆-Dihydroxyalkylgruppen,
- R₂₃ ausgewählt ist aus der Gruppe R₂₆-C(=O)-; den linearen oder verzweigen, gesättigten oder ungesättigten C₁-C₂₂-Kohlenwasserstoffgruppen R₂₇; und dem Wasserstoffatom,
- R₂₅ ausgewählt ist aus der Gruppe R₂₈-C(=O)-; den linearen oder verzweigen, gesättigten oder ungesättigten C₁-C₆-Kohlenwasserstoffgruppen R₂₉; und dem Wasserstoffatom,
- R₂₄, R₂₆ und R₂₈, gleich oder verschieden, ausgewählt sind aus den linearen oder verzweigen, gesättigten oder ungesättigten C₇-C₂₁-Kohlenwasserstoffgruppen,
- r, s und t, gleich oder verschieden, ganze Zahlen von 2 bis 6 sind,
- r1 und t1, gleich oder verschieden, 0 oder 1 sind,
- y eine ganze Zahl von 1 bis 10 ist,
- x und z, gleich oder verschieden, ganze Zahlen von 0 bis 10 sind,
- X⁻ ein Anion ist,
mit der Maßgabe, dass r2 + r1 = 2r und t1 + t2 = 2t, und dass die Summe x + y + z gleich 1 bis 15 ist,
vorausgesetzt, dass wenn x = 0 ist, dann R₂₃ R₂₇ bezeichnet und dass wenn z = 0 ist, R₂₅ R₂₉ bezeichnet.

5. Verfahren nach dem vorhergehenden Anspruch, wobei die sogenannte erste kosmetische Zusammensetzung ein oder mehrere kationische Tenside umfasst, ausgewählt aus den Ammoniumsalzen der Formel (Ia) oder (IVa), vorzugsweise der Formel (IVa), wobei:
- R₂₂ eine Methyl- oder Ethylgruppe bezeichnet,
- x und y gleich 1 sind,
- z gleich 0 oder 1 ist,
- r, s und t gleich 2 sind,
- R₂₃ ausgewählt ist aus der Gruppe R₂₆-C(=O)-; den Methyl-, Ethyl- oder C₁₄-C₂₂-Kohlenwasserstoffgruppen; dem Wasserstoffatom,
- R₂₅ ausgewählt ist aus der Gruppe R₂₈-C(=O)-; dem Wasserstoffatom,
- R₂₄, R₂₆ und R₂₈, gleich oder verschieden, ausgewählt sind aus den linearen oder verzweigten, gesättigten oder ungesättigten C₁₃-C₁₇-Kohlenwasserstoffgruppen, und vorzugsweise aus den linearen oder verzweigten, gesättigten oder ungesättigten C₁₃-C₁₇-Alkyl- und Alkenylgruppen;
und noch besser ausgewählt sind aus den Cetyltrimethylammonium-, Behenyltrimethylammonium-, Dipalmitoylethylhydroxyethylmethylammoniumsalzen und Mischungen davon; und insbesondere aus Behenyltrimethylammoniumchlorid oder -methosulfat, Cetyltrimethylammoniumchlorid oder -methosulfat, Dipalmitoylethylhydroxyethylmethylammoniumchlorid oder -methosulfat und Mischungen davon.

6. Verfahren nach einem der vorhergehenden Ansprüche, wobei die sogenannte erste kosmetische Zusammensetzung mindestens zwei verschiedene kationische Tenside umfasst; insbesondere mindestens ein kationisches Tensid ausgewählt aus denjenigen der Formel (Ia) und mindestens ein kationisches Tensid ausgewählt aus denjenigen der Formel (IVa), wobei die Formeln wie in Anspruch 4 definiert sind.

7. Verfahren nach einem der vorhergehenden Ansprüche, wobei die sogenannte erste kosmetische Zusammensetzung ein oder mehrere kationische Tenside in einer Menge im Bereich von 0,1 bis 3,5 Gew.-%, vorzugsweise von 0,2 bis 3,5 Gew.-%, bevorzugt von 0,3 bis 3 Gew.-%, bezogen auf das Gesamtgewicht der Zusammensetzung, umfasst.

8. Verfahren nach einem der vorhergehenden Ansprüche, wobei die die sogenannte erste kosmetische Zusammensetzung ein oder mehrere Aminosilikone umfasst, insbesondere ausgewählt aus:
a) Polysiloxanen mit der Formel (A): 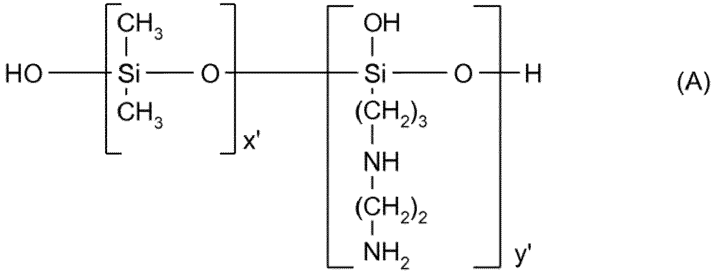 wobei x' und y' ganze Zahlen sind, so dass das gewichtsmittlere Molekulargewicht (Mw) etwa zwischen 5.000 und 500.000 liegt;
b) Aminosilikonen der Formel (B):
R'ₐG₃₋ₐ-Si(OSiG₂)ₙ-(OSiG_{b}R'_{2-b})ₘ-O-SiG₃₋ₐ-R'ₐ (B)
wobei:
- G, gleich oder verschieden, ein Wasserstoffatom, eine Phenyl-, OH-, C₁-C₈-Alkyl-, zum Beispiel Methyl-, oder C₁-C₈-Alkoxy-, zum Beispiel Methoxygruppe, bezeichnet,
- a, gleich oder verschieden, 0 oder eine ganze Zahl von 1 bis 3, insbesondere 0, bezeichnet,
- b 0 oder 1, insbesondere 1, bezeichnet,
- m und n Zahlen sind, so dass die Summe (n + m) im Bereich von 1 bis 2.000, insbesondere von 50 bis 150 liegt, wobei n eine Zahl von 0 bis 1.999, und insbesondere von 49 bis 149 bezeichnen kann, und m eine Zahl von 1 bis 2.000 und insbesondere von 1 bis 10 bezeichnen kann;
- R', gleich oder verschieden, einen einwertigen Rest der Formel -CqH2qL bezeichnet, wobei q eine Zahl im Bereich von 2 bis 8 ist, und L eine gegebenenfalls quaternisierte Aminogruppe ist, ausgewählt aus den Gruppen:
- N(R")₂; -N⁺(R")₃A-; -NR"-Q-N(R")₂ und -NR"-Q-N⁺(R")₃A-,
wobei R", gleich oder verschieden, Wasserstoff, Phenyl, Benzyl oder einen gesättigten einwertigen Kohlenwasserstoffrest, zum Beispiel einen C1-C20-Alkylrest, bezeichnet; Q eine lineare oder verzweigte Gruppe der Formel CᵣH₂ᵣ bezeichnet, wobei r eine ganze Zahl im Bereich von 2 bis 6, vorzugsweise von 2 bis 4 ist; und A- für ein kosmetisch verträgliches Anion, insbesondere ein Halogenid, wie etwa Fluorid, Chlorid, Bromid oder Iodid, steht;
c) Aminosilikonen mit der Formel (H): 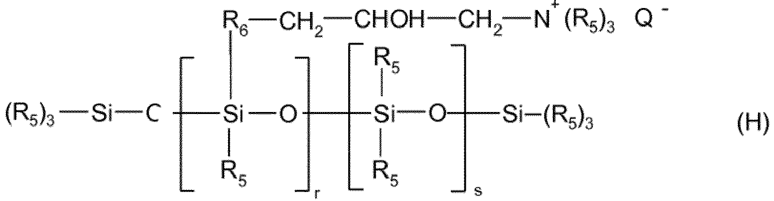 wobei:
- R₅ für einen einwertigen Kohlenwasserstoffrest mit 1 bis 18 Kohlenstoffatomen und insbesondere einen C₁-C₁₈-Alkylrest, oder einen C₂-C₁₈-Alkenylrest, zum Beispiel einen C₂-C₁₈-Methylrest, steht;
- R₆ für einen zweiwertigen Kohlenwasserstoffrest, insbesondere einen C₁-C₁₈-Alkylenrest oder einen zweiwertigen C₁-C₁₈-Alkylenoxyrest, zum Beispiel einen C₁-C₈-Alkylenoxyrest steht, der über eine SiC-Bindung an das Si gebunden ist;
- Q⁻ ein Anion ist, wie etwa ein Halogenidion, Chloridion, oder ein Salz einer organischen Säure, insbesondere Acetat;
- r für einen durchschnittlichen statistischen Wert im Bereich von 2 bis 20, insbesondere von 2 bis 8 steht;
- s für einen durchschnittlichen statistischen Wert im Bereich von 20 bis 200, insbesondere von 20 bis 50 steht;
d) quaternären Ammoniumsilikonen der Formel (I): 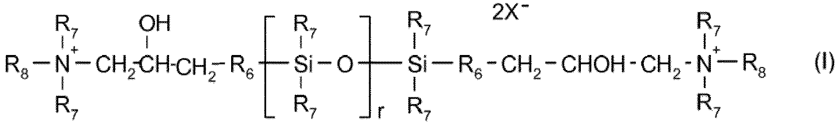 wobei:
- R₇, gleich oder verschieden, für einen einwertigen Kohlenwasserstoffrest mit 1 bis 18 Kohlenstoffatomen, und insbesondere einen C₁-C₁₈-Alkylrest, einen C₂-C₁₈-Alkenylrest oder einen Ring, umfassend 5 oder 6 Kohlenstoffatome, zum Beispiel Methyl, steht;
- R₆ für einen zweiwertigen Kohlenwasserstoffrest, insbesondere einen C₁-C₁₈-Alkylenrest oder einen zweiwertigen C₁-C₁₈-Alkylenoxyrest, zum Beispiel einen C₁-C₈-Alkylenoxyrest steht, der über eine SiC-Bindung an das Si gebunden ist;
- R₈, gleich oder verschieden, für ein Wasserstoffatom, einen einwertigen Kohlenwasserstoffrest mit 1 bis 18 Kohlenstoffatomen, und insbesondere einen C₁-C₁₈-Alkylrest, einen C₂-C₁₈-Alkenylrest, einen -R₆-NHCOR₇-Rest steht;
- X⁻ ein Anion ist, wie etwa ein Halogenidion, Chloridion, oder ein Salz einer organischen Säure, insbesondere Acetat;
- r für einen durchschnittlichen statistischen Wert im Bereich von 2 bis 200, insbesondere von 5 bis 100 steht;
e) Aminosilikonen der Formel (J): 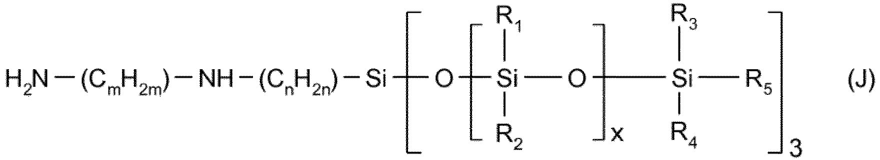 wobei:
- R₁, R₂, R₃ und R₄, gleich oder verschieden, einen C₁-C₄-Alkylrest oder eine Phenylgruppe bezeichnen,
- R₅ einen C₁-C₄-Alkylrest oder eine Hydroxylgruppe bezeichnet,
- n eine ganze Zahl im Bereich von 1 bis 5 ist,
- m eine ganze Zahl im Bereich von 1 bis 5 ist, und
- x so ausgewählt ist, dass die Aminzahl im Bereich von 0,01 bis 1 meq/g liegt;
f) polyoxyalkylenierten Mehrblock-Aminosilikonen des Typs (AB)n, wobei A ein Polysiloxanblock ist und B ein Polyoxyalkylenblock ist, der mindestens eine Amingruppe umfasst.

9. Verfahren nach einem der vorhergehenden Ansprüche, wobei die sogenannte erste kosmetische Zusammensetzung ein oder mehrere Aminosilikone umfasst, ausgewählt aus:
- "Trimethylsilylamodimethicon" genannten Silikonen mit der Formel (C): 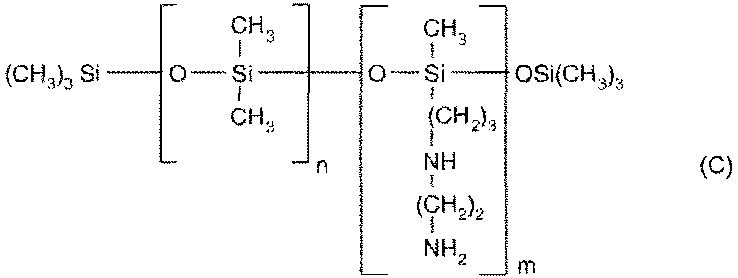 wobei m und n Zahlen sind, so dass die Summe (n + m) im Bereich von 1 bis 2.000, insbesondere von 50 bis 150 liegt, wobei n eine Zahl von 0 bis 1.999, und insbesondere von 49 bis 149 bezeichnen kann, und m eine Zahl von 1 bis 2.000 und insbesondere von 1 bis 10 bezeichnen kann;
- Silikonen der folgenden Formel (D): 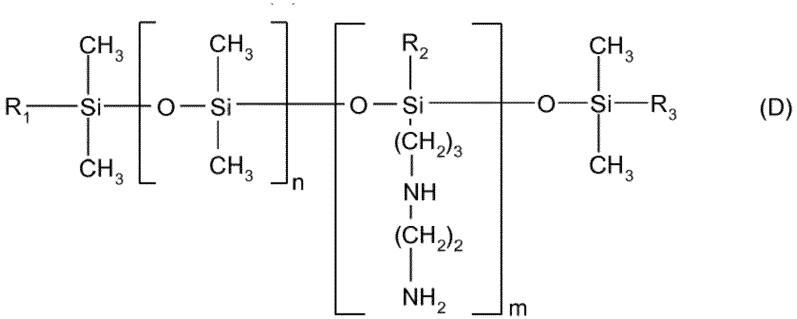 wobei:
- m und n Zahlen sind, so dass die Summe (n + m) im Bereich von 1 bis 1.000, insbesondere von 50 bis 250 und bevorzugter von 100 bis 200 liegt; wobei n eine Zahl von 0 bis 999 und insbesondere von 49 bis 249 und bevorzugter von 125 bis 175 bezeichnen kann und m eine Zahl von 1 bis 1.000, insbesondere von 1 bis 10, bevorzugter von 1 bis 5 bezeichnen kann;
- R1, R2, R3, gleich oder verschieden, für einen Hydroxy- oder C₁-C₄-Alkoxyrest stehen, wobei mindestens einer der Reste R1 bis R3 für einen Alkoxyrest steht;
- Silikonen der folgenden Formel (E): 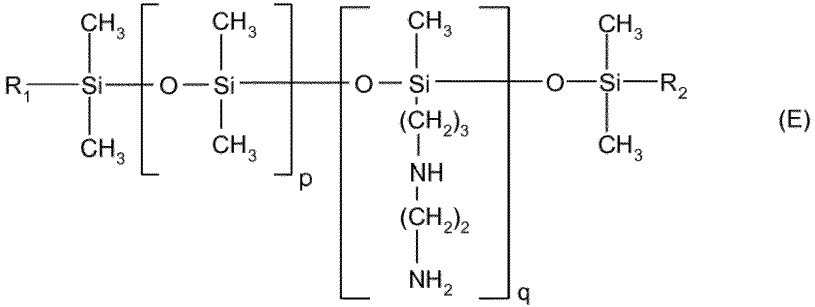 wobei:
- p und q Zahlen sind, so dass die Summe (p+q) im Bereich von 1 bis 1.000, insbesondere von 50 bis 350 und bevorzugter von 150 bis 250 liegt; p eine Zahl von 0 bis 999 und insbesondere von 49 bis 349 und bevorzugter von 159 bis 239 bezeichnen kann und q eine Zahl von 1 bis 1.000, insbesondere von 1 bis 10 und bevorzugter von 1 bis 5 bezeichnen kann;
- R1, R2, verschieden, für einen Hydroxy- oder C₁-C₄-Alkoxyrest stehen, wobei mindestens einer der Reste R1 oder R2 für einen Alkoxyrest steht;
- Silikonen der folgenden Formel (F): 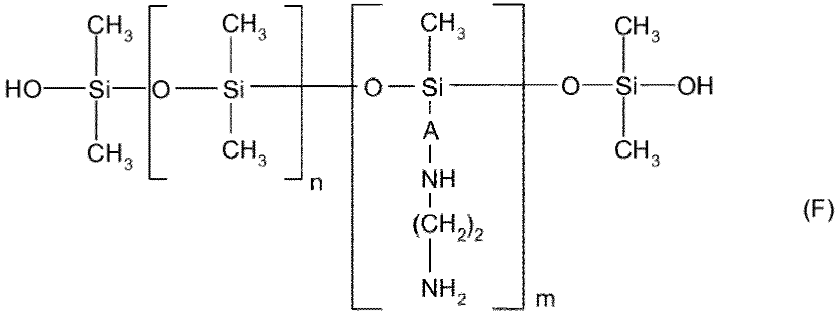 wobei:
- m und n Zahlen sind, so dass die Summe (n + m) im Bereich von 1 bis 2.000 und insbesondere von 50 bis 150 liegt, wobei n eine Zahl von 0 bis 1.999, und insbesondere von 49 bis 149 bezeichnen kann, und m eine Zahl von 1 bis 2.000 und insbesondere von 1 bis 10 bezeichnen kann;
- A einen linearen oder verzweigten Alkylenrest mit 4 bis 8 Kohlenstoffatomen und vorzugsweise 4 Kohlenstoffatomen bezeichnet. Dieser Rest ist vorzugsweise linear.
- Silikonen der folgenden Formel (G): 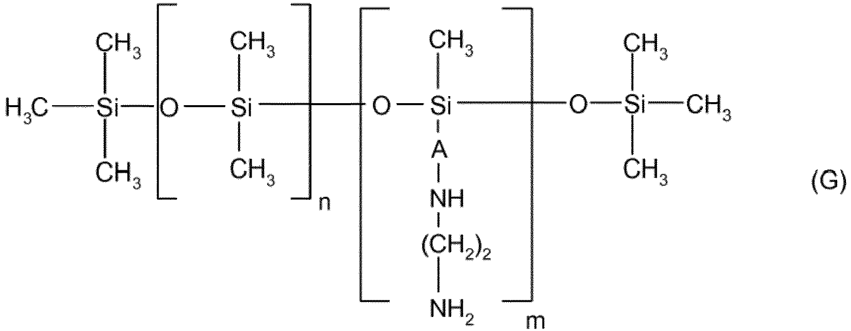 wobei:
- m und n Zahlen sind, so dass die Summe (n + m) im Bereich von 1 bis 2.000 und insbesondere von 50 bis 150 liegt, wobei n eine Zahl von 0 bis 1.999, und insbesondere von 49 bis 149 bezeichnen kann, und m eine Zahl von 1 bis 2.000 und insbesondere von 1 bis 10 bezeichnen kann;
- A einen linearen oder verzweigten Alkylenrest mit 4 bis 8 Kohlenstoffatomen und vorzugsweise 4 Kohlenstoffatomen bezeichnet;
und insbesondere mit der Formel (F).

10. Verfahren nach einem der vorhergehenden Ansprüche, wobei die sogenannte erste kosmetische Zusammensetzung das oder die Silikone in einer Menge im Bereich von 0,1 bis 2 Gew.-%, vorzugsweise von 0,2 bis 1,7 Gew.-%, bevorzugt von 0,3 bis 1,6 Gew.-%, bezogen auf das Gesamtgewicht der Zusammensetzung, umfasst.

11. Verfahren nach einem der vorhergehenden Ansprüche, wobei die sogenannte erste kosmetische Zusammensetzung das oder die Aminosilikone in einer Menge im Bereich von 0,1 bis 2 Gew.-%, vorzugsweise von 0,2 bis 1,7 Gew.-%, bevorzugt von 0,3 bis 1,6 Gew.-%, bezogen auf das Gesamtgewicht der Zusammensetzung, umfasst.

12. Verfahren nach einem der vorhergehenden Ansprüche, wobei die sogenannte erste kosmetische Zusammensetzung eine oder mehrere feste Nicht-Silikonfettstoffe umfasst, insbesondere ausgewählt aus Fettalkoholen, Fettsäureestern und/oder Fettalkoholestern und Nicht-Silikonwachsen, Ceramiden und Mischungen davon.

13. Verfahren nach einem der vorhergehenden Ansprüche, wobei die sogenannte erste kosmetische Zusammensetzung den oder die Nicht-Silikonfettstoffe in einer Menge im Bereich von 0,1 bis 20 Gew.-%, vorzugsweise von 0,5 bis 15 Gew.-%, vorzugsweise von 1 bis 10 Gew.-%, bezogen auf das Gesamtgewicht der Zusammensetzung, umfasst.

14. Verfahren nach einem der vorhergehenden Ansprüche, wobei die sogenannte erste kosmetische Zusammensetzung den oder die festen Nicht-Silikonfettstoffe in einer Menge im Bereich von 0,5 bis 15 Gew.-%, vorzugsweise 1 bis 10 Gew.-%, vorzugsweise von 1,5 bis 8 Gew.-%, bezogen auf das Gesamtgewicht der Zusammensetzung, umfasst.

15. Verfahren nach einem der vorhergehenden Ansprüche, wobei die sogenannte erste kosmetische Zusammensetzung Wasser in einer Konzentration vorzugsweise im Bereich von 50 bis 99 Gew.-%, insbesondere von 60 bis 98 Gew.-%, besser von 70 bis 97 Gew.-%, bezogen auf das Gesamtgewicht der Zusammensetzung, umfasst.

16. Verfahren nach einem der vorhergehenden Ansprüche, wobei die sogenannte zweite kosmetische Zusammensetzung ein oder mehrere kationische Tenside umfasst, ausgewählt aus gegebenenfalls polyoxyalkylenierten, primären, sekundären oder tertiären Fettaminsalzen; quaternären Ammoniumsalzen und Mischungen davon;
und insbesondere ausgewählt aus:
- quaternären Ammoniumsalzen der Formel (Ia): 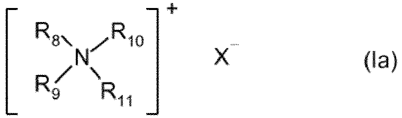 wobei:
die Gruppen R₈ bis R₁₁, gleich oder verschieden, für eine aliphatische lineare oder verzweigte Gruppe, umfassend 1 bis 30 Kohlenstoffatome, oder eine aromatische Gruppe, wie Aryl oder Alkylaryl, stehen, wobei mindestens eine der Gruppen R₈ bis R₁₁ 8 bis 30 Kohlenstoffatome, vorzugsweise 12 bis 24 Kohlenstoffatome umfasst; die aliphatischen Gruppen Heteroatome, wie insbesondere Sauerstoff, Stickstoff, Schwefel und Halogene umfassen können; und
- X⁻ ein Anion ist, das insbesondere ausgewählt ist aus der Gruppe der Halogenide, Phosphate, Acetate, Lactate, Alkyl (C₁-C₄) sulfate, Alkyl(C₁-C₄)sulfonate oder Alkyl(C₁-C₄)arylsulfonate;
- quaternären Ammoniumsalzen von Imidazolin der Formel (IIa): 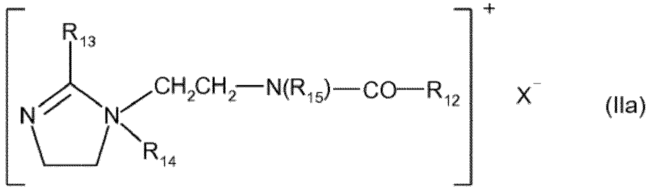 wobei:
R₁₂ für eine Alkenyl- oder Alkylgruppe steht, umfassend 8 bis 30 Kohlenstoffatome, zum Beispiel abgeleitet von Talgfettsäuren,
R₁₃ für ein Wasserstoffatom, eine C₁-C₄-Alkylgruppe oder eine Alkenyl- oder Alkylgruppe, umfassend 8 bis 30 Kohlenstoffatome, steht,
R₁₄ für eine C₁-C₄-Alkylgruppe steht,
R₁₅ für ein Wasserstoffatom oder eine C₁-C₄-Alkylgruppe steht,
X⁻ ein Anion ist, das insbesondere ausgewählt ist aus der Gruppe der Halogenide, Phosphate, Acetate, Lactate, Alkyl (C₁-C₄) sulfate, Alkyl(C₁-C₄)sulfonate oder Alkyl(C₁-C₄)arylsulfonate;
- quaternären Di- oder Triammoniumsalzen der Formel (IIIa): 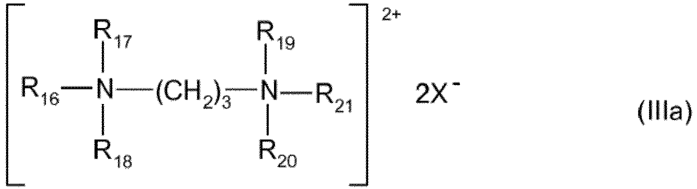 wobei:
- R₁₆ eine Alkylgruppe bezeichnet, umfassend 16 bis 30 Kohlenstoffatome, die gegebenenfalls hydroxyliert und/oder gegebenenfalls durch ein oder mehrere Sauerstoffatome unterbrochen ist,
- R₁₇ Wasserstoff, eine Alkylgruppe, umfassend 1 bis 4 Kohlenstoffatome oder eine Gruppe -(CH₂)₃-N⁺ (R₁₆ₐ) (R₁₇ₐ) (R₁₈ₐ) bezeichnet, wobei R₁₆ₐ, R₁₇ₐ, R₁₈ₐ, gleich oder verschieden, den Wasserstoff oder eine Alkylgruppe, umfassend 1 bis 4 Kohlenstoffatome, bezeichnen,
- R₁₈, R₁₉, R₂₀ und R₂₁, gleich oder verschieden, den Wasserstoff oder eine Alkylgruppe, umfassend 1 bis 4 Kohlenstoffatome, bezeichnen und
- X⁻ ein Anion ist, insbesondere ausgewählt aus der Gruppe der Halogenide, Acetate, Phosphate, Nitrate, Alkyl (C₁-C₄) sulfate, Alkyl(C₁-C₄)sulfonate und Alkyl (C₁-C₄) arylsulfonate, insbesondere Methylsulfat und Ethylsulfat;
- quaternären Ammoniumsalzen, die eine oder mehrere Esterfunktionen enthalten, der folgenden Formel (IVa): 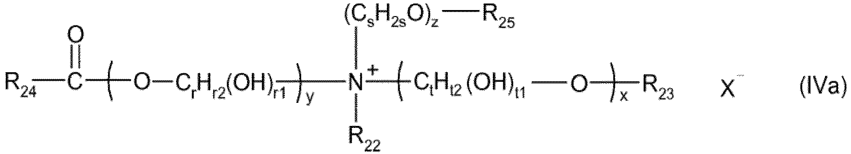 wobei:
- R₂₂ ausgewählt ist aus den C₁-C₆-Alkylgruppen und den C₁-C₆-Hydroxyalkyl- oder C₁-C₆-Dihydroxyalkylgruppen,
- R₂₃ ausgewählt ist aus der Gruppe R₂₆-C(=O)-; den linearen oder verzweigen, gesättigten oder ungesättigten C₁-C₂₂-Kohlenwasserstoffgruppen R₂₇; und dem Wasserstoffatom,
- R₂₅ ausgewählt ist aus der Gruppe R₂₈-C(=O)-; den linearen oder verzweigen, gesättigten oder ungesättigten C₁-C₆-Kohlenwasserstoffgruppen R₂₉; und dem Wasserstoffatom,
- R₂₄, R₂₆ und R₂₈, gleich oder verschieden, ausgewählt sind aus den linearen oder verzweigen, gesättigten oder ungesättigten C₇-C₂₁-Kohlenwasserstoffgruppen,
- r, s und t, gleich oder verschieden, ganze Zahlen von 2 bis 6 sind,
- r1 und t1, gleich oder verschieden, 0 oder 1 sind,
- y eine ganze Zahl von 1 bis 10 ist,
- x und z, gleich oder verschieden, ganze Zahlen von 0 bis 10 sind,
- X⁻ ein Anion ist,
mit der Maßgabe, dass r2 + r1 = 2r und t1 + t2 = 2t, und dass die Summe x + y + z gleich 1 bis 15 ist,
vorausgesetzt, dass wenn x = 0 ist, dann R₂₃ R₂₇ bezeichnet und dass wenn z = 0 ist, R₂₅ R₂₉ bezeichnet.

17. Verfahren nach dem vorhergehenden Anspruch, wobei die sogenannte zweite kosmetische Zusammensetzung ein oder mehrere kationische Tenside umfasst, die aus den Ammoniumsalzen der Formel (Ia) oder (IVa), vorzugsweise der Formel (IVa), ausgewählt sind, wobei:
- R₂₂ eine Methyl- oder Ethylgruppe bezeichnet,
- x und y gleich 1 sind,
- z gleich 0 oder 1 ist,
- r, s und t gleich 2 sind,
- R₂₃ ausgewählt ist aus der Gruppe R₂₆-C(=O)-; den Methyl-, Ethyl- oder C₁₄-C₂₂-Kohlenwasserstoffgruppen; dem Wasserstoffatom,
- R₂₅ ausgewählt ist aus der Gruppe R₂₈-C(=O)-; dem Wasserstoffatom,
- R₂₄, R₂₆ und R₂₈, gleich oder verschieden, ausgewählt sind aus den linearen oder verzweigten, gesättigten oder ungesättigten C₁₃-C₁₇-Kohlenwasserstoffgruppen, und vorzugsweise aus den linearen oder verzweigten, gesättigten oder ungesättigten C₁₃-C₁₇-Alkyl- und Alkenylgruppen;
und noch besser ausgewählt sind aus den Cetyltrimethylammonium-, Behenyltrimethylammonium-, Dipalmitoylethylhydroxyethylmethylammoniumsalzen und Mischungen davon; und insbesondere aus Behenyltrimethylammoniumchlorid oder -methosulfat, Cetyltrimethylammoniumchlorid oder -methosulfat, Dipalmitoylethylhydroxyethylmethylammoniumchlorid oder -methosulfat und Mischungen davon.

18. Verfahren nach einem der vorhergehenden Ansprüche, wobei die sogenannte zweite kosmetische Zusammensetzung ein oder mehrere kationische Tenside in einer Menge im Bereich von 0,05 bis 10 Gew.-%, vorzugsweise von 0,1 bis 5 Gew.-%, bevorzugt von 0,3 bis 3 Gew.-%, bezogen auf das Gesamtgewicht der Zusammensetzung, umfasst.

19. Verfahren nach einem der vorhergehenden Ansprüche, wobei die sogenannte zweite kosmetische Zusammensetzung ein oder mehrere Organosilane umfasst, ausgewählt aus den Verbindungen der Formel (I) und/oder Oligomeren davon:
**R₁Si(OR₂)_{z}(R₃)ₓ(OH)_{y}** **(I)**
wobei:
- R₁ eine lineare oder verzweigte, gesättigte oder ungesättigte, cyclische oder acyclische C₁- bis C₂₂-, insbesondere C₂- bis C₂₀-Kohlenwasserstoffkette ist, die durch eine Gruppe substituiert sein kann, ausgewählt aus den Amingruppen NH₂ oder NHR (wobei R ein lineares oder verzweigtes C₁- bis C₂₀-, insbesondere C₁- bis C₆-Alkyl oder ein C₃- bis C₄₀-Cycloalkyl oder ein aromatischer C₆- bis C₃₀-Rest ist); der Hydroxygruppe (OH); einer Thiolgruppe; einer Arylgruppe (insbesondere einer Benzylgruppe), die mit einer NH₂- oder NHR-Gruppe substituiert ist oder nicht; wobei R₁ mit einem Heteroatom (O, S, NH) oder einer Carbonylgruppe (CO) unterbrochen sein kann;
- R₂ und R₃, gleich oder verschieden, für eine lineare oder verzweigte Alkylgruppe stehen, die 1 bis 6 Kohlenstoffatome umfasst,
- y eine ganze Zahl im Bereich von 0 bis 3 bezeichnet,
- z eine ganze Zahl im Bereich von 0 bis 3 bezeichnet, und
- x eine ganze Zahl im Bereich von 0 bis 2 bezeichnet,
- wobei z+x+y=3.

20. Verfahren nach einem der vorhergehenden Ansprüche, wobei die sogenannte zweite kosmetische Zusammensetzung das oder die Organosilane in einer Menge im Bereich von 0,1 bis 15 Gew.-%, vorzugsweise von 1 bis 10 Gew.-%, bevorzugt von 2 bis 8 Gew.-%, bezogen auf das Gesamtgewicht der Zusammensetzung, umfasst.

21. Verfahren nach einem der vorhergehenden Ansprüche, wobei die sogenannte zweite kosmetische Zusammensetzung ein oder mehrere kationische Polymere umfasst, ausgewählt aus:
(1) Homopolymeren oder Copolymeren, die von Acryl- oder Methacrylestern oder -amiden abgeleitet sind und mindestens eine der Einheiten der folgenden Formel umfassen: 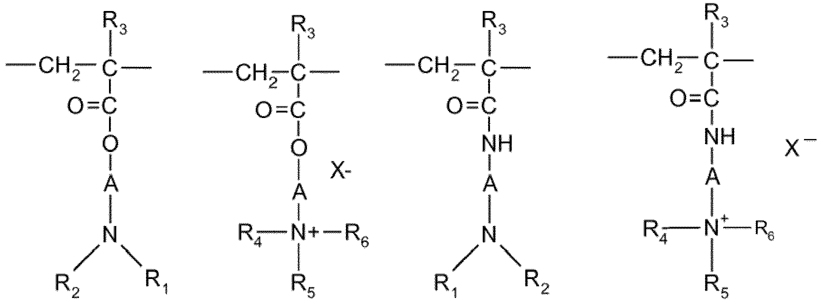 wobei:
- R3, gleich oder verschieden, ein Wasserstoffatom oder einen CH3-Rest bezeichnet;
- A, gleich oder verschieden, für eine lineare oder verzweigte zweiwertige Alkylgruppe mit 1 bis 6 Kohlenstoffatomen, vorzugsweise 2 oder 3 Kohlenstoffatomen oder eine Hydroxyalkylgruppe mit 1 bis 4 Kohlenstoffatomen steht;
- R4, R5, R6, gleich oder verschieden, für eine Alkylgruppe mit 1 bis 18 Kohlenstoffatomen oder einen Benzylrest stehen; vorzugsweise eine Alkylgruppe mit 1 bis 6 Kohlenstoffatomen;
- R1 und R2, gleich oder verschieden, für ein Wasserstoffatom oder eine Alkylgruppe mit 1 bis 6 Kohlenstoffatomen, vorzugsweise Methyl oder Ethyl, stehen;
- X ein Anion bezeichnet, das von einer mineralischen oder organischen Säure, wie etwa einem Methosulfatanion oder einem Halogenid, wie etwa Chlorid oder Bromid, abgeleitet ist;
(2) Polymeren, bestehend aus Piperazinyleinheiten und aus zweiwertigen Alkylen- oder Hydroxyalkylenresten mit linearen oder verzweigten Ketten, gegebenenfalls unterbrochen durch Sauerstoff-, Schwefel-, Stickstoffatome oder durch aromatische oder heterocyclische Ringe, sowie den Oxidations- und/oder Quaternisierungsprodukten dieser Polymere;
(3) wasserlöslichen Polyaminoamiden, die insbesondere durch Polykondensation einer sauren Verbindung mit einem Polyamin hergestellt werden; wobei diese Polyaminoamide mit einem Epihalogenhydrin, einem Diepoxid, einem Dianhydrid, einem ungesättigten Dianhydrid, einem ungesättigten Bis-Derivat, einem Bishalohydrin, einem Bisazetidinium, einem Bishaloacyldiamin, einem Bisalkylhalogenid oder alternativ mit einem Oligomer vernetzt sein können, das aus der Umsetzung einer difunktionellen Verbindung resultiert, die mit Bishalohydrin, einem Bisazetidinium, einem Bishalogenacyldiamin, einem Bisalkylhalogenid, einem Epihalogenhydrin, einem Diepoxid oder einem ungesättigten Bis-Derivat reagieren kann; wobei das Vernetzungsmittel in Anteilen im Bereich von 0,025 bis 0,35 Mol pro Amingruppe des Polyaminoamids verwendet wird; wobei diese Polyaminoamide alkyliert oder, wenn sie eine oder mehrere tertiäre Aminfunktionen enthalten, quaternisiert sein können;
(4) Polyaminoamidderivaten, die aus der Kondensation von Polyalkylenpolyaminen mit Polycarbonsäuren, gefolgt von einer Alkylierung mit difunktionellen Mitteln, resultieren;
(5) Polymeren, die durch Umsetzung eines Polyalkylenpolyamins, das zwei primäre Amingruppen und mindestens eine sekundäre Amingruppe umfasst, mit einer Dicarbonsäure, ausgewählt aus Diglykolsäure und gesättigten aliphatischen Dicarbonsäuren mit 3 bis 8 Kohlenstoffatomen erhalten werden; wobei das Molverhältnis zwischen dem Polyalkylenpolyamin und der Dicarbonsäure vorzugsweise zwischen 0,8:1 und 1,4:1 liegt; wobei das resultierende Polyaminoamid mit Epichlorhydrin in einem Molverhältnis von Epichlorhydrin zur sekundären Amingruppe des Polyaminoamids vorzugsweise zwischen 0,5:1 und 1,8:1 umgesetzt wird;
(6) Alkyldiallylamin- oder Dialkyldiallylammoniumcyclopolymeren, wie Homopolymeren oder Copolymeren, die als Hauptkettenbestandteil Einheiten der Formel (I) oder (II) enthalten: 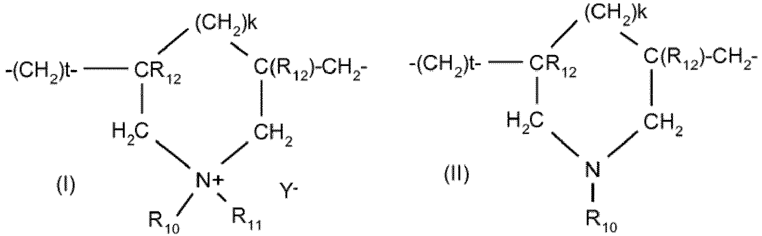 wobei:
- k und t gleich 0 oder 1 sind, wobei die Summe k + t gleich 1 ist;
- R12 ein Wasserstoffatom oder einen Methylrest bezeichnet;
- R10 und R11 unabhängig voneinander eine C1-C6-Alkylgruppe, eine C1-C5-Hydroxyalkylgruppe, eine C1-C4-Amidoalkylgruppe bezeichnen; oder R10 und R11 zusammen mit dem Stickstoffatom, an das sie gebunden sind, auch eine heterocyclische Gruppe wie Piperidyl oder Morpholinyl bezeichnen können; R10 und R11, unabhängig voneinander vorzugsweise eine C₁-C₄-Alkylgruppe bezeichnen;
- Y⁻ ein Anion wie Bromid, Chlorid, Acetat, Borat, Citrat, Tartrat, Bisulfat, Bisulfit, Sulfat oder Phosphat ist;
(7) quaternären Diammoniumpolymeren, umfassend Wiederholungseinheiten der Formel: 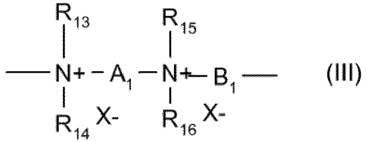 wobei:
- R13, R14, R15 und R16, gleich oder verschieden, für aliphatische, alicyclische oder arylaliphatische Reste, umfassend 1 bis 20 Kohlenstoffatome, oder für aliphatische C1-C12-Hydroxyalkylreste stehen können,
oder R13, R14, R15 und R16, zusammen oder getrennt, mit den Stickstoffatomen, an die sie gebunden sind, auch Heterocyclen bilden, die gegebenenfalls ein zweites Nicht-StickstoffHeteroatom umfassen,
oder R13, R14, R15 und R16 auch für einen linearen oder verzweigten C1-C6-Alkylrest stehen, substituiert mit einer Nitril-, Ester-, Acyl-, Amid- oder -CO-O-R17-D- oder -CO-NH-R17-D-Gruppe, wobei R17 ein Alkylen ist und D eine quaternäre Ammoniumgruppe ist;
- A1 und B1 für lineare oder verzweigte, gesättigte oder ungesättigte, zweiwertige Polymethylengruppen mit 2 bis 20 Kohlenstoffatomen stehen, die, gebunden an oder eingebettet in die Hauptkette, einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und
- X⁻ ein Anion bezeichnet, das von einer mineralischen oder organischen Säure abgeleitet ist;
mit der Maßgabe, dass A1, R13 und R15 mit den zwei Stickstoffatomen, an die sie gebunden sind, einen Piperazinring bilden können;
wobei wenn A1 ferner einen linearen oder verzweigten, gesättigten oder ungesättigten Alkylen- oder Hydroxyalkylenrest bezeichnet, B1 auch eine Gruppe (CH2)n-CO-D-OC-(CH2)p- bezeichnen kann, wobei n und p, gleich oder verschieden, ganze Zahlen im Bereich von 2 bis 20 sind, und D bezeichnet:
a) einen Glykolrest der Formel -O-Z-O-, worin Z einen linearen oder verzweigten Kohlenwasserstoffrest oder eine Gruppe bezeichnet, die einer der folgenden Formeln entspricht: -(CH2CH2O)x-CH2CH2- und -[CH2CH(CH3)O]y-CH2CH(CH3)-, worin x und y eine ganze Zahl von 1 bis 4, die für einen definierten und einzigartigen Polymerisationsgrad steht, oder eine beliebige Zahl von 1 bis 4, die für einen durchschnittlichen Polymerisationsgrad steht, bezeichnen;
b) einen sekundären Bis-Diaminrest, wie ein Piperazinderivat;
c) einen primären bis-Diaminrest der Formel -NH-Y-NH-, worin Y einen linearen oder verzweigten Kohlenwasserstoffrest oder auch den zweiwertigen Rest -CH2-CH2-S-S-CH2-CH2- bezeichnet;
d) eine Ureylengruppe der Formel -NH-CO-NH-.
Vorzugsweise ist X⁻ ein Anion, wie Chlorid oder Bromid. Diese Polymere weisen ein zahlenmittleres Molekulargewicht (Mn) im Allgemeinen zwischen 1.000 und 100.000 auf;
(8) polyquaternären Ammoniumpolymeren, umfassend Einheiten der Formel: 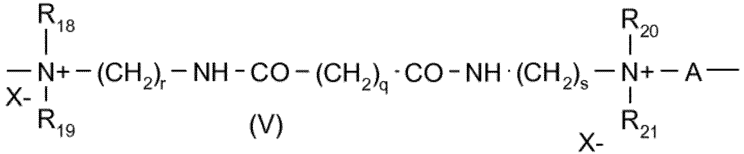 wobei:
- R18, R19, R20 und R21, gleich oder verschieden, für ein Wasserstoffatom oder einen Methyl-, Ethyl-, Propyl-, β-Hydroxyethyl-, β-Hydroxypropyl- oder -CH2CH2(OCH2CH2)pOH-Rest stehen, worin p gleich 0 oder einer ganze Zahl zwischen 1 und 6 ist, vorausgesetzt, dass R18, R19, R20 und R21 nicht gleichzeitig für ein Wasserstoffatom stehen,
- r und s, gleich oder verschieden, ganze Zahlen zwischen 1 und 6 sind,
- q gleich 0 oder einer ganzen Zahl zwischen 1 und 34 ist,
- X- ein Anion wie ein Halogenid bezeichnet,
- A einen zweiwertigen Dihalogenidrest bezeichnet oder vorzugsweise für -CH2-CH2-O-CH2-CH2- steht;
(9) quaternären Vinylpyrrolidon- und Vinylimidazolpolymeren;
(10) Polymeren, die in ihrer Struktur umfassen:
(a) eine oder mehrere Einheiten mit der folgenden Formel (A): 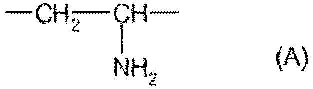
(b) gegebenenfalls eine oder mehrere Einheiten mit der folgenden Formel (B): 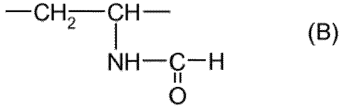 vorzugsweise ausgewählt aus denen der Familien (1), (6) und (7) .

22. Verfahren nach einem der vorhergehenden Ansprüche, wobei die sogenannte zweite kosmetische Zusammensetzung ein oder mehrere kationische Polymere enthält, ausgewählt aus 2-Methacryloyloxyethyltrimethylammoniumchlorid (Polyquaternium-37), Dimethyldiallylammoniumchlorid (Polyquaternium-6) und Mischungen davon.

23. Verfahren nach einem der vorhergehenden Ansprüche, wobei die sogenannte zweite kosmetische Zusammensetzung ein oder mehrere kationische Polymere mit einer Ladungsdichte von größer oder gleich 4 meq/g in einer Menge im Bereich von 0,01 Gew.-% bis 15 Gew.-%, vorzugsweise 0,1 bis 10 Gew.-% und vorzugsweise 0,2 bis 5 Gew.-%, bezogen auf das Gesamtgewicht der Zusammensetzung, umfasst.

24. Verfahren nach einem der vorhergehenden Ansprüche, wobei die sogenannte zweite kosmetische Zusammensetzung Wasser in einer Konzentration vorzugsweise im Bereich von 40 bis 99 Gew.-%, insbesondere von 50 bis 95 Gew.-%, besser von 60 bis 90 Gew.-%, bezogen auf das Gesamtgewicht der Zusammensetzung, umfasst.

25. Verfahren nach einem der vorhergehenden Ansprüche, wobei die sogenannte zweite kosmetische Zusammensetzung eine oder mehrere organische Säuren umfasst, vorzugsweise ausgewählt aus gesättigten oder ungesättigten Carbonsäuren; Sulfonsäuren; und Mischungen davon.

26. Verfahren nach einem der vorhergehenden Ansprüche, umfassend vor dem Schritt (i) zum Auftragen einer sogenannten ersten kosmetischen Zusammensetzung auf die Haare einen sogenannten Schritt zum Waschen der Haare, der das Auftragen einer Waschzusammensetzung, umfassend vorzugsweise ein oder mehrere waschaktive Tenside, auf die Haare umfasst; wobei auf den vorausgehenden Waschschritt vorzugsweise ein Schritt zum Spülen, beispielsweise mit Wasser, folgt.

27. Verfahren nach Anspruch 26, wobei die Waschzusammensetzung ein oder mehrere Tenside umfasst, die insbesondere aus anionischen Tensiden und amphoteren Tensiden und Mischungen davon ausgewählt sind; die vorzugsweise in einem Gesamtgehalt vorliegen, der im Bereich von 5 bis 35 Gew.-%, vorzugsweise von 10 bis 30 Gew.-%, bevorzugt von 15 bis 25 Gew.-%, bezogen auf das Gesamtgewicht der Zusammensetzung, liegen kann.

28. Verfahren nach einem der Ansprüche 26 und 27, wobei die Waschzusammensetzung ein oder mehrere kationische Polymere mit einer Ladungsdichte von größer oder gleich 4 meq/g, vorzugsweise größer oder gleich 5 meq/g; insbesondere mit einer Kationen-Ladungsdichte im Bereich von 4 bis 20 meq/g oder sogar von 5 bis 20 meq/g umfasst;
wobei die kationischen Polymere vorzugsweise aus jenen der oben in Anspruch 21 genannten Familien (1), (6) und (7) ausgewählt sind; noch besser aus 2-Methacryloyloxyethyltrimethylammoniumchlorid (Polyquaternium-37), Dimethyldiallylammoniumchlorid (Polyquaternium-6) und Mischungen davon ausgewählt sind.

29. Verfahren nach einem der Ansprüche 26 bis 28, wobei die Waschzusammensetzung ein oder mehrere kationische Polymere mit einer Ladungsdichte von größer oder gleich 4 meq/g in einer Menge im Bereich von 0,01 Gew.-% bis 15 Gew.-%, vorzugsweise 0,1 bis 10 Gew.-% und vorzugsweise 0,2 bis 5 Gew.-%, bezogen auf das Gesamtgewicht der Zusammensetzung, umfasst.

30. Verfahren nach einem der Ansprüche 26 bis 29, wobei die Waschzusammensetzung mindestens ein kationisches Polymer mit einer Ladungsdichte von größer oder gleich 4 meq/g umfasst, das mit mindestens einem kationischen Polymer mit einer Ladungsdichte von größer oder gleich 4 meq/g identisch ist, das in der zweiten kosmetischen Zusammensetzung vorhanden ist.

31. Verfahren nach einem der vorhergehenden Ansprüche, zum Reinigen und/oder Pflegen und/oder Konditionieren der Haare, um insbesondere sowohl eine sofortige als auch dauerhafte Konditionierung zu erhalten.

## Claims

1. Cosmetic hair treatment process, comprising:
- a step (i) of applying to said hair a "first" cosmetic composition, comprising one or more cationic surfactants, one or more silicones and one or more non-silicone fatty substances, and then
- a step (ii) of applying to said hair a "second" cosmetic composition, comprising one or more cationic surfactants, one or more cationic polymers with a cationic charge density of greater than or equal to 4 meq./g, and one or more organosilanes;
said process not comprising an intermediate rinsing step between said application steps (i) and (ii).

2. Process according to Claim 1, in which the application step (i) is followed by a step of leaving on the "first" cosmetic composition, preferably for 1 to 15 minutes.

3. Process according to either of the preceding claims, in which the application step (ii) is followed by a step of leaving on the "second" cosmetic composition, preferably for 1 to 25 minutes, especially 1 to 20 minutes, and a rinsing step, and then optionally a drying step.

4. Process according to one of the preceding claims, in which the "first" cosmetic composition comprises one or more cationic surfactants chosen from optionally polyoxyalkylenated primary, secondary or tertiary fatty amine salts, quaternary ammonium salts, and mixtures thereof; and especially chosen from:
- the quaternary ammonium salts of formula (Ia): 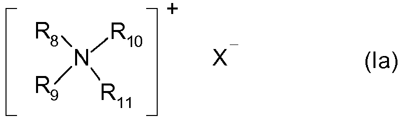 in which:
the groups R₈ to R₁₁, which may be identical or different, represent a linear or branched aliphatic group containing from 1 to 30 carbon atoms, or an aromatic group such as aryl or alkylaryl, at least one of the groups R₈ to R₁₁ containing from 8 to 30 and preferably from 12 to 24 carbon atoms, it being possible for the aliphatic groups to comprise heteroatoms such as, in particular, oxygen, nitrogen, sulfur and halogens; and
- X⁻ is an anion chosen especially from the group of halides, phosphates, acetates, lactates, (C₁-C₄)alkyl sulfates, and (C₁-C₄)alkyl- or (C₁-C₄)alkylarylsulfonates;
- the quaternary ammonium salts of imidazoline of formula (IIa) : 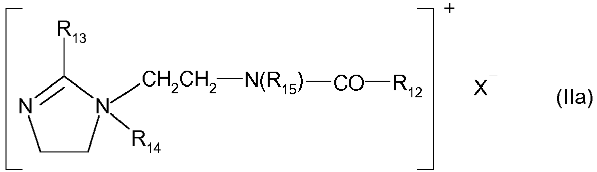 in which
R₁₂ represents an alkenyl or alkyl group comprising from 8 to 30 carbon atoms, for example derived from tallow fatty acids,
R₁₃ represents a hydrogen atom, a C₁-C₄ alkyl group or an alkenyl or alkyl group comprising from 8 to 30 carbon atoms,
R₁₄ represents a C₁-C₄ alkyl group,
R₁₅ represents a hydrogen atom or a C₁-C₄ alkyl group,
X⁻ is an anion chosen especially from the group of halides, phosphates, acetates, lactates, (C₁-C₄)alkyl sulfates, and (C₁-C₄)alkyl- or (C₁-C₄)alkylarylsulfonates;
- the di- or triquaternary ammonium salts of formula (IIIa) : 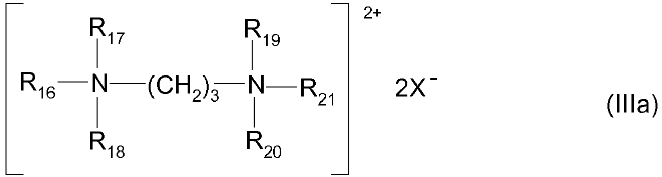 in which:
- R₁₆ denotes an alkyl group comprising from 16 to 30 carbon atoms, which is optionally hydroxylated and/or optionally interrupted with one or more oxygen atoms,
- R₁₇ denotes hydrogen, an alkyl group comprising from 1 to 4 carbon atoms or a group -(CH₂)₃-N⁺(R₁₆ₐ) (R₁₇ₐ)(R₁₈ₐ) ; R₁₆ₐ, R₁₇ₐ and R₁₈ₐ, which may be identical or different, denoting hydrogen or an alkyl group comprising from 1 to 4 carbon atoms,
- R₁₈, R₁₉, R₂₀ and R₂₁, which may be identical or different, denote hydrogen or an alkyl group comprising from 1 to 4 carbon atoms, and
- X⁻ is an anion, chosen especially from the group of halides, acetates, phosphates, nitrates, (C₁-C₄) alkyl sulfates and (C₁-C₄) alkyl- and (C₁-C₄) alkylarylsulfonates, in particular methyl sulfate and ethyl sulfate;
- the quaternary ammonium salts containing one or more ester functions, of formula (IVa) below: 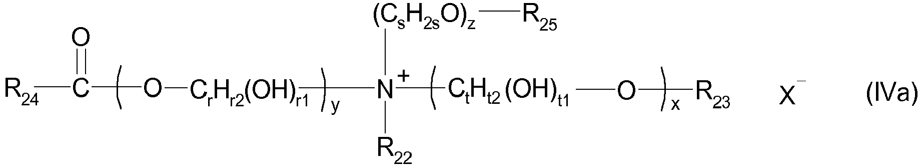
in which:
- R₂₂ is chosen from C₁-C₆ alkyl groups and C₁-C₆ hydroxyalkyl or dihydroxyalkyl groups,
- R₂₃ is chosen from the group R₂₆-C(=O)-; linear or branched, saturated or unsaturated C₁-C₂₂ hydrocarbon-based groups R₂₇; and a hydrogen atom,
- R₂₅ is chosen from the group R₂₈-C(=O)-; linear or branched, saturated or unsaturated C₁-C₆ hydrocarbon-based groups R₂₉; and a hydrogen atom,
- R₂₄, R₂₆ and R₂₈, which may be identical or different, are chosen from saturated or unsaturated, linear or branched C₇-C₂₁ hydrocarbon-based groups,
- r, s and t, which may be identical or different, are integers ranging from 2 to 6,
- r1 and t1, which may be identical or different, are equal to 0 or 1,
- y is an integer ranging from 1 to 10,
- x and z, which may be identical or different, are integers ranging from 0 to 10,
- X⁻ is an anion,
it being understood that r2 + r1 = 2r and t1 + t2 = 2t, and that the sum x + y + z ranges from 1 to 15,
with the proviso that when x = 0 then R₂₃ denotes R₂₇ and that when z = 0 then R₂₅ denotes R₂₉.

5. Process according to the preceding claim, in which the "first" cosmetic composition comprises one or more cationic surfactants chosen from the ammonium salts of formula (Ia) or (IVa), preferably of formula (IVa), in which:
- R₂₂ denotes a methyl or ethyl group,
- x and y are equal to 1,
- z is equal to 0 or 1,
- r, s and t are equal to 2,
- R₂₃ is chosen from the group R₂₆-C(=O)-, methyl, ethyl or C₁₄-C₂₂ hydrocarbon-based groups; and a hydrogen atom,
- R₂₅ is chosen from the group R₂₈-C(=O)-; and a hydrogen atom,
- R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups;
and better still from cetyltrimethylammonium, behenyltrimethylammonium and dipalmitoylethylhydroxyethylmethylammonium salts and mixtures thereof; and more particularly from behenyltrimethylammonium chloride or methosulfate, cetyltrimethylammonium chloride or methosulfate, dipalmitoylethylhydroxyethylmethylammonium chloride or methosulfate, and mixtures thereof.

6. Process according to one of the preceding claims, in which the "first" cosmetic composition comprises at least two different cationic surfactants; especially at least one cationic surfactant chosen from those of formula (Ia) and at least one cationic surfactant chosen from those of formula (IVa), the formulae being as defined in Claim 4.

7. Process according to one of the preceding claims, in which the "first" cosmetic composition comprises one or more cationic surfactant(s) in an amount ranging from 0.1% to 3.5% by weight, preferably from 0.2% to 3.5% by weight and preferentially from 0.3% to 3% by weight, relative to the total weight of the composition.

8. Process according to one of the preceding claims, in which the "first" cosmetic composition comprises one or more amino silicones, chosen especially from:
a) the polysiloxanes corresponding to formula (A): 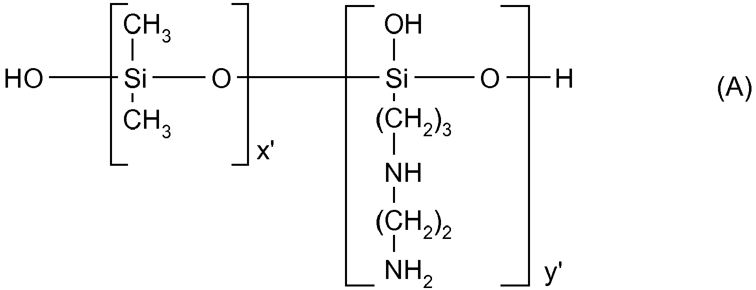 in which x' and y' are integers such that the weight-average molecular weight (Mw) is between 5000 and 500 000 approximately;
b) the amino silicones corresponding to formula (B):
R'ₐG₃₋ₐ-Si(OSiG₂)ₙ-(OSiG_{b}R'_{2-b})ₘ-O-SiG₃₋ₐ-R'ₐ (B)
in which:
- G, which may be identical or different, denotes a hydrogen atom or a phenyl, OH or C₁-C₈ alkyl, for example methyl, or C₁-C₈ alkoxy, for example methoxy, group,
- a, which may be identical or different, denotes 0 or an integer from 1 to 3, in particular 0,
- b denotes 0 or 1, in particular 1,
- m and n are numbers such that the sum (n + m) ranges from 1 to 2000 and in particular from 50 to 150, n possibly denoting a number from 0 to 1999 and in particular from 49 to 149, and m possibly denoting a number from 1 to 2000 and in particular from 1 to 10;
- R', which may be identical or different, denotes a monovalent radical of formula -C_{q}H_{2q}L in which q is a number ranging from 2 to 8 and L is an optionally quaternized amino group chosen from the following groups:
-N(R")₂; -N⁺(R")₃A-; -NR"-Q-N(R")₂ and -NR"-Q-N⁺(R")₃A-, in which R", which may be identical or different, denotes hydrogen, phenyl, benzyl, or a saturated monovalent hydrocarbon-based radical, for example a C₁-C₂₀ alkyl radical; Q denotes a linear or branched group of formula CᵣH₂ᵣ, r being an integer ranging from 2 to 6, preferably from 2 to 4; and A- represents a cosmetically acceptable anion, in particular a halide such as fluoride, chloride, bromide or iodide;
c) the amino silicones corresponding to formula (H): 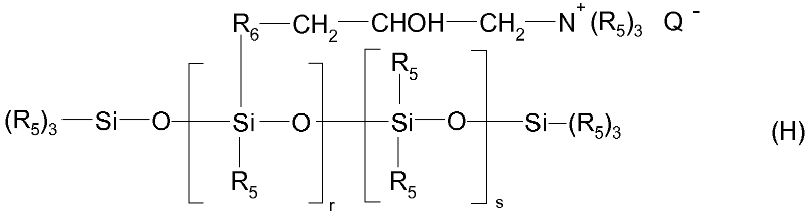 in which:
- R₅ represents a monovalent hydrocarbon-based radical containing from 1 to 18 carbon atoms, and in particular a C₁-C₁₈ alkyl or C₂-C₁₈ alkenyl, for example methyl radical;
- R₆ represents a divalent hydrocarbon-based radical, in particular a C₁-C₁₈ alkylene radical or a divalent C₁-C₁₈, for example C₁-C₈, alkyleneoxy radical linked to the Si via an SiC bond;
- Q⁻ is an anion such as a halide, in particular chloride, ion or an organic acid salt, in particular acetate;
- r represents a mean statistical value ranging from 2 to 20 and in particular from 2 to 8;
- s represents a mean statistical value ranging from 20 to 200 and in particular from 20 to 50;
d) the quaternary ammonium silicones of formula (I): 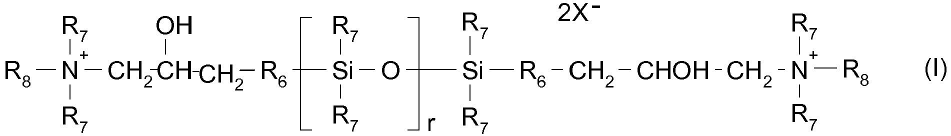 in which:
- R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example a methyl radical;
- R₆ represents a divalent hydrocarbon-based radical, in particular a C₁-C₁₈ alkylene radical or a divalent C₁-C₁₈, for example C₁-C₈, alkyleneoxy radical linked to the Si via an SiC bond;
- R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
- X⁻ is an anion such as a halide, in particular chloride, ion or an organic acid salt, in particular acetate;
- r represents a mean statistical value ranging from 2 to 200 and in particular from 5 to 100;
e) the amino silicones of formula (J): 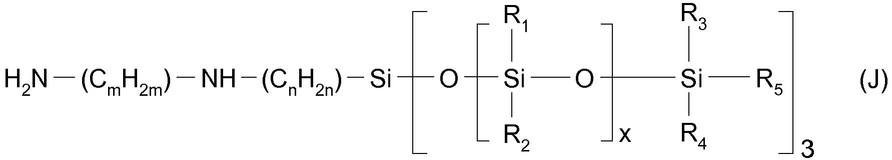 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5, and
- x is chosen such that the amine number ranges from 0.01 to 1 meq./g;
f) the multiblock polyoxyalkylenated amino silicones, of the type (AB)n, A being a polysiloxane block and B being a polyoxyalkylene block comprising at least one amine group.

9. Process according to one of the preceding claims, in which the "first" cosmetic composition comprises one or more amino silicones chosen from:
- the "trimethylsilyl amodimethicone" silicones corresponding to formula (C): 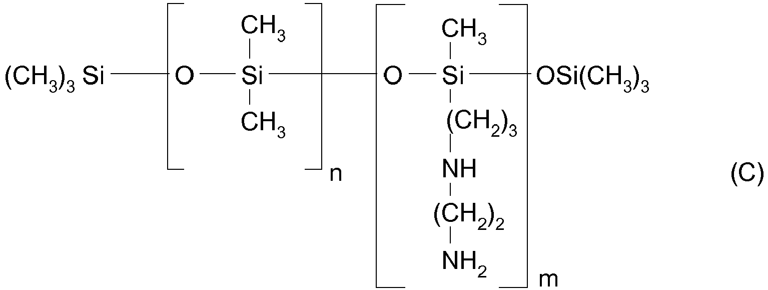 in which m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- the silicones of formula (D) below: 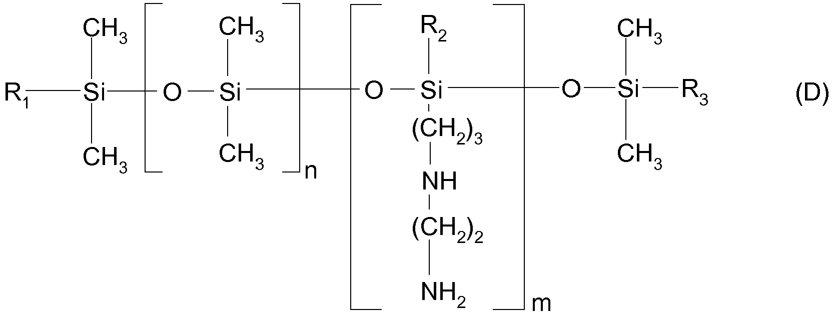 in which:
- m and n are numbers such that the sum (n + m) ranges from 1 to 1000 and in particular from 50 to 250 and more particularly from 100 to 200; it being possible for n to denote a number from 0 to 999 and in particular from 49 to 249 and more particularly from 125 to 175, and for m to denote a number from 1 to 1000 and in particular from 1 to 10, and more particularly from 1 to 5;
- R1, R2 and R3, which may be identical or different, represent a hydroxyl or C1-C4 alkoxy radical, at least one of the radicals R1 to R3 denoting an alkoxy radical;
- the silicones of formula (E) below: 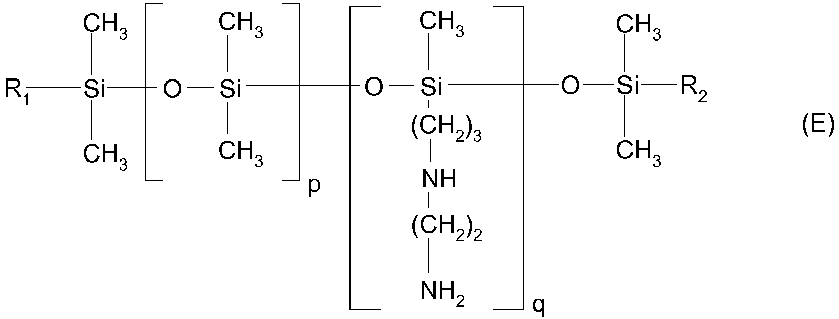 in which:
- p and q are numbers such that the sum (p + q) ranges from 1 to 1000, in particular from 50 to 350 and more particularly from 150 to 250; it being possible for p to denote a number from 0 to 999 and in particular from 49 to 349 and more particularly from 159 to 239, and for q to denote a number from 1 to 1000, in particular from 1 to 10 and more particularly from 1 to 5;
- R1 and R2, which are different, represent a hydroxyl or C1-C4 alkoxy radical, at least one of the radicals R1 or R2 denoting an alkoxy radical;
- the silicones of formula (F) below: 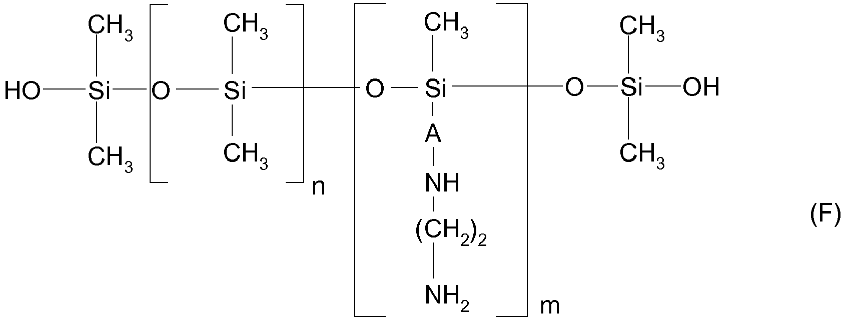 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms. This radical is preferably linear.
- the silicones of formula (G) below: 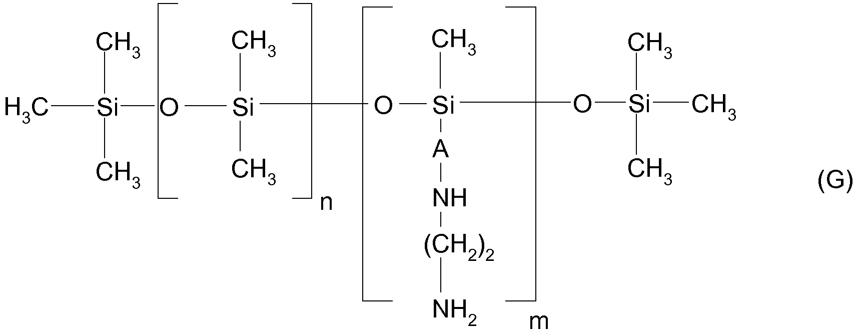 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms;
and most particularly corresponding to formula (F).

10. Process according to one of the preceding claims, in which the "first" cosmetic composition comprises the silicone(s) in an amount ranging from 0.1% to 2% by weight, preferably from 0.2% to 1.7% by weight and preferentially from 0.3% to 1.6% by weight, relative to the total weight of the composition.

11. Process according to one of the preceding claims, in which the "first" cosmetic composition comprises the amino silicone (s) in an amount ranging from 0.1% to 2% by weight, preferably from 0.2% to 1.7% by weight and preferentially from 0.3% to 1.6% by weight, relative to the total weight of the composition.

12. Process according to one of the preceding claims, in which the "first" cosmetic composition comprises one or more solid non-silicone fatty substances, chosen especially from fatty alcohols, fatty acid and/or fatty alcohol esters, non-silicone waxes, ceramides, and mixtures thereof.

13. Process according to one of the preceding claims, in which the "first" cosmetic composition comprises said non-silicone fatty substance(s) in an amount ranging from 0.1% to 20% by weight, preferably from 0.5% to 15% by weight and preferentially from 1% to 10% by weight, relative to the total weight of the composition.

14. Process according to one of the preceding claims, in which the "first" cosmetic composition comprises said solid non-silicone fatty substance(s) in an amount ranging from 0.5% to 15% by weight, preferably from 1% to 10% by weight and preferentially from 1.5% to 8% by weight, relative to the total weight of the composition.

15. Process according to one of the preceding claims, in which the "first" cosmetic composition comprises water in a concentration preferably ranging from 50% to 99% by weight, especially from 60% to 98% by weight and better still from 70% to 97% by weight, relative to the total weight of said composition.

16. Process according to one of the preceding claims, in which the "second" cosmetic composition comprises one or more cationic surfactants chosen from optionally polyox-yalkylenated primary, secondary or tertiary fatty amine salts, quaternary ammonium salts, and mixtures thereof; and especially chosen from:
- the quaternary ammonium salts of formula (Ia): 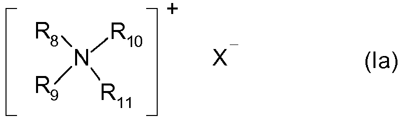 in which:
the groups R₈ to R₁₁, which may be identical or different, represent a linear or branched aliphatic group containing from 1 to 30 carbon atoms, or an aromatic group such as aryl or alkylaryl, at least one of the groups R₈ to R₁₁ containing from 8 to 30 and preferably from 12 to 24 carbon atoms, it being possible for the aliphatic groups to comprise heteroatoms such as, in particular, oxygen, nitrogen, sulfur and halogens; and
- X⁻ is an anion chosen especially from the group of halides, phosphates, acetates, lactates, (C₁-C₄)alkyl sulfates, and (C₁-C₄)alkyl- or (C₁-C₄)alkylarylsulfonates;
- the quaternary ammonium salts of imidazoline of formula (IIa) : 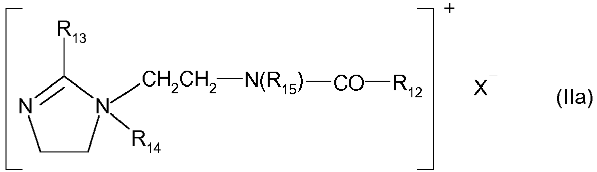 wherein
R₁₂ represents an alkenyl or alkyl group comprising from 8 to 30 carbon atoms, for example derived from tallow fatty acids,
R₁₃ represents a hydrogen atom, a C₁-C₄ alkyl group or an alkenyl or alkyl group comprising from 8 to 30 carbon atoms,
R₁₄ represents a C₁-C₄ alkyl group,
R₁₅ represents a hydrogen atom or a C₁-C₄ alkyl group,
X⁻ is an anion chosen especially from the group of halides, phosphates, acetates, lactates, (C₁-C₄)alkyl sulfates,
and (C₁-C₄) alkyl- or (C₁-C₄) alkylarylsulfonates;
- the di- or triquaternary ammonium salts of formula (IIIa) : 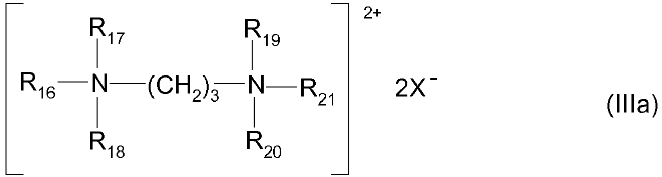 in which:
- R₁₆ denotes an alkyl group comprising from 16 to 30 carbon atoms, which is optionally hydroxylated and/or optionally interrupted with one or more oxygen atoms,
- R₁₇ denotes hydrogen, an alkyl group comprising from 1 to 4 carbon atoms or a group -(CH₂)₃-N⁺(R₁₆ₐ)(R₁₇ₐ)(R₁₈ₐ) ; R₁₆ₐ, R₁₇ₐ and R₁₈ₐ, which may be identical or different, denoting hydrogen or an alkyl group comprising from 1 to 4 carbon atoms,
- R₁₈, R₁₉, R₂₀ and R₂₁, which may be identical or different, denote hydrogen or an alkyl group comprising from 1 to 4 carbon atoms, and
- X⁻ is an anion, chosen especially from the group of halides, acetates, phosphates, nitrates, (C₁-C₄) alkyl sulfates and (C₁-C₄) alkyl- and (C₁-C₄) alkylarylsulfonates, in particular methyl sulfate and ethyl sulfate;
- the quaternary ammonium salts containing one or more ester functions, of formula (IVa) below: 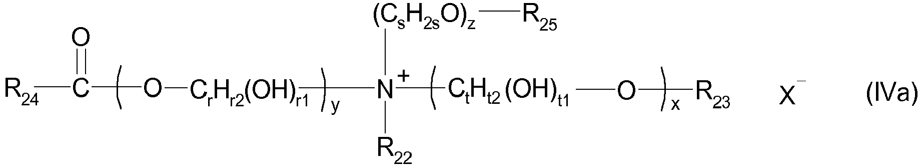 in which:
- R₂₂ is chosen from C₁-C₆ alkyl groups and C₁-C₆ hydroxyalkyl or dihydroxyalkyl groups,
- R₂₃ is chosen from the group R₂₆-C(=O)-; linear or branched, saturated or unsaturated C₁-C₂₂ hydrocarbon-based groups R₂₇; and a hydrogen atom,
- R₂₅ is chosen from the group R₂₈-C(=O)-; linear or branched, saturated or unsaturated C₁-C₆ hydrocarbon-based groups R₂₉; and a hydrogen atom,
- R₂₄, R₂₆ and R₂₈, which may be identical or different, are chosen from saturated or unsaturated, linear or branched C₇-C₂₁ hydrocarbon-based groups,
- r, s and t, which may be identical or different, are integers ranging from 2 to 6,
- r1 and t1, which may be identical or different, are equal to 0 or 1,
- y is an integer ranging from 1 to 10,
- x and z, which may be identical or different, are integers ranging from 0 to 10,
- X⁻ is an anion,
it being understood that r2 + r1 = 2r and t1 + t2 = 2t, and that the sum x + y + z ranges from 1 to 15,
with the proviso that when x = 0 then R₂₃ denotes R₂₇ and that when z = 0 then R₂₅ denotes R₂₉.

17. Process according to the preceding claim, in which the "second" cosmetic composition comprises one or more cationic surfactants chosen from the ammonium salts of formula (Ia) or (IVa), preferably of formula (IVa), in which:
- R₂₂ denotes a methyl or ethyl group,
- x and y are equal to 1,
- z is equal to 0 or 1,
- r, s and t are equal to 2,
- R₂₃ is chosen from the group R₂₆-C(=O)-, methyl, ethyl or C₁₄-C₂₂ hydrocarbon-based groups; and a hydrogen atom,
- R₂₅ is chosen from the group R₂₈-C(=O)-; and a hydrogen atom,
- R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups;
and better still from cetyltrimethylammonium, behenyltrimethylammonium and dipalmitoylethylhydroxyethylmethylammonium salts and mixtures thereof; and more particularly from behenyltrimethylammonium chloride or methosulfate, cetyltrimethylammonium chloride or methosulfate, dipalmitoylethylhydroxyethylmethylammonium chloride or methosulfate, and mixtures thereof.

18. Process according to one of the preceding claims, in which the "second" cosmetic composition comprises one or more cationic surfactant(s) in an amount ranging from 0.05% to 10% by weight, preferably from 0.1% to 5% by weight and preferentially from 0.3% to 3% by weight, relative to the total weight of the composition.

19. Process according to one of the preceding claims, in which the "second" cosmetic composition comprises one or more organosilanes chosen from the compounds of formulae (I) and/or oligomers thereof:
R₁Si(OR₂)_{z}(R₃)ₓ(OH)_{y} (I)
in which:
- R₁ is a cyclic or acyclic, linear or branched, saturated or unsaturated C₁ to C₂₂, in particular C₂ to C₂₀, hydrocarbon-based chain, which may be substituted with a group chosen from amine groups NH₂ or NHR (R being a linear or branched C₁ to C₂₀, in particular C₁ to C₆, alkyl, a C₃ to C₄₀ cycloalkyl or a C₆ to C₃₀ aromatic radical) ; the hydroxyl group (OH); a thiol group; an aryl group (more particularly benzyl), which is possibly substituted with an NH₂ or NHR group; it being possible for R₁ to be interrupted with a heteroatom (O, S or NH) or a carbonyl group (CO),
- R₂ and R₃, which may be identical or different, represent a linear or branched alkyl group comprising from 1 to 6 carbon atoms,
- y denotes an integer ranging from 0 to 3,
- z denotes an integer ranging from 0 to 3, and
- x denotes an integer ranging from 0 to 2,
- with z + x + y = 3.

20. Process according to one of the preceding claims, in which the "second" cosmetic composition comprises said organosilane(s) in an amount ranging from 0.1% to 15% by weight, preferably from 1% to 10% by weight and preferentially from 2% to 8% by weight, relative to the total weight of the composition.

21. Process according to one of the preceding claims, in which the "second" cosmetic composition comprises one or more cationic polymers chosen from:
(1) homopolymers or copolymers derived from acrylic or methacrylic esters or amides and comprising at least one of the units of the following formulae: 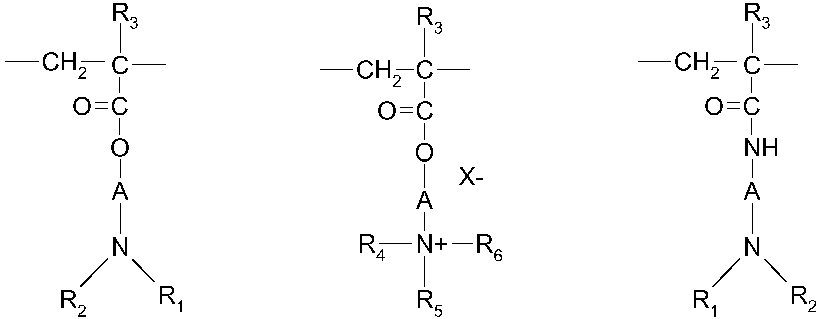 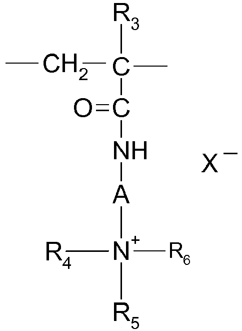 in which:
- R3, which may be identical or different, denote a hydrogen atom or a CH₃ radical;
- A, which may be identical or different, represent a linear or branched divalent alkyl group of 1 to 6 carbon atoms, preferably 2 or 3 carbon atoms, or a hydroxyalkyl group of 1 to 4 carbon atoms;
- R4, R5 and R6, which may be identical or different, represent an alkyl group containing from 1 to 18 carbon atoms or a benzyl radical, preferably an alkyl group containing from 1 to 6 carbon atoms;
- R1 and R2, which may be identical or different, represent a hydrogen atom or an alkyl group containing from 1 to 6 carbon atoms, preferably methyl or ethyl;
- X denotes an anion derived from a mineral or organic acid, such as a methosulfate anion or a halide such as chloride or bromide;
(2) polymers formed from piperazinyl units and divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers;
(3) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides can be alkylated or, if they comprise one or more tertiary amine functions, they can be quater-nized;
(4) polyaminoamide derivatives resulting from the condensation of polyalkylene polyamines with polycarboxylic acids followed by alkylation with difunctional agents;
(5) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyamino amide being reacted with epichlorohydrin in a mole ratio of epichlorohydrin relative to the secondary amine group of the polyaminoamide preferably of between 0.5:1 and 1.8:1;
(6) alkyldiallylamine or dialkyldiallylammonium cyclo-polymers, such as homopolymers or copolymers comprising, as the main chain constituent, units corresponding to formula (I) or (II) : 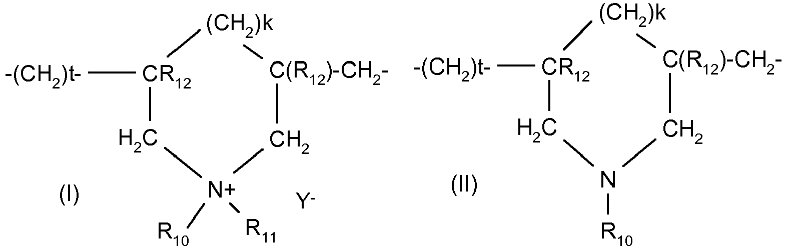 in which
- k and t are equal to 0 or 1, the sum k + t being equal to 1;
- R12 denotes a hydrogen atom or a methyl radical;
- R10 and R11, independently of each other, denote a C1-C6 alkyl group, a C1-C5 hydroxyalkyl group, a C1-C4 ami-doalkyl group; or alternatively R10 and R11 may denote, together with the nitrogen atom to which they are attached, a heterocyclic group such as piperidyl or morpholinyl; R10 and R11, independently of each other, preferably denote a C1-C4 alkyl group;
- Y⁻ is an anion such as bromide, chloride, acetate, borate, citrate, tartrate, bisulfate, bisulfite, sulfate or phosphate;
(7) quaternary diammonium polymers comprising repeating units of formula: 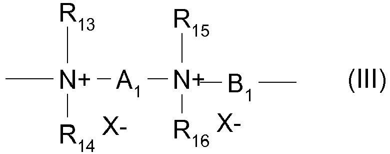 in which:
- R13, R14, R15 and R16, which may be identical or different, represent aliphatic, alicyclic or arylaliphatic radicals comprising from 1 to 20 carbon atoms or C1-C12 hydroxyalkyl aliphatic radicals,
or else R13, R14, R15 and R16, together or separately, form, with the nitrogen atoms to which they are attached, heterocycles optionally comprising a second non-nitrogen heteroatom;
or else R13, R14, R15 and R16 represent a linear or branched C1-C6 alkyl radical substituted with a nitrile, ester, acyl, amide or -CO-O-R17-D or -CO-NH-R17-D group, where R17 is an alkylene and D is a quaternary ammonium group;
- A1 and B1 represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
- X⁻ denotes an anion derived from a mineral or organic acid;
it being understood that A1, R13 and R15 can form, with the two nitrogen atoms to which they are attached, a piperazine ring;
in addition, if A1 denotes a linear or branched, saturated or unsaturated alkylene or hydroxyalkylene radical, B1 may also denote a group (CH₂)n-CO-D-OC-(CH₂)p- with n and p, which may be identical or different, being integers ranging from 2 to 20, and D denoting:
a) a glycol residue of formula -O-Z-O-, in which Z denotes a linear or branched hydrocarbon-based radical, or a group corresponding to one of the following formulae: -(CH₂CH₂O)ₓ-CH₂CH₂- and -[CH₂CH(CH₃)O]_{y}-CH₂CH(CH₃)-, in which x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula -NH-Y-NH-, in which Y denotes a linear or branched hydrocarbon-based radical, or else the divalent radical -CH₂-CH₂-S-S-CH₂-CH₂- ;
d) a ureylene group of formula -NH-CO-NH-.
Preferably, X⁻ is an anion, such as chloride or bromide. These polymers have a number-average molar mass (Mn) generally of between 1000 and 100 000;
(8) polyquaternary ammonium polymers comprising units of formula (V): 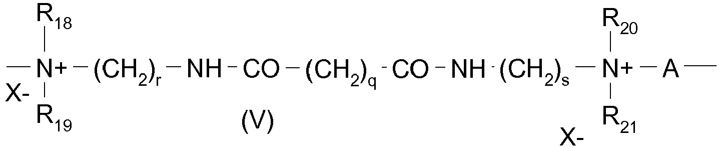 in which:
- R18, R19, R20 and R21, which may be identical or different, represent a hydrogen atom or a methyl, ethyl, propyl, β-hydroxyethyl, β-hydroxypropyl or-CH₂CH₂(OCH₂CH₂)pOH radical, in which p is equal to 0 or to an integer between 1 and 6, with the proviso that R18 R19, R20 and R21 do not simultaneously represent a hydrogen atom,
- r and s, which may be identical or different, are integers between 1 and 6,
- q is equal to 0 or to an integer between 1 and 34,
- X⁻ denotes an anion such as a halide,
- A denotes a divalent dihalide radical or preferably represents -CH₂-CH₂-O-CH₂-CH₂-;
(9) quaternary polymers of vinylpyrrolidone and of vinylimidazole;
(10) polymers comprising in their structure:
(a) one or more units corresponding to formula (A) below: 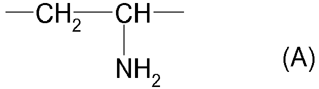
(b) optionally one or more units corresponding to formula (B) below: 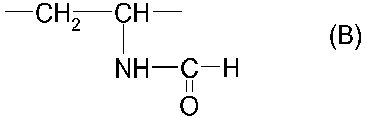 preferably chosen from those of families (1), (6) and (7) .

22. Process according to one of the preceding claims, in which the "second" cosmetic composition comprises one or more cationic polymers chosen from 2-methacryloyloxy-ethyltrimethylammonium chloride (Polyquaternium-37), di-methyldiallylammonium chloride (Polyquaternium-6) and mixtures thereof.

23. Process according to one of the preceding claims, in which the "second" cosmetic composition comprises one or more cationic polymers with a charge density of greater than or equal to 4 meq./g, in an amount ranging from 0.01% to 15% by weight, preferably from 0.1% to 10% by weight and preferentially from 0.2% to 5% by weight, relative to the total weight of the composition.

24. Process according to one of the preceding claims, in which the "second" cosmetic composition comprises water in a concentration preferably ranging from 40% to 99% by weight, especially from 50% to 95% by weight and better still from 60% to 90% by weight, relative to the total weight of said composition.

25. Process according to one of the preceding claims, in which the "second" cosmetic composition comprises one or more organic acids, preferably chosen from saturated or unsaturated carboxylic acids; sulfonic acids; and mixtures thereof.

26. Process according to one of the preceding claims, comprising, prior to said step (i) of applying a "first" cosmetic composition to said hair, a step of "washing" the hair, comprising the application to said hair of a washing composition, preferably comprising one or more detergent surfactants; preferably, said preliminary washing step being followed by a rinsing step, for example with water.

27. Process according to Claim 26, in which the washing composition comprises one or more surfactants chosen especially from anionic surfactants and amphoteric surfactants, and mixtures thereof, preferably present in a total content which may range from 5% to 35% by weight, preferably from 10% to 30% by weight and preferentially from 15% to 25% by weight, relative to the total weight of the composition.

28. Process according to either of Claims 26 and 27, in which the washing composition comprises one or more cationic polymers with a charge density of greater than or equal to 4 meq./g, preferably greater than or equal to 5 meq./g; especially with a cationic charge density ranging from 4 to 20 meq./g, or even from 5 to 20 meq./g; preferably, said cationic polymers being chosen from those of families (1), (6) and (7) above in claim 21; better still chosen from 2-methacryloyloxyethyltrimethylammonium chloride (Polyquaternium-37), dimethyldiallylammonium chloride (Polyquaternium-6) and mixtures thereof.

29. Process according to one of Claims 26 to 28, in which the washing composition comprises one or more cationic polymer(s) with a charge density of greater than or equal to 4 meq./g in an amount ranging from 0.01% to 15% by weight, preferably from 0.1% to 10% by weight and preferentially from 0.2% to 5% by weight, relative to the total weight of said composition.

30. Process according to one of Claims 26 to 29, in which the washing composition comprises at least one cationic polymer with a charge density of greater than or equal to 4 meq./g identical to at least one cationic polymer with a charge density of greater than or equal to 4 meq./g present in the second cosmetic composition.

31. Process according to one of the preceding claims, for cleansing and/or caring for and/or conditioning the hair, especially for obtaining conditioning that is both immediate and long-lasting.
